# EUROPÄISCHE PATENTSCHRIFT

(11) **EP 2 304 045 B1**
(45) Veröffentlichungstag und Bekanntmachung des Hinweises auf die Patenterteilung: **09.05.2018**
(21) Anmeldenummer: 09777435.0
(22) Anmeldetag: 25.07.2009
(51) Int. Cl.: C12P 21/02, C12N 15/70

(54) **CHAPERON-"TOOLBOX"**
CHAPERONE TOOLBOX
BOÎTE À OUTIL DE CHAPERONS

(30) Priorität: 28.07.2008 DE 102008035152
(43) Veröffentlichungstag der Anmeldung: 06.04.2011
(73) Patentinhaber: evocatal GmbH, 40789 Monheim am Rhein (DE)
(72) Erfinder: JÄGER, Karl-Erich, 45479 Mülheim/Ruhr (DE); EGGERT, Thorsten, 45259 Essen (DE); HUMMEL, Werner, 52445 Titz (DE); SEIDEL, Andrea, 81545 München (DE); ROSENAU, Frank, 43230 Kirchheim unter Tech (DE)
(74) Vertreter: Michalski Hüttermann & Partner Patentanwälte mbB
(86) Internationale Anmeldenummer: PCT/EP2009/005398
(87) Internationale Veröffentlichungsnummer: WO 2010/012424

(56) Entgegenhaltungen:
- WO-A1-2006/067511
- WO-A2-03/057897
- ANDREA KREUZ: "Coexpression von Chaperonen und Faltungskatalysatoren aus Pseudomonas aeruginosa: Neue Strategien zur Optimisierung mikrobieller Expressionssysteme" August 2006 (2006-08), XP002548448 Düsseldorf Gefunden im Internet: URL:http://docserv.uni-duesseldorf.de/serv lets/DocumentServlet?id=3591> [gefunden am 2009-10-01]
- DE MARCO ARIO ET AL: "Chaperone-based procedure to increase yields of soluble recombinant proteins produced in E. coli", BMC BIOTECHNOLOGY, BIOMED CENTRAL LTD. LONDON, GB, vol. 7, no. 1, 12 June 2007 (2007-06-12), page 32, XP021029472, ISSN: 1472-6750, DOI: 10.1186/1472-6750-7-32

## Beschreibung

### Gebiet der Erfindung

Die Erfindung betrifft das Gebiet der Genexpression und die Herstellung von Proteinen unter Verwendung von transformierten Wirtszellen und rekombinanten DNA-Vektoren. Die Erfindung beschreibt ein Verfahren zur Expression von Zielproteinen, dadurch gekennzeichnet, dass Faltungshelferproteine, welche auch als Chaperone bezeichnet werden, coexprimiert werden. Die coexprimierten Chaperone werden dabei aus einer Liste von einzelnen Faltungshelferproteinen ausgewählt und eine Vielzahl von Kombinationen aus zwei, drei oder mehreren Proteinen gebildet (Chaperon-Toolbox), die eine Expression gruppierter Chaperone-Gene mit heterologen Genen für Zielproteine ermöglicht.

Darüber hinaus betrifft die Erfindung die Verwendung von Plasmiden, die Polynucleotide, die für Faltungshelferproteine (Chaperone) codieren, umfassen, wobei das Plasmid zur Expression der Faltungshelferproteine (Chaperone) befähigt ist; und sie betrifft ein Verfahren, die Faltungshelferproteine-codierenden Polynucleotide auszuwählen und zwei, drei oder mehrere davon randomisiert zu kombinieren.

### Hintergrund der Erfindung

Mit der voranschreitenden Nutzung von Proteinen als Biokatalysatoren oder als Pharmaka und der stetig steigenden Anzahl an Sequenzdaten pro- und eukaryotischer Genome besteht ein zunehmendes Interesse an leistungsfähigen industriell nutzbaren Expressionssystemen. Da nur sehr wenige Wirtsstämme für die Expression zur Verfügung stehen, werden die meisten rekombinanten Proteine heterolog exprimiert, d. h. das zu exprimierende Zielprotein stammt nicht aus dem Organismus, der als Wirtsstamm für die Expression dient. Ein häufig auftretendes Problem bei der heterologen Produktion rekombinanter Proteine ist die Aggregation des Zielproteins (Bildung von sog. Inclusion bodies) im Cytoplasma des Wirtsstamms. Der Grund für die Aggregation sind inkorrekte inter- und intramolekulare Wechselwirkungen von Proteinketten des Zielproteins, wobei diese Aggregate in aller Regel biologisch bzw. biochemisch inaktiv sind. Die Ursachen sind zum Teil bekannt, beispielsweise kann das Gen des Zielproteins Tripletts von Polynucleotiden (Codons) enthalten, die für den Wirtsstamm nicht gut oder gar nicht genutzt werden können. Weitere Probleme können durch die fehlende Knüpfung von Disulfidbrücken entstehen oder durch eine zu hohe Expressionsrate des Zielproteins. Daher wurden unterschiedliche Strategien entwickelt, um die Bildung von Inclusion bodies zu reduzieren oder sogar zu verhindern. Dazu zählen beispielsweise das Herabsetzen der Inkubationstemperatur, die Reduktion der Expressionsrate durch Verwendung entsprechender Vektoren, die Wahl eines anderen Expressionswirtes, die Veränderung des Zielgens oder die Coexpression von Faltungshelferproteinen [1].

Faltungshelferproteine kommen in allen Zellen vor, sie sind für eine Reihe von Proteinen zur Ausbildung der richtigen Struktur notwendig. Speziell bei Zellstress ist die Gefahr des vermehrten Auftretens fehlgefalteter Proteine besonders groß, daher werden unter diesen Bedingungen eine Vielzahl sogenannter "Heat shock-Proteine (HSP)" als Faltungshelfer gebildet. In einigen Fällen sind die biochemischen Funktionen von Faltungshelferproteinen gut untersucht. So gibt es in Prokaryoten schon bei der Bildung der wachsenden Polypeptidkette am Ribosom eine Wechselwirkung mit dem Triggerfaktor Tf, danach wird die Faltung des Zielproteins von den Faltungshelferproteinen DnaK und DnaJ kontrolliert und in der Folge steht noch der Proteinkomplex aus GroEL und GroES bereit, den Faltungsprozess zu lenken (siehe z.B. Review [2]).

Führt man sich vor Augen, wie vielfältig die Wechselwirkungen neusynthetisierter Proteine mit Faltungshelfern bis zum Erreichen der endgültigen Struktur sind, ist es nicht überraschend, dass es bei der heterologen Überexpression von Proteinen in hohem Ausmaß zu fehlgefalteten Proteinen kommt, die dann als Proteinaggregate (Inclusion bodies) anfallen oder direkt degradiert werden. Zur Überwindung dieses Problems wurden daher die bekannten Systeme bzw. Komponenten an Chaperone ebenfalls mit exprimiert, um zu versuchen, Proteinaggregate zu vermeiden. In der Literatur sind Beispiele dafür beschrieben, dass sowohl die Coexpression von einzelnen Chaperonen wie dem Triggerfaktor Tf oder von DnaK als auch von Chaperonsystemen, die aus natürlicherweise ebenfalls zusammenwirkenden Proteinen bestehen, wie dem GroEL/GroES- (GroELS-) System oder DnaK gemeinsam mit DnaJ zu einer erhöhten Bildung von löslichem Zielprotein führen können [3]. Allerdings hat sich auch gezeigt, dass kein Faltungshelferprotein oder eine Kombinationen von solchen als generelle Lösung gegen Fehlfaltung angesehen werden kann [4].

Eine detaillierte Zusammenstellung, welche Proteine überhaupt Faltungshelfer-Funktion besitzen, zeigt, dass es verschiedene Gruppen solcher Proteine mit unterschiedlichen biochemischen Wirkungsmechanismen gibt, beispielsweise molekulare Chaperone. Dabei handelt es sich um eine Gruppe verwandter Proteine, die aufgrund ihrer Molekulargewichte in Klassen oder Familien unterteilt werden können (Heatshock-Proteine Hsp 60, Hsp 70, Hsp 90, Hsp 100 oder sHsp). Faltungshelfer-Funktion findet man auch bei den Peptidyl-Prolyl cis-trans Isomerasen, (PPlasen) [5] oder den Dsb-Proteinen (disulfidebond Proteinen), die die Bildung von Disulfidbrücken katalysieren können [6].

Bislang ist aber nicht von allen Faltungshelfer-Proteinen bekannt, inwiefern sie tatsächlich eine Funktion als Faltungshelfer bei der Expression heterologer Gene ausüben. Neben solchen Proteinen, die eindeutig als Faltungshelfer bezeichnet werden können, wie der Triggerfaktor Tf, DnaK, DnaJ, GroEL oder auch GroES gibt es Proteine, von denen man nur auf Grund ihrer Sequenzmotive vermuten kann, dass sie für Faltungshelferproteine codieren (putative Faltungshelferproteine).

Versuche zur Coexpression von Faltungshelfer-Proteinen haben für das Zielprotein sehr unterschiedliche Ergebnisse gezeigt: Bei der Verwendung einzelner Faltungshelferproteine zeigt sich, dass es sowohl positive als auch negative als auch keine Einflüsse auf die Expression eines Zielproteins gibt. Ergebnisse, die mit einem Zielprotein gefunden wurden, lassen sich nicht auf andere Proteine übertragen. Dies trifft auch für die Kombination von mehreren Helferproteinen zu. Ein genereller und umfassender Ansatz zur Verbesserung der Expression heterologer Gene durch Coexpression von Faltungshelferproteinen ist daher bislang nicht verfügbar.

In BMC Biotechnology, Bd. 7, Nr. 1, 12. Juni 2007 offenbaren De Marco Ario *et al.* ein Chaperon-basiertes Verfahren zur Verbesserung der Ausbeute von in E. Coli erzeugten löslichen rekombinanten Proteine. In der internationalen Patentanmeldung WO 03/057897 A2 wird ein Verfahren zur Expression von rekombinanten Zielproteinen unter Verwendung der Chaperon-Gene GroEL, GRoES, Dank, DnaJ, GRpe, und derer Homologe beschrieben. In der internationalen Patentanmeldung WO2006/067511 A1 wird ein Verfahren zur Herstellung eines Zielproteins unter Verwendung einer Kombination zweier unterschiedlicher Chaperone beschrieben.

**Aufgabe** der Erfindung ist es daher, ein Verfahren zur verbesserten Expression von einer Vielzahl unterschiedlicher Proteine, insbesondere mittels heterogener Expression von rekombinanten Proteinen bereitzustellen.

**Gelöst** wird diese Aufgabe durch ein Verfahren gemäß Anspruch 1.

Mit dem erfindungsgemäßen Verfahren werden verschiedene Gene, die für Faltungshelferproteine codieren, aus einer Vielzahl verfügbarer Gene derart miteinander kombiniert, dass einerseits verschiedene biochemische Prinzipien von Chaperonen miteinander kombiniert werden, andererseits die Anzahl der Kombinationen derart gering gehalten wird, dass im Rahmen einer Verfahrensentwicklung zur Produktion von Proteinen ein vertretbarer experimenteller Aufwand erforderlich ist.

In einer Ausgestaltung der Erfindung werden die wenigstens drei für unterschiedliche Chaperone codierenden Nukleotidsequenzen permutierend aus einer Gruppe von für Chaperone codierenden Nukleotidsequenzen des Organismus Pseudomonas aeruginosa ausgewählt.

Ein wesentlicher Schritt zur Realisierung des Ziels, mehrere Gene und damit mehrere Faltungshelferproteine in eine Produktionszelle einzubringen, besteht in der Nutzung von Vektoren, die für Faltungshelferproteine codierende Gene tragen. Um mehrere dieser Gene in Produktionszellen einzubringen, können verschiedene Wege beschritten werden, beispielsweise indem Zellen mit einem einzigen Vektor transformiert werden, der mehrere inserierte Gene trägt. Alternativ können aber auch Zellen mit mehreren Vektoren, die jeweils nur ein Gen oder eine geringere Zahl von Genen tragen, transformiert werden, so dass man durch die Kombination mehrerer Vektoren in der Folge zu den gewünschten Kombinationen an Faltungshelfern kommt. Dies kann beispielsweise durch Klonierung der Einzelchaperone in verschiedene, miteinander kompatible Vektorsysteme unter Kontrolle eines oder mehrerer geeigneter, induzierbarer Promotoren erreicht werden. Die Kompatibilität der Vektorsysteme bezüglich ihrer ORI (origin of replication) und entsprechender Selektionsmarkergene ist dabei entscheidend für die Kopienzahl der Plasmide pro Zelle und die damit verbundenen vergleichbaren Konzentrationen der entsprechenden Chaperone. Solche Plasmidbibliotheken können in einem einzigen Schritt transformiert werden, wodurch eine kombinatorische Zusammenstellung und Coexpression unterschiedlicher Chaperone gewährleistet wird.

Zur Lösung der erfindungsgemäßen Aufgabe wurde daher eine Genexpressionsbank ("Chaperon-Toolbox") konstruiert, die in Wirtszellen eine randomisierte Expression einzelner oder gruppierter Faltungshelfergene mit heterologen Genen für Zielproteine ermöglicht. Wirtszellen können dabei pro- oder eukaryotische Zellen sein, also beispielsweise Hefen oder Pilze, Pflanzen- oder Säugetierzellen oder Zellen einer Zellkultur, speziell beispielsweise *Escherichia coli, Bacillus subtilis, Pseudomonas* sp., *Saccharomyces* sp., *Pichia* sp., *Hansenula* sp., *Schizosaccharomyces pombe, Corynebacterium* sp. oder *Aspergillus* sp.

Zur Konstruktion der Genexpressionsbank wurden folgende Teilschritte bearbeitet:
1. Aufgrund der unterschiedlichen Funktionen im Faltungsprozeß sollten möglichst viele verschiedene Faltungshelfer Bestandteil dieser Chaperon-Toolbox sein. Dazu sollten zunächst durch ein umfassendes "in silico"-Screening sämtliche Gene identifiziert werden, die für putative Faltungshelfer in einem Bakterium (in diesem Fall *Pseudomonas aeruginosa*) codieren.
2. Die identifizierten Gene, die für Faltungshelfer in diesem Organismus codieren, sollten aus dem Chromosom des Wildtyp-Stammes *P. aeruginosa* isoliert und in spezielle Klonierungsplasmide eingebracht werden.
3. Anschließend sollte eine Genexpressionsbank erzeugt werden. Darin sollten nicht nur eine Vielzahl einzelner Chaperone und Faltungskatalysatoren vorliegen, sondern vor allem randomisierte Kombinationen von drei Faltungshelfergenen auf je einem *broad-host-range* Expressionsplasmid der Expressionsbank verfügbar gemacht werden.

Durch Coexpressionsversuche mit verschiedenen Zielproteinen aus Pro- und Eukaryoten sollte schließlich die Nützlichkeit dieser Genexpressionsbank untersucht werden. Dabei war das Ziel, die jeweiligen Faltungsmodulatoren oder Kombinationen dieser Proteine, die zu einer Verbesserung der Ausbeute löslichen, aktiven Zielproteins führen, zu identifizieren.

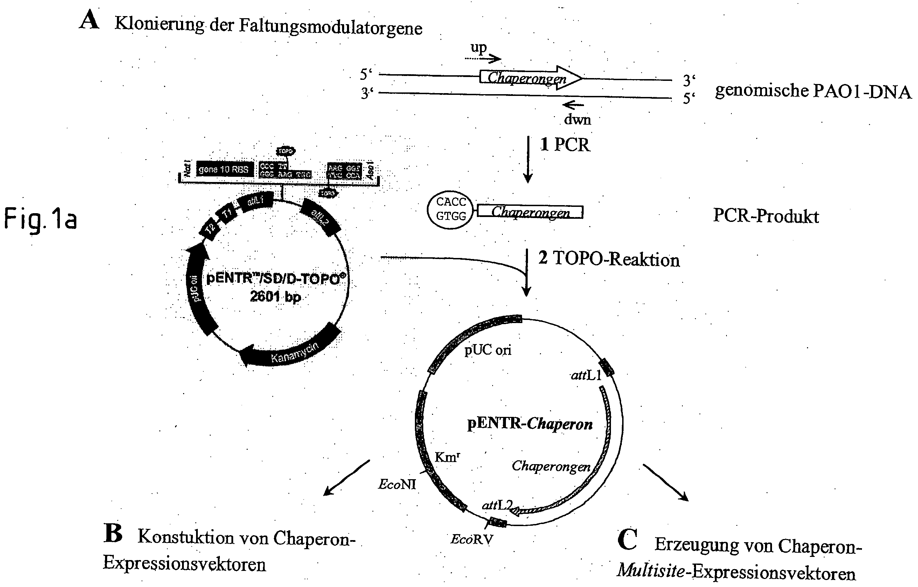

**Fig. 1A bis 1C****: Übersicht über die Klonierungsstrategie zur Erzeugung einer Chaperon-Genexpressionsbank.** Die Abbildung ist in drei Punkte unterteilt. Unter **A** ist die Amplifikation und Klonierung der Faltungshelfergene dargestellt. **B** beschreibt die Konstruktion von Expressionsvektoren mit einzelnen Chaperone und **C** zeigt die Schritte zur Erzeugung von *Multisite*-Plasmiden, auf denen drei unterschiedliche Chaperon-Gene auf einem Vektor vorliegen. Nähere Beschreibungen der Abbildung erfolgen im Text. Die einzelnen Klonierungsschritte werden nachfolgend detailliert erläutert.

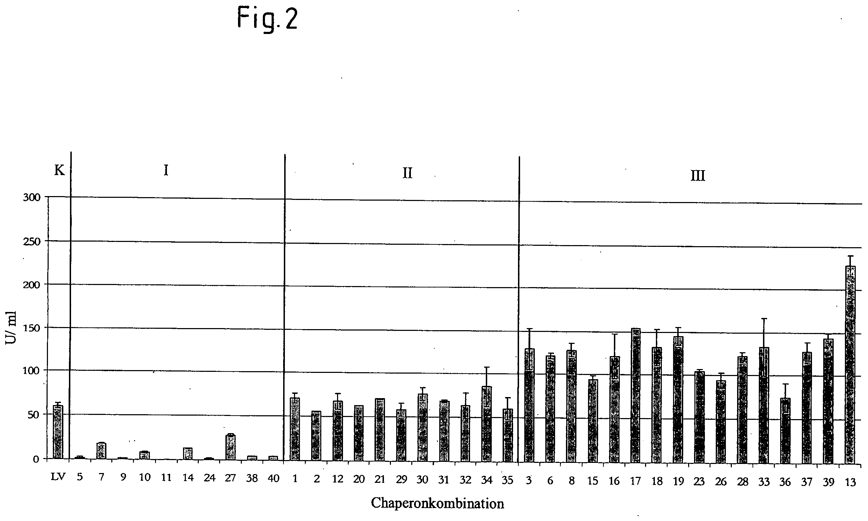

**Fig. 2****: Einfluß von *P. aeruginosa* Faltungshelfern auf die Aktivität der löslichen *R. erythropolis* ADH in** ***E. coli.*** Der Nachweis der Enzymaktivität erfolgte durch Einsatz von 10 µl Protein der löslichen Proteinfraktion, die auf gleiche Zelldichten eingestellt war. Die Aktivität ist in U/ ml angegeben und die Standardabweichung aus drei voneinander unabhängigen Messungen durch Fehlerbalken dargestellt. Die Stämme sind durch die Angabe der *Multisite*-Plasmid-Nummer benannt (s. Tab. 6). Durch die Unterteilung in Ergebnisgruppen (K = Kontrolle, bzw. Gruppe I-III) werden Stämme zusammengefaßt, die vergleichbare ADH-Aktivitäten aufweisen (LV = Leervektor, d.h. ein Stamm mit RE-ADH-Vektor und dem Vektor, der in den übrigen Stämmen jeweils Gene für Faltungshelferproteine trägt, im Fall des LV-Stamms keine solche Gene trägt).

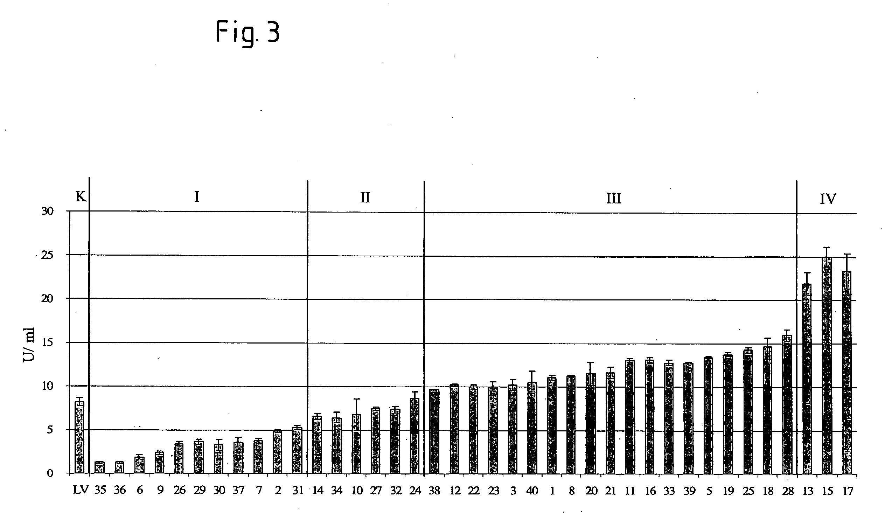

**Fig. 3****: Einfluß von einzelnen *P. aeruginosa* Faltungshelfern auf die Aktivität der *R. ruber* ADH in *E. coli.*** Nach Aufschluß und Fraktionierung der Proteinproben wurde jeweils die Aktivität im photometrischen Test gemessen. Die Aktivität ist in U/ml angegeben. Dargestellt sind Mittelwerte aus drei voneinander unabhängigen Messungen, Standardabweichungen sind durch Fehlerbalken angegeben. Die in den jeweiligen Stämmen enthaltenen Faltungshelfer sind angegeben. Durch die Unterteilung in Ergebnisgruppen (K = Kontrolle, bzw. Gruppe I-IV) werden Stämme zusammengefaßt, die vergleichbare ADH-Aktivitäten aufweisen.

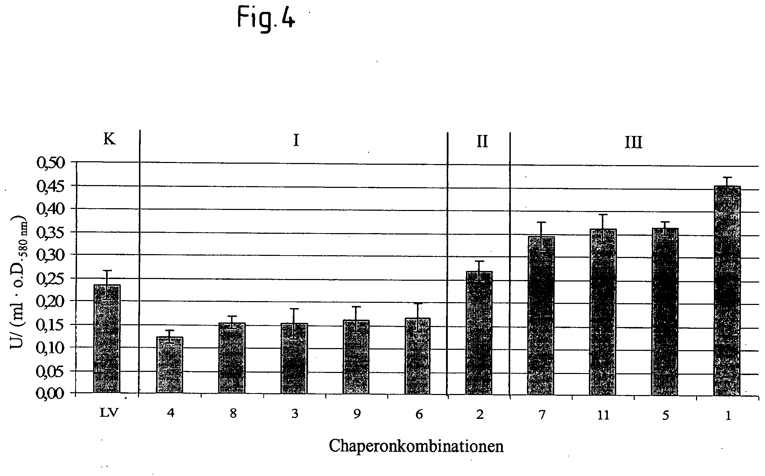

**Fig. 4****: Bestimmung der Esteraseaktivität der katalytisch aktiven EstAα-Domäne aus *P. aeruginosa* nach Coexpression mit verschiedenen Kombinationen von Faltungshelferproteinen.** Die Aktivität der EstAα-Domäne der Esterase wurde in der löslichen Fraktion der unterschiedlichen Expressionsstämme bestimmt. Als Kontrolle diente der Stamm mit EstAα und Chaperon-Leervektor (LV). Die Werte der Aktivitäten sind Mittelwerte aus drei unabhängigen Messungen. Abhängig von der ermittelten Aktivität wurden die Stämme in Ergebnisgruppen unterteilt (K = Kontrolle; I-III). Die in den Expressionsstämmen enthaltenen *Multisite*-Expressionsplasmide sind als Ziffern angegeben; die darin enthaltenen Chaperone sind in Tab. 6 aufgeführt.

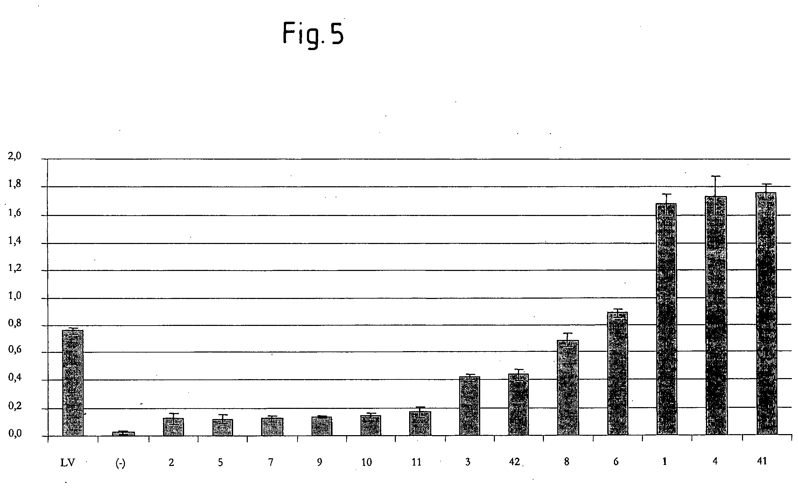

**Fig. 5****: Einfluß der Coexpression von verschiedenen Faltungshelferkombinationen auf die Löslichkeit und Aktivität des LipA Proteins aus S. *marcescens.*** Die Aktivität **(A)** des löslichen LipA-Proteins ist für alle untersuchten Stämme in nkat/OD (580 nm) angegeben. Die Werte stammen aus drei unabhängigen Messungen, anhand derer die Standardabweichungen berechnet und in Form von Fehlerbalken angegeben wurden. Als Kontrolle dienten der Stamm mit Lipase-Expressionsplasmid und Chaperon-Leervektor (LV) sowie der Stamm BL21(DE3) mit beiden Leervektoren (-). Die Nummer der Plasmide mit Kombinationen aus Chaperonen und Foldasen ist angegeben (s. Tabelle 6). In einer durchgeführten SDS-PAGE sind 10 µl von GZE-Proben aufgetragen. Deutlicher wird die Menge an LipA-Protein (60 kDa) in einem durchgeführten Zymogrammgel (Daten nicht gezeigt).
   Durch Coexpression von Faltungshelfern zusammen mit dem Zielprotein LipA konnten deutliche Unterschiede zu dem Kontrollstamm BL21 (DE3)-LV aufgezeigt werden. Ausschließlich durch zwei Kombinationen von Chaperonen und Faltungskatalysatoren, den Kombinationen 6 und 8 (s. Tab. 6), wurde die Aktivität der Lipase nicht beeinflußt (Fig. 5, **A:** Gruppe II). Bei einer Anzahl von acht Coexpressionsstämmen führte die Expression der jeweiligen Faltungsenzyme zu einer verringerten Produktion aktiven LipA-Proteins (Fig. 5; **A:** Gruppe I). Mit Hilfe von drei Kombinationen von Faltungshelfern aus *P*. *aeruginosa* konnte eine Verbesserung der Aktivität festgestellt werden. Bei den Expressionsstämmen, in denen die Plasmide pEXP-1, pEXP-4 bzw. pEXP-41 vorlagen, konnte die Aktivität der Lipase LipA im photometrischen Enzymtest um 125 % gesteigert werden (Fig. 5; **A:** Gruppe III).

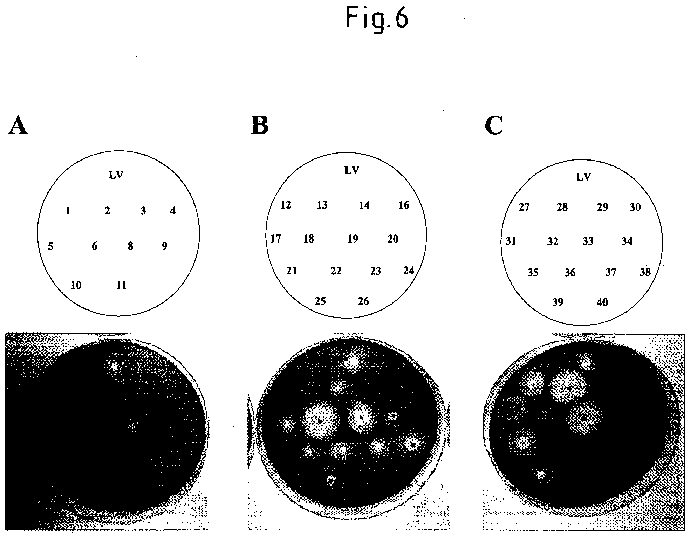

**Fig. 6****: Bestimmung der lipolytischen Aktivität von CALB durch Umsatz des Substrates Tributyrin auf Agarplatten.** Die erzeugten Expressionsstämme wurden auf Tributyrin-haltige Platten gepickt und über Nacht bei 30°C inkubiert. Die Aktivität wird durch Bildung eines Hofes um die Kolonie herum detektiert. Die Ergebnisse konnten in zwei unabhängigen Anzuchten verifiziert werden. Im oberen Teil der Abbildung sind die Nummern der Chaperonkombinationen aufgeführt, die in den Stämmen vorlagen, die auf die Tributyrinplatten gepickt wurden (unterer Teil der Abbildung). Die in den Teststämmen enthaltenen Faltungsmodulatoren sind durch die Nummern der Expressionsplasmide angegeben (s. Tab. 6).

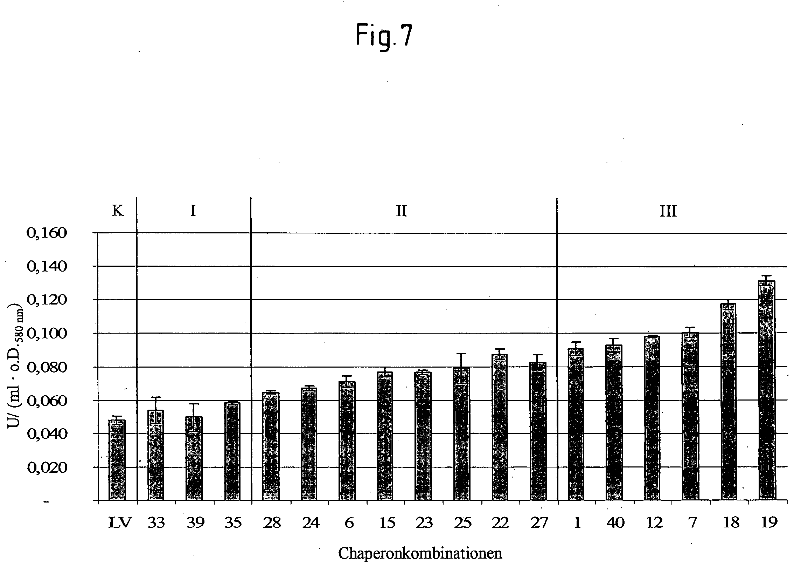

**Fig. 7****: Nachweis der Aktivität löslichen CALB-Proteins nach einer Überexpression in Flüssigmedium.** Die gemessene Aktivität ausgewählter Expressionsstämme ist in U/ ml angegeben. Die in den Stämmen vorliegenden Chaperonkombinationen sind durch die aufgeführten Zahlen unter dem Graphen genannt (s. Tab. 6). Die Daten sind Mittelwerte aus drei unabhängigen Messungen. Die Standardabweichung ist durch Fehlerbalken angezeigt. Je nach Verhältnis der Enzymaktivität der Teststämme zur Kontrolle (K) wurden sie in Ergebnisgruppen zusammengefaßt (l-lll).

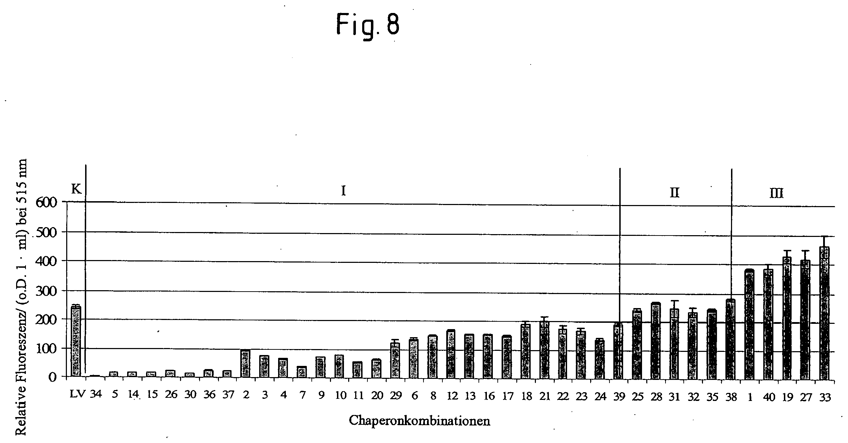

**Fig. 8****: Einfluß von *P. aeruginosa* Faltungshelfern auf die relative Fluoreszenz des Faltungsreporters GFP.** Die relative Fluoreszenz des GFP wurde nach Anregung bei 475 nm pro 1 OD und ml Probe bei einer Emissionswellenlänge von 515 nm bestimmt. Die Standardabweichungen aus zwei voneinander unabhängigen Messungen sind durch Fehlerbalken angegeben. Die in den Teststämmen enthaltenen pEXP-Plasmide sind durch die Nummern angegeben (s. Tab. 6).

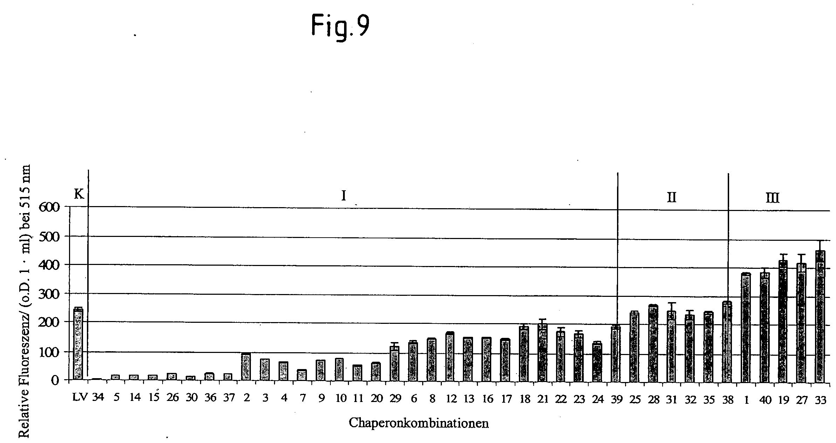

**Fig. 9****: Einfluß von *P. aeruginosa* Faltungshelfern auf die relative Fluoreszenz des Faltungsreporters GFP.** Die relative Fluoreszenz des GFP wurde nach Anregung bei 475 nm pro o.D. 1 und ml Probe bei einer Emissionswellenlänge von 515 nm bestimmt. Die Standardabweichungen aus zwei voneinander unabhängigen Messungen sind durch Fehlerbalken angegeben. Die in den Teststämmen enthaltenen pEXP-Plasmide sind durch die Nummern angegeben (s. Tab. 6).

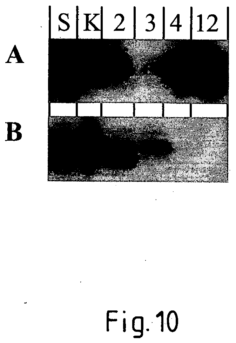

**Fig. 10****: Einfluß von Chaperonen und Faltungskatalysatoren aus *P. aeruginosa* auf die Stabilität des humanen IF2αb-Proteins.** Zur Bestimmung der Lokalisation und Menge des produzierten Zielproteins wurden Proteinproben aus dem Kulturüberstand (A) und aus GZE (B) in einem Western-Blot unter Verwendung eines spezifischen Antikörpers detektiert. Als Kontrolle wurde Standardprotein (0,05 µg; S) bzw. der IFN2αb-Expressionsstamm mit Chaperon-Leervektor (K) aufgetragen. Die coexprimierten Chaperonkombinationen sind durch Nummern gekennzeichnet (s. Tab. 6).

Es wurde überraschend entdeckt, dass Kombinationen von zwei, drei oder mehreren Faltungshelferproteinen (Chaperonen) zu einer signifikant verbesserten Expression eines Zielproteins führen können, auch wenn es sich nicht um natürlicherweise vorkommende Kombinationen handelt. Kombinationen bedeutet in diesem Fall, dass mehrere auf willkürlichem kombinatorischem Wege erzeugte für Faltungshelfer codierende Gene, beispielsweise zwei oder drei, in einer Zelle gemeinsam mit dem Gen für ein Zielprotein coexprimiert werden und zusammenwirken. Das erfindungsgemäße Verfahren, die Konstrukte und Wirtszellen sind geeignet für die Produktion von Zielproteinen in Standard-Kultursystemen in kleinem Maßstab sowie für Produktionssysteme in großem Maßstab, einschließlich und nicht begrenzt auf Fermentationssysteme, Hohlfaserkultursysteme, Becher- und Kolbensysteme und Suspensionskultursysteme.

Die notwendigen molekularbiologischen Arbeitsmethoden sind dem Fachmann gut bekannt und in einer Vielzahl von Laborhandbüchern und Texten nachzulesen, also Protokolle und Verfahren für die Manipulation von Nukleinsäuren, PCR-Amplifikation von Nucleinsäuren, Konstruktion von Expressionsvektoren, die Transformation von Wirtszellen und die Vermehrung transformierter Zellen für die Produktion von Proteinen; siehe beispielsweise das Standard-Klonierungshandbuch von Sambrook [7].

Die nachfolgenden Beispiele veranschaulichen die Erfindung.

### Beispiel 1

### Identifizierung von Chaperonen (Faltungshelferproteinen) in Pseudomonas aeruginosa

Um für die Konstruktion einer Expressionsbank von Kombinationen verschiedenster Faltungshelferproteinen eine möglichst große Heterogenität solcher Proteine einsetzen zu können, sollten beispielhaft für die Vorgehensweise alle bekannten definierten und alle putativen Sequenzen eines Mikroorganismus isoliert und eingesetzt werden. Seit dem Jahr 2000 ist das 6,33 Mbp große Genom des Bakteriums *P. aeruginosa* vollständig sequenziert, die Sequenzdaten sind unter www.pseudomonas.com verfügbar [8]. Diese Datenbank umfaßt 5.570 annotierte Leserahmen (open reading frames) und wurde für die Sequenzanalyse von Faltungshelferproteinen zugrunde gelegt. Dabei wurde die Möglichkeit genutzt, in der Pseudomonas-Datenbank Funktionssuchen durchführen zu können. So wurde eine umfassende Datenbankanalyse zur Identifikation von Chaperonen, Hitzeschockproteinen, Peptidyl-Prolyl cis-trans Isomerasen (PPlasen) und Disulfidbrücken-bildende-Proteine (Dsb-Proteine) durchgeführt, wobei eine große Anzahl von Genen identifiziert wurden, deren Genprodukte hohe Sequenzübereinstimmungen zu bereits beschriebenen, bekannten Faltungshelfern aus anderen Bakterien aufweisen. Anschließend wurden Sequenzvergleiche mit der von der DNA-Sequenz abgeleiteten Aminosäuresequenz des jeweiligen *P. aeruginosa* Faltungshelfers durchgeführt, um die erhaltenen Ergebnisse zu verifizieren. Die auf diese Weise erhaltenen Gene sind in Tabelle 1 aufgeführt.

Zusätzlich zu den molekularen Chaperonen, den Hitzeschockproteinen oder auch den PPlasen, die bereits in *P. aeruginosa* annotiert sind, konnten in im Rahmen dieser Erfindung weitere Leserahmen gefunden werden, die bisher lediglich als putative Faltungshelferproteine in der Pseudomonas-Datenbank geführt wurden. Nach einem Homologievergleich mit ihren ermittelten Aminosäuresequenzen konnte mit Hilfe einer Funktion, die die Suche nach Proteindomänen erlaubt, weiter untersucht werden, welche solcher Domänen, die für Chaperone und Hitzeschockproteine charakteristisch sind, diese putativen Proteine enthalten.

Auf diese Weise konnten sieben weitere Gene für molekulare Chaperone bzw. Hitzeschockproteine identifiziert werden, welche in die vorgesehene Chaperon-Coexpressionsbank aufgenommen wurden. Bei diesen Kandidaten sind unterschiedliche Proteingruppen der molekularen Chaperone vertreten. So besitzt das putative Protein PA1068 eine Hsp90-ATPase-Domäne [9], die auch im HtpG-Protein aus *E. coli* vorliegt. Zwei weitere orfs (PA2725 und PA3365) weisen eine AAA- (= ATPase associated with various cellular activities) Domäne in ihrer abgeleiteten Proteinsequenz auf [10, 11], die in verschiedenen Hitzeschockproteinen vorkommt. Für den Leserahmen PA3647 konnte gezeigt werden, daß dieser eine OmpH (outer membrane protein like)-ähnliche Domäne besitzt. Mit diesem Kandidaten konnte ein homologes Protein zu dem periplasmatischen Chaperon Skp aus *E. coli* identifiziert werden, das in diesem Organismus als einziges echtes periplasmatisches Chaperon beschrieben ist [12, 13]. Dort ist dieses Chaperon an der Reifung von Proteinen der äußeren Membran beteiligt [14]. Auch das putative Protein PA3842 weist eine Homologie von 56 % zu einem bereits in *Y. pestis* beschriebenen Chaperon auf [15]. Mit dem putativen Protein PA4873 konnte ein mögliches Chaperon identifiziert werden, das eine Hsp70-Domäne enthält [16], und eine Ähnlichkeit von 50 % zu dem DnaK-Protein aus *R. capsulatus* besitzt. Damit konnte neben dem in einem Operon codierten DnaK-Protein (orf PA4761) ein zweiter Vertreter der Hsp70-Familie in *P. aeruginosa* ermittelt werden. Schließlich konnte mit PA5195 ein putatives homologes Protein zu dem *E. coli* Protein YrfH identifiziert werden.

Ferner konnten fünf weitere Leserahmen in *P. aeruginosa* gefunden werden, die als Proteine der Peptidyl-Prolyl cis-trans Isomerasen (PPlasen) angesehen werden. In den meisten Fällen konnte eine PPlase-Domäne gefunden werden, die für den FKBP-Typ (FK506-binding protein) dieser Enzyme charakteristisch ist [17, 18]. Hierzu zählen die durch die orfs PA3262, PA3717, PA4558 und PA5254 codierten Proteine. Zwei weitere Gene konnten über die Möglichkeit, Proteindomänen zu identifizieren, gefunden werden, deren Genprodukte eine Rotamase-Domäne aufweisen [17]. Dies sind die orfs PA0699 und PA3871, die beide Homologien zu bereits beschriebenen und charakterisierten Proteinen aus anderen Organismen aufweisen. Diese Gene für putative Chaperone sind in Tabelle 2 aufgeführt.

Die für das Dsb-System notwendigen Proteine [19-21] konnten alle an Hand annotierter Sequenzen identifiziert werden.

Schließlich muß noch eine Enzymgruppe erwähnt werden, deren Mitglieder an der richtigen Faltung und Herstellung von Proteinen beteiligt sind, die zum Bewegungsapparat von *P. aeruginosa* gehören. Zu dieser Gruppe können vier Kandidaten gezählt werden. Hierbei handelt es sich um die Chaperone CupA2, CupA5 sowie CupB2 und CupB4. Auch für diese Gruppe konnte mit PA0499 zusätzlich ein Leserahmen identifiziert werden, dessen putatives Genprodukt eine für pili assembly chaperones charakteristische Domäne enthält [22]. Die entsprechenden Gene wurden ebenfalls in die Chaperon-Toolbox aufgenommen.

Insgesamt konnten somit 58 unterschiedliche Leserahmen identifiziert werden, deren DNA-Sequenzen im Anhang zu finden sind. Unter den identifizierten Leserahmen befinden sich 53 einzelne Faltungshelfergene sowie 5 Operons.

**Tabelle 1: Annotierte Faltungshelferproteine in Pseudomonas aeruginosa. Die aufgeführten Gene wurden durch Sequenzvergleiche identifiziert. Sie gehören zur Gruppe der Heatschockproteine, Peptidyl-Prolyl cis-trans Isomerasen, und der Disulfidbrückenbildenden (Dsb) Proteine, welche insgesamt als Chaperone bezeichnet werden. Bei diesen bereits annotierten Open-reading-frames (orf) ist der Genname mit angegeben. Außerdem sind die Größen der identifizierten Gene [bp] angegeben. Die Homologien gegenüber bekannten Proteinen [%] und der Ursprungsorganismus sind genannt.**

| ***orf*-Nummer** | **Genname** | **Gengröße [bp]** | **Ähnlichkeit [%]** |
|---|---|---|---|
| **Annotierte Chaperone und Heatschockproteine** | | | |
| PA1596 | *htpG* | 1.905 | 78% zu HtpG aus *E. coli* |
| PA1801 | *clpP* | 642 | 86% zu ClpP aus *E. coli* |
| PA1802 | *clpX* | 1.281 | 85% zu ClpX aus *E*. *coli* |
| PA2614 | *lolA* | 627 | 51% zu LolA aus *E. coli* |
| PA2863 | *lipH* | 1.023 | 57% zu LipH aus *Pseudomonas mendocina* |
| PA3126 | *ibpA* | 450 | 67% zu IbpA aus *E. coli* |
| PA3221 | *csaA* | 336 | 68% zu CsaA aus *B. subtilis* |
| PA3810 | *hscA* | 1.860 | 87% zu HscA aus *E*. *coli* |
| PA3811 | *hscB* | 522 | 80% zu HscB aus *Azotobacter vinelandii* |
| PA4385 | *groEL* | 1.644 | 95% zu GroEL aus *A. vinelandii* |
| PA4386 | *groES* | 294 | 96% zu GroES aus *A. vinelandii* |
| PA4760 | *dnaJ* | 1.134 | 78% zu DnaJ aus *E*. *coli* |
| PA4761 | *dnaK* | 1.914 | 87% zu DnaK aus *E*. *coli* |
| PA4762 | *grpE* | 561 | 63% zu GrpE aus *E. coli* |
| PA5053 | *hslV* | 534 | 100% zu HslV aus *E*. *coli* |
| PA5054 | *hslU* | 1.344 | 54% zu HslU aus *E*. *coli* |
| PA5128 | *secB* | 489 | 71% zu SecB aus *E. coli* |
| PA5193 | *yrfl* | 894 | 62% zu Yrfl aus *E*. *coli* |
| | | | |

| **Annotierte Peptidyl-Prolyl cis-trans Isomerasen** | | | |
|---|---|---|---|
| PA0594 | *surA* | 1.293 | 60% zu SurA aus *E*. *coli* |
| PA0837 | *slyD* | 486 | 65% zu SlyD aus *E*. *coli* |
| PA1793 | *ppiB* | 498 | 77% zu CypB aus *E. coli* |
| PA1800 | *tig* | 1.311 | 72% zu TF aus *E. coli* |
| PA1805 | *ppiD* | 1.866 | 51% zu PPlase D aus *E. coli* |
| PA1996 | *ppiC1* | 279 | 68% zu PPlase C aus *E*. *coli* |
| PA3227 | *ppiA* | 564 | 73% zu PPlase A aus *E. coli* |
| PA4176 | *ppiC2* | 282 | 67% zu PPlase C aus *E*. *coli* |
| PA4572 | *fklB* | 618 | 67% zu FklB aus *E. coli* |
| | | | |

| **Annotierte Dsb-Proteine** | | | |
|---|---|---|---|
| PA0538 | *dsbB* | 510 | 51% zu DsbB aus *E*. *coli* |
| PA1481 | *ccmG* | 543 | 67% zu DsbE aus *E. coli* |
| PA2476 | *dsbG* | 771 | 71 % zu DsbG aus *E*. *coli* |
| PA3737 | *dsbC* | 729 | 58% zu DsbC aus *E*. *coli* |
| PA4845 | *dsbD* | 1.776 | 94% zu DipZ aus *P. aeruginosa* |
| PA5256 | *dsbH* | 492 | 51% zu DsbH aus *Burkholderia cepacia* |
| PA5489 | *dsbA* | 636 | 82% zu DsbA aus *A. vinelandii* |

**Tabelle 2: Putative Chaperone in Pseudomonas aeruginosa. Über spezifische Domänen in der Proteinsequenz konnten weitere Gene identifiziert werden, die als Kandidaten für klassische Heatschockproteine und Peptidyl-Prolyl cis-trans Isomerasen (PPlasen) angesehen werden können. Neben der Nummer des orf ist die Größe des Gens [bp] angegeben. Die für die jeweiligen Faltungshelferproteine charakteristische Domäne ist ebenfalls angeführt.**

| ***orf-*Nummer** | **Gengröße [bp]** | **identifizierte Proteindomäne** | **Ähnlichkeit** [**%**] |
|---|---|---|---|
| **Putative Chaperone und Hitzeschockproteine** | | | |
| **PA1068** | 1.911 | Hsp90-ATPase | 47% zu HtpG aus *E*. *coli* |
| **PA2725** | 1.884 | AAA-Familie | 51% zu Hsp100-Region aus *Trypanomsoma brucei* |
| **PA3365** | 1.116 | AAA-Familie | 99% zu AmiB aus *P. aeruginosa* |
| **PA3647** | 507 | OmpH-Domäne | 48% zu Skp aus *E*. *coli* |
| **PA3842** | 351 | CesT-Chaperon Domäne | 56% zu Chapeon aus *Yersinis pestis* |
| **PA4873** | 1.266 | Hsp70-Domäne | 50% zu DnaK aus *Rhodobacter capsulatus* |
| **PA5195** | 396 | S4-Domäne | 67% zu YrfH aus *E. coli* |
| | | | |

| **Putative Peptidyl-Prolyl *cis-trans* Isomerasen** | | | |
|---|---|---|---|
| **PA0699** | 945 | Rotamase-Domäne | 48% zu PPlase-Domäne aus *Acinetobacter sp*. |
| **PA3262** | 762 | FKBP-Typ PPlase-Domäne | 62 % zu *fkpB*-Gen aus *E. coli* |
| **PA3717** | 342 | FKBP-Typ PPlase-Domäne | 66% zu Rotamase aus *Neisseria meningitidis* |
| **PA3871** | 819 | Rotamase-Domäne | 47% zu NifM aus *Azotobacter croococcum* |
| **PA4558** | 441 | FKBP-Typ PPlase-Domäne | 63% zu FKBP-Typ PPlase aus *E*. *coli* |
| **PA5254** | 630 | FKBP-Typ PPlase-Domäne | 51% zu FklB aus *E. coli* |

### Beispiel 2

### Amplifikation der identifizierten Gene

Nach Identifikation der annotierten und putativen Chaperon-Gene in *P. aeruginosa* wurden diese aus dem Chromosom des Wildtyp Stammes PAO1 isoliert werden. Dazu wurden diese Gene mittels PCR amplifiziert. Die Oligonukleotide zur Amplifikation sämtlicher Gene wurden so gewählt, daß ein DNA-Fragment erzeugt werden konnte, welches ein Gen vom Start- bis zum Stop-Codon enthielt. Außerdem wurde an das 5'-Ende der Primer, die an den stromaufwärts gelegenen DNA-Bereich binden, eine Topoisomerase-Erkennungssequenz aus den vier Basen CACC angefügt, wodurch eine spätere EinSchritt-Klonierung mit dem TOPO-Klonierungskit der Firma Invitrogen (Karlsruhe) ermöglicht werden sollte.

Um die unterschiedlichen Gene und die entsprechenden Operons der Chaperonsysteme zu amplifizieren, wurden Polymerasekettenreationen durchgeführt. Dazu wurden für jedes notwendige PCR-Produkt mehrere parallele Reaktionen angesetzt, zudem wurden in verschiedenen Ansätzen unter anderem die Annealingtemperatur, die Elongationszeit oder die Zyklusanzahl variiert. Im Anschluß an die durchgeführten Reaktionen wurde für jedes PCR-Produkt und jede Reaktionsbedingung ein Aliquot von 5 µl des Reaktionsansatzes auf ein Agarosegel aufgetragen und somit die Größe und DNA-Menge bestimmt.

Schließlich konnten insgesamt 55 Polymerasekettenreaktionen erfolgreich durchgeführt und die PCR-Produkte in die nachfolgende Klonierungsreaktion eingesetzt werden.

### Beispiel 3

### Klonierung der Chaperon-Gene

Zur Klonierung der entsprechend Beispiel 2 erzeugten PCR-Produkte in einen geeigneten Klonierungsvektor kann das hier näher beschriebene "pENTR® Directional TOPO® Cloning Kit" (Invitrogen, Karlsruhe) verwendet werden, es sind aber auch andere Systeme genauso geeignet.

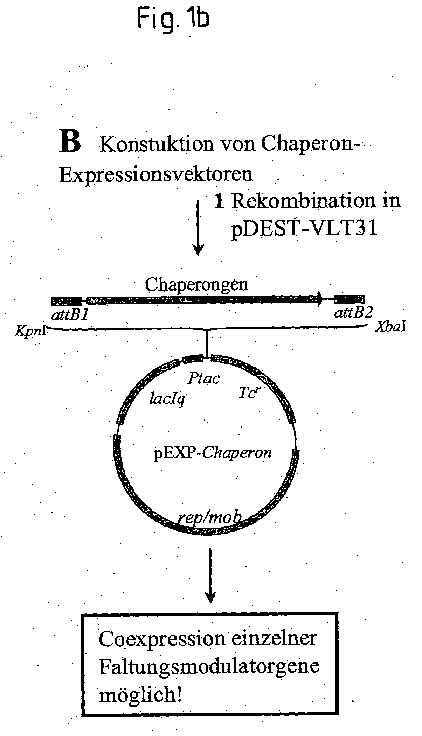

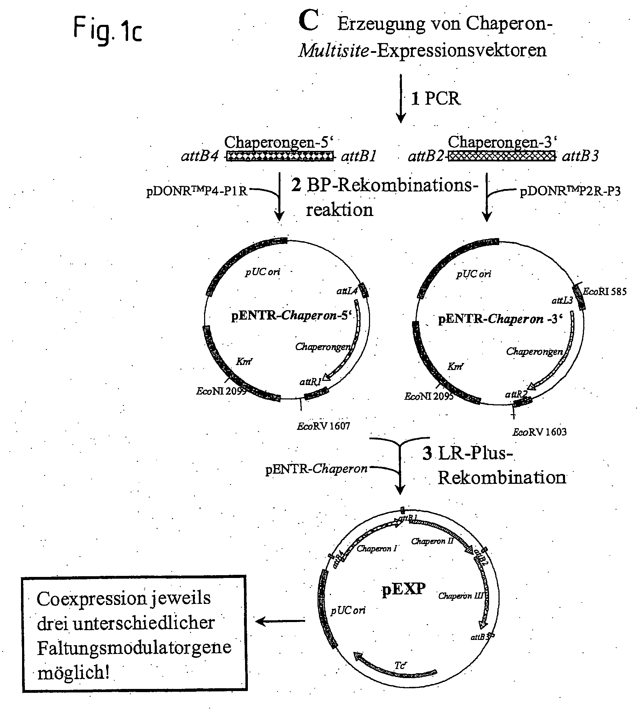

Die hier beschriebene Methode basiert auf dem Einsatz des Enzyms Topoisomerase I aus dem Virus Vaccinia, das einen der beiden Stränge der doppelsträngigen DNA an spezifischen Stellen öffnen kann [23]. Mit Hilfe dieses Einschritt-Klonierungs-Systems wurden die erhaltenen PCR-Produkte in den Vektor pENTR/SD/D-TOPO eingebracht. Dieser Vektor besitzt neben den Rekombinationsstellen attL1 und attL2 ein Kanamycin-Resistenzgen, eine für Bakterien geeignete Ribosomenbindestelle (RBS) sowie einen ColE1-Replikationsursprung, welcher eine Replikation mit hoher Kopienzahl in *E. coli* ermöglicht. Eine schematische Darstellung der durchgeführten Klonierung ist in Fig. 1 (A.2) gezeigt. Auf diese Weise wurden alle erzeugten PCR-Produkte in den Zielvektor pENTR/SD/D-TOPO eingebracht und zur Bestätigung sequenziert. Die hergestellten Plasmide wurden als pENTR- mit dem jeweiligen Namen des einklonierten Faltungshelfergens (Chaperon-Gens) bezeichnet, zum Beispiel pENTR-tig. In Tabelle 3 sind unter "pENTR-" die erfolgreich hergestellten pENTR-Plasmide durch ein Kreuz (X) markiert.

**Tabelle 3: Zusammenstellung der erfolgreich erzeugten pENTR-Plasmide. Die für die weiteren Experimente vorliegenden Plasmide sind durch ein X gekennzeichnet.**

| **orf** | **Name** | **pENTR-** |
|---|---|---|
| PA0499 | | **X** |
| PA0594 | *surA* | **X** |
| PA0699 | | **X** |
| PA0837 | *slyD* | **X** |
| PA1068 | | **X** |
| PA1481 | *ccmG* | **X** |
| PA1596 | *htpG* | **X** |
| PA1793 | *ppiB* | **X** |
| PA1800 | *tig* | **X** |
| PA1802 | *clpX* | **X** |
| PA1805 | *ppiD* | **X** |
| PA1996 | *ppiC1* | **X** |
| PA2129 | *cupA2* | **X** |
| PA2132 | *cupA5* | **X** |
| PA2476 | *dsbG* | **X** |
| PA2614 | *lolA* | **X** |
| PA2725 | | **X** |
| PA2863 | *lipH* | **X** |
| PA3126 | *ibpA* | **X** |
| PA3221 | *csaA* | **X** |
| PA3227 | *ppiA* | **X** |
| PA3262 | | **X** |
| PA3365 | | **X** |
| PA3647 | *skp* | **X** |
| PA3717 | | **X** |
| PA3737 | *dsbC* | **X** |
| PA3810 | *hscA* | **X** |
| PA3811 | *hscB* | **X** |
| PA3810&11 | *hscAB* | **X** |
| PA3842 | | **X** |
| PA3871 | | **X** |
| PA4083 | *cupB4* | **X** |
| PA4085 | *cupB2* | **X** |
| PA4176 | *ppiC2* | **X** |
| PA4385 | *groEL* | **X** |
| PA4386 | *groES* | **X** |
| PA4385&86 | *groELS* | **X** |
| PA4558 | | **X** |
| PA4572 | *fklB* | **X** |
| PA4651 | | **X** |
| PA4760 | *dnaJ* | **X** |
| PA4761 | *dnaK* | **X** |
| PA4760&61 | *dnaKJ* | **X** |
| PA4762 | *grpE* | **X** |
| PA4760-62 | *grpE-dnaKJ* | **X** |
| PA4873 | | **X** |
| PA5053 | *hslV* | **X** |
| PA5054 | *hslU* | **X** |
| PA5053&54 | *hslVU* | **X** |
| PA5128 | *secB* | **X** |
| PA5193 | *yrfl* | **X** |
| PA5195 | | **X** |
| PA5254 | | **X** |
| PA5256 | *dsbH* | **X** |
| PA5489 | *dsbA* | **X** |

### Beispiel 4

### Konstruktion von Expressionsplasmiden

Im Anschluß an die erfolgreiche Klonierung der Chaperon-Gene in den pENTR-Vektor konnten die erzeugten Plasmide dazu verwendet werden, Expressionsvektoren herzustellen. Dabei kann beispielsweise die "Gateway®"-Technologie der Firma Invitrogen (Karlsruhe) angewandt werden, ebenso können aber auch eine Reihe anderer Systeme für diesen Zweck eingesetzt werden. Dieses System beruht auf Mechanismen der homologe Rekombination, die für den Bakteriophagen Lambda gut beschrieben sind [24].

Um diese Klonierungsmethode anwenden zu können, mußte in den Zielvektor eine Destination-Kassette eingebracht wurde. Diese Kassette konnte aus dem Vektor pGATRfA (s. Tab. 4) isoliert und über die Restriktionsschnittstellen Kpnl und Xbal in den Vektor pVLT31 (s. Tab. 4) einkloniert werden. Das daraus resultierende Plasmid wird im Folgenden als pDEST-VLT31 bezeichnet. Der Expressionsvektor pVLT31 wurde für diese Anwendung ausgewählt, da er sowohl einen induzierbaren tac-Promotor besitzt, als auch ein Shuttlevektor ist, der in *E. coli* ebenso wie in *P. aeruginosa* und weiteren Gramnegativen Bakterien stabil repliziert werden kann. So kann die Chaperon-Toolbox später in einem breiten Spektrum von Expressionsstämmen eingesetzt werden.

Durch die eingebrachte Destination-Kassette erhält der Vektor zu seiner Tetracyclin-Resistenz (Tc^{r}) eine zusätzliche Chloramphenicol-Resistenz (Cm^{r}) und das Gen ccdB, das für einen Gyrasehemmer codiert [25, 26]. Flankierend besitzt dieses DNA-Fragment die beiden Rekombinationsstellen attR1 und attR2. Für die Konstruktion des pDEST-VLT31-Vektors mußte der *E. coli* Stamm DB3.1 verwendet werden. Dieser kann trotz Expression des ccdB-Gens überleben, da durch eine Mutation in dem ccdA-Gen, das für die Topoisomerase II codiert, das CcdB-Protein nicht wirken kann. Nachdem der Zielvektor pDEST-VLT31 erfolgreich hergestellt werden konnte, wurden Rekombinationsreaktionen mit den pENTR-Plasmiden (s. Beispiel 3) durchgeführt.

Im Anschluß an die Reaktion wurde ein Aliquot der Ansätze in den Stamm *E*. *coli* TOP10 transformiert und die erhaltenen Klone weiter untersucht. Da die Expression des ccdB-Gens für TOP10-Zellen letal ist, wird eine positive Selektion dieser Rekombination ermöglicht. Um falsch-positive Klone auszuschließen, wurde zusätzlich auf den Verlust der Cm-Resistenz selektiert, bevor die Plasmid-DNA isoliert und in Restriktionsanalysen mit den Enzymen Kpnl und Xbal untersucht wurde.

So konnten 47 Expressionsplasmide konstruiert werden, die einzelne Gene oder Operons von Faltungshelfern aus *P. aeruginosa* enthielten.

**Tabelle 4: Übersicht der verwendeten Vektoren.**

| **Plasmide** | **genetische Eigenschaften** | **Referenz/ Quelle** |
|---|---|---|
| **Vektoren für *E. coli*** | | |
| **pDEST™ R4-R3** | Ap^{r}, Cm^{r}, *ccdB*, *att*R4, *att*R3, | Invitrogen, Karlsruhe |
| **pDONR™ P4-P1R** | Km^{r}, Cm^{r}, *ccdB*, *att*P4, *aff*P1R | Invitrogen, Karlsruhe |
| **pDONR™ P2R-P3** | Km^{r}, Cm^{r}, *ccdB*, attP2R, *att*P3 | Invitrogen, Karlsruhe |
| **pENTR/SD/D-TOPO** | Km^{r}, RBS, *att*L1, *att*L2 | Invitrogen, Karlsruhe |
| **pET16b** | P_{T7} P_{lac} Ap^{r} ColE1, His-Tag | NOVAGEN, Madison, USA |
| **pET22b (+)** | P_{T7Φ10}Ap^{r} ColE1 pelB *lacl*^{q} | NOVAGEN, Madison, USA |
| **pGATRfA** | Cm^{r} Ap^{r} *ccdB att*R1 *att*R2 | Invitrogen, Karlsruhe |
| **Vektoren mit breitem Wirtsspektrum** | | |
| **pBBR1 MCS** | P_{lac} P_{T7} Cm^{r} *mob lacZα* | [27] |
| **pBBR1 MCS-3** | P_{lac} P_{T7} *lacZα* Tc^{r} *mob rep* | [28] |
| **pSWgfp** | pTZ110 mit promotorlosem *gfp* | [29] |
| **pUCPKS** | P_{lac} P_{T7} Apr ColE1-SF *lacZα* | [30] |
| **pVLT31** | *lacl^{q}*, *P_{tac}*, Tc^{r}, *mob, rep* | [31] |
| **rekombinante Plasmide** | | |
| **pBaBIFN1** | P_{tac} Apr Tc^{r} *IF2α*ColE1 | Fa. Wacker, München |
| **pBaBIFN1ΔTc^{r}** | P_{tac} Apr ΔTc^{r} (*Sal*I/ *Nhe*I) *IF2α* ColE1 | diese Arbeit |
| **pBBL7** | pBBR1 MCS + *Xmn*I/ *Sma*I-Fragment, 2,8 kb, *lipA*/*H*, P_{lac} | [32] |
| **pDEST-BBRTc-R4R3** | 1,7 kb-*ccdB*-Kassette aus pDEST™ R4-R3 in *Sma*I-site des pBBR1 MCS-3 | diese Arbeit |
| **pDEST-VLT31** | *Kpn*I/ *Xba*I-*ccdB*-Kassette aus pGATRfA in pVLT31 | diese Arbeit |
| **pENTR-*Chaperon*** | **pENTR/SD/D-TOPO mit entspr. Chaperongen** | **diese Arbeit** |
| **pENTR-*Chaperon*-3'** | pDONR™P2R-P3 mit Chaperongen; 3'-Position im Multisite-Vektor | diese Arbeit |
| **pENTR-*Chaperon*-5'** | pDONR™P4-P1R mit Chaperongen; 5'-Position im Multisite-Vektor | diese Arbeit |
| **pET16b-SMlipA** | pET16b-Derivat; *S. marcescens lipA-*Gen in *NcoI-Bam*HI einkloniert (aus pLipRB) | [33] |
| **pET22b-CalB** | pET22b-Derivat; *C. antarctica CALB-*Gen einkloniert | [34] |
| **pET19b α-Dom (His₆)** | EstA N-Terminus in pET19b *Nde*I/ *Xho*I kloniert (N-His₆) | [35] |
| **pET22b-gfp** | pET22b-Derivat; *GFP* aus pSWgfp in *Eco*RI/ *Hin*dIII einkloniert | diese Arbeit |
| **pEXP-*Chaperon*** | pVLT31 mit einzelnem Chaperon/-system | diese Arbeit |
| **pEXP**-**1 ... 42** | pBBR1 MCS-3 mit drei Chaperongenen kombiniert | diese Arbeit |
| **pRE**-**ADH** | pKA1-Derivat, *re-adh* Gen in *Nde*I/ BamHI kloniert | [36] |
| **pRR-ADH** | pKA1-Derivat, *rr-adh* Gen über *Nde*I/ *Bam*HI kloniert | [36] |

### Beispiel 5

### Herstellung von Multisite-Expressionsplasmiden

Damit ein Zielprotein nacheinander mit mehreren Chaperonen in Kontakt kommen kann, wurden für die Toolbox Plasmide konstruiert, mit deren Hilfe die Gene von mehreren Faltungshelfern gleichzeitig in einer Zelle zusammen mit dem Zielgen coexprimiert werden können, so dass ein Zielprotein zwei, drei oder mehrere Faltungshelferproteine in der Zelle vorfindet. Dazu kann beispielsweise das "MultiSite Gateway® Three-Fragment Vector Construction Kit" der Firma Invitrogen eingesetzt werden, das nach Angaben des Herstellers eine schnelle und effiziente Klonierung von drei unterschiedlichen DNA-Fragmenten in einen gemeinsamen Vektor ermöglicht. Ebenso können aber auch andere Vektoren für diesen Zweck eingesetzt werden. Das dem Kit der Firma Invitrogen zugrunde liegende System beruht auf dem Prinzip der homologen Rekombination, das vom Bakteriophagen Lambda abgeleitet wurde. Mit Hilfe dieses Kits wurde eine Plasmidbank erzeugt, welche die Expression von jeweils drei unterschiedlichen Faltungshelfergenen erlaubt.

Mit dieser Methode erhält man einen Vektor, in dem die drei Zielgene in der gewünschten Reihenfolgen hintereinander angeordnet und lediglich durch die resultierenden attB-Stellen voneinander getrennt sind. Die erzeugten Expressionsvektoren werden als pEXP bezeichnet und erhalten fortlaufende Nummern.

In den folgenden Abschnitten 5a) und 5b) wird die Konstruktion der verschiedenen pEXP-Plasmide im Detail erläutert.

### 5a) Konstruktion der Ausgangsvektoren für Multisite-Rekombinationen

Um eine Kombination von drei verschiedenen Genen in einem Zielplasmid erzeugen zu können, mußten zunächst die notwendigen Ausgangsvektoren konstruiert werden. Dazu mußte zum einen das Expressionsplasmid pBBR1MCS-3 mit der attR4R3-Destination-Kassette versehen werden, welches dann als Zielvektor in die Rekombinationsreaktion eingesetzt werden konnte. Vorteile dieses Plasmides sind, daß es in verschiedenen Gramnegativen Bakterien repliziert werden kann, und, daß Gene so einkloniert werden können, daß ihre Expression durch einen induzierbaren lac-Promotor reguliert werden kann. Des weiteren mußte die 1.704 bp große Destination-Kassette aus dem Plasmid pDESTTMR4-R3 isoliert werden. Dazu wurden die beiden Primer ccdBattR4-up und ccdBattR3-dwn (s. Tabelle 5) verwendet. Nach erfolgreicher Aufreinigung des erhaltenen PCR-Produktes konnte dieses in die Smal-Erkennungsstelle des Shuttlevektors pBBR1MCS-3 kloniert werden. Im Anschluß daran wurde das Konstrukt in *E*. *coli* TOP10-Zellen eingebracht und positive Klone auf Tc- und Cm-haltigem LB-Medium selektioniert. Diese Klonierung war erfolgreich und in der Folge wurde mit dem Plasmid weiter gearbeitet, in das die Zielgene unter Kontrolle des lac-Promotors kloniert werden konnten; dieser Vektor wird im Weiteren pDEST-BBRTc-R4R3 genannt.

Neben dem späteren Zielvektor mußten für die angestrebten Rekombinationsreaktionen die unterschiedlichen pENTR-Vektoren erzeugt werden. Die Vektoren, welche die Gene enthalten, die nachher das mittlere Gen liefern sollten, wurden bereits erfolgreich konstruiert, sie entsprechen den pENTR-Chaperon-Konstrukten aus Beispiel 3. Bei diesen Vektoren liegen die klonierten Gene zwischen den att-Stellen L1 und L2. Für die Konstruktion der beiden weiteren pENTR-Vektoren mußten die Zielgene mit anderen Erkennungsstellen reamplifiziert werden. Um die Gene der Chaperone und Faltungskatalysatoren mit den Sequenzen für attL4 und attR1 klonieren zu können, mußten sie in den Vektor pDONRTMP4-P1 R eingebracht werden. Damit die Rekombinationsstellen attR2 und attL3 neben den Zielgenen vorlagen, mußten diese in den Vektor pDONRTMP2R-P3 kloniert werden. Für beide Klonierungen mußten die Gene mit entsprechenden Erkennungsstellen amplifiziert, aufgereinigt und über eine BP-Rekombinationsreaktion in die beiden pDONR-Vektoren eingebracht werden. Deshalb wurden Primer gewählt, die teilweise homolog zu Bereichen des bereits vorhandenen pENTR-Vektors waren und zusätzlich die für die Rekombinationsreaktion essentiellen att-Stellen aufwiesen. Für die Klonierungen der Vektoren pENTR-Chaperon-5' mußten die Sequenzen für attB4 und attB1 an das PCR-Produkt angefügt werden, um in den Vektor pDONRTMP4-P1 R eingebracht werden zu können. Für die Vektoren pENTR-Chaperon-3' waren es die attB2 und attB3 Erkennungsstellen.

Mit den Primern AttB4-5'-up und AttB1-5'-dwn konnten die PCR-Produkte amplifiziert werden, die in einer anschließenden Reaktion in den Vektor pDONRTMP4-P1 R eingebracht werden sollten. Die daraus resultierenden Plasmide wurden als pENTR-5' bezeichnet. Das Primerpaar AttB2-3'-up und AttB3-3'-dwn wurde zur Konstruktion von pENTR-3'-Plasmiden eingesetzt.

### 5b) Erzeugen von Expressionsvektoren

Mit den erzeugten pENTR-Plasmiden (s. Beispiel 5a) konnten nun verschiedene Rekombinationsreaktionen durchgeführt werden. So wurden beispielsweise die Plasmide pENTR-tig-5', pENTR-dnaKJ und pENTR-clpX-3' in gleichen molaren DNA-Mengen mit dem Vektor pDEST-BBRTc-R4R3 in die Klonierungsreaktion eingesetzt. In dieser Reaktion sollten also die Gene tig - dnaKJ - clpX hintereinander in den Vektor eingebaut werden, der als pEXP-1 bezeichnet wurde. Beim gerichteten Vorgehen konnten auf diese Weise 40 Konstrukte hergestellt werden, die eine Kombination von drei Genen bzw. Operons enthielten (s. Tab. 6).

**Tabelle 5: Übersicht über die verwendeten Oligonukleotide.**

| **Bezeichnung** | **DNA-Sequenz (in 5'→ 3' Richtung)** | **Merkmal/ Modifikation** |
|---|---|---|
| **AttB2-3'-up** | | *att*B2-Erkennungssequenz |
| **AttB3-3'-dwn** | | *att*B3-Erkennungssequenz |
| **AttB1**-**5**'-**dwn** | | *att*B1- |
| | | Erkennungssequenz |
| **AttB4-5'-up** | | *att*B4-Erkennungssequenz |
| **ccdBattR3-dwn** | CAA CTA TGT ATA ATA AAG TTG AAC | |
| **ccdBattR4-up** | CAA CTT TGT ATA GAA AAG TTG A | |
| **dwn-skp** | TAT TGT CGA CTC GCG GCG CCA | *Sal*I |
| **M13 forward** | TGT AAA ACG ACG GCC AG | |
| **M13 reverse** | CAG GAA ACA GCT ATG AC | |
| **PA-Nummer-up** | up-Primer zur Amplifizierung der *P. aeruginosa* Chaperon- und Faltungskatalysatorgene zur Klonierung in den Vektor pENTR/SD/D-TOPO | CACC-Sequenz am 5'-Ende; nötig für TOPO-Klonierung |

**Tabelle 6: Auflistung der erzeugten Multisite-Expressionsplasmide. Die Plasmide wurden mit fortlaufenden Nummern versehen (pEXP-1 - pEXP-40). Der Genname sowie die Position des Gens auf dem pEXP-Plasmid sind angegeben.**

| | Nr. | 5' | Mitte | 3' | | Nr. | 5' | Mitte | 3' |
|---|---|---|---|---|---|---|---|---|---|
| pEXP- | 1 | *tig* | *dnaKJ* | *clpX* | pEXP- | 21 | *tig* | *hslVU* | *lolA* |
| pEXP- | 2 | *tig* | *groELS* | *clpX* | pEXP- | 22 | *csaA* | *hslVU* | *lolA* |
| pEXP- | 3 | *tig* | *dnaKJ* | *ppiD* | pEXP- | 23 | *PA3647* | *hslVU* | *yrfI* |
| pEXP- | 4 | *ppiA* | *grpEdnaKJ* | *tig* | pEXP- | 24 | *PA4873* | *hslVU* | *yrfI* |
| pEXP- | 5 | *ppiA* | *groELS* | *tig* | pEXP- | 25 | *ppiA* | *hslVU* | *lol*A |
| pEXP- | 6 | *dnaJ* | *groELS* | *tig* | pEXP- | 26 | *ppiC1* | *hslVU* | *PA3717* |
| pEXP- | 7 | *dnaJ* | *dnaKJ* | *tig* | pEXP- | 27 | *ppiC2* | *hslVU* | *PA5195* |
| pEXP- | 18 | ppi*A* | *dnaKJ* | *tig* | pEXP- | 28 | *groES* | *hslVU* | *dsbG* |
| pEXP- | 9 | *ppiA* | *dnaKJ* | *ppiD* | pEXP- | 29 | *dnaJ* | *hslVU* | *dsbG* |
| pEXP- | 10 | *ppiA* | *grpEdnaKJ* | *ppiD* | pEXP- | 30 | *surA* | *hscAB* | *dsbG* |
| pEXP- | 11 | *ppiA* | *groELS* | *ppiD* | pEXP- | 31 | *csaA* | *hscAB* | *ppiD* |
| pEXP- | 12 | *groES* | *dnaKJ* | *PA5195* | pEXP- | 32 | *PA3647* | *hscAB* | *yrfI* |
| pEXP- | 13 | *csaA* | *groELS* | *ppiD* | pEXP- | 33 | *PA3262* | *hscAB* | *lolA* |
| pEXP- | 14 | *PA3647* | *groELS* | *ppiD* | pEXP- | 34 | *ppiA* | *hscAB* | *lolA* |
| pEXP- | 15 | *PA4873* | *groELS* | *yifI* | pEXP- | 35 | *ppiC2* | *hscAB* | *PA5195* |
| pEXP- | 16 | *ppiC1* | *groELS* | *PA3717* | pEXP- | 36 | *groES* | *hscAB* | *dsbG* |
| pEXP- | 17 | *ppiC2* | *groELS* | *PA3717* | pEXP- | 37 | *csaA* | *dnaKJ* | *hslU* |
| pEXP- | 18 | *dsbC* | *groELS* | *PA5195* | pEXP- | 38 | *tig* | gro*ELS* | *dsbG* |
| pEXP- | 19 | gro*ES* | *groELS* | *PA5195* | pEXP- | 39 | *fklB* | *groELS* | *dsbG* |
| pEXP- | 20 | *dnaJ* | *groELS* | *PA5195* | pEXP- | 40 | *surA* | *groELS* | *hslU* |

**Tabelle 6b: Auflistung der erzeugten Multisite-Expressionsplasmide gem. Tabelle 6 unter Verwendung der orfs der Gene. Die Plasmide wurden mit fortlaufenden Nummern versehen (pEXP-1 - pEXP-40). Die orfs der Gene sowie die Position des Gens auf dem pEXP-Plasmid sind angegeben.**

| Nr | 5' | Mitte | 3' | Nr | 5' | Mitte | 3' |
|---|---|---|---|---|---|---|---|
| pEXP-1 | PA1800 | PA4760/61 | PA1802 | pEXP-21 | PA1800 | PA5053/54 | PA2614 |
| pEXP-2 | PA1800 | PA4385/86 | PA1802 | pEXP-22 | PA3221 | PA5053/54 | PA2614 |
| pEXP-3 | PA1800 | PA4760/61 | PA1805 | pEXP-23 | PA3647 | PA5053/54 | PA5193 |
| pEXP-4 | PA3227 | PA4760-62 | PA1800 | pEXP-24 | PA4873 | PA5053/54 | PA5193 |
| pEXP-5 | PA3227 | PA4385/86 | PA1800 | pEXP-25 | PA3227 | PA5053/54 | PA2614 |
| pEXP-6 | PA4760 | PA4385/86 | PA1800 | pEXP-26 | PA1996 | PA5053/54 | PA3717 |
| pEXP-7 | PA4760 | PA4760/61 | PA1800 | pEXP-27 | PA4176 | PA5053/54 | PA5195 |
| pEXP-8 | PA3227 | PA4760/61 | PA1800 | pEXP-28 | PA4386 | PA5053/54 | PA2476 |
| pEXP-9 | PA3227 | PA4760/61 | PA1805 | pEXP-29 | PA4760 | PA5053/54 | PA2476 |
| pEXP-10 | PA3227 | PA4760-62 | PA1805 | pEXP-30 | PA0594 | PA3811 | PA2476 |
| pEXP-11 | PA3227 | PA4385/86 | PA1805 | pEXP-31 | PA3221 | PA3811 | PA1805 |
| pEXP-12 | PA4386 | PA4760/61 | PA5195 | pEXP-32 | PA3647 | PA3811 | PA5193 |
| pEXP-13 | PA3221 | PA4385/86 | PA1805 | pEXP-33 | PA3262 | PA3811 | PA2614 |
| pEXP-14 | PA3647 | PA4385/86 | PA1805 | pEXP-34 | PA3227 | PA3811 | PA2615 |
| pEXP-15 | PA4873 | PA4385/86 | PA5193 | pEXP-35 | PA4176 | PA3811 | PA5195 |
| pEXP-16 | PA1996 | PA4385/86 | PA3717 | pEXP-36 | PA4386 | PA3811 | PA2476 |
| pEXP-17 | PA4176 | PA4385/86 | PA3717 | pEXP-37 | PA3221 | PA4760/61 | PA5054 |
| pEXP-18 | PA3737 | PA4385/86 | PA5195 | pEXP-38 | PA1800 | PA4385/86 | PA2476 |
| pEXP-19 | PA4386 | PA4385/86 | PA5195 | pEXP-39 | PA4572 | PA4385/86 | PA2476 |
| pEXP-20 | PA4760 | A4385/86 | PA5195 | pEXP-40 | PA0594 | PA4385/86 | PA5054 |

Die in der oben genannten Tabelle 6b mit entsprechenden PA-Nummern gekennzeichneten ORFs sind in der nachfolgenden Konkordanztabelle den in dem Sequenzlisting angeführten Sequenzen zugeordnet.

| Nr | 5' | Mitte | 3' | Nr | 5' | Mitte | 3' |
|---|---|---|---|---|---|---|---|
| pEXP-1 | SEQ 10 | SEQ 36/37 | SEQ 12 | pEXP-21 | SEQ 10 | SEQ 41/42 | SEQ 16 |
| pEXP-2 | SEQ 10 | SEQ 32/33 | SEQ 12 | pEXP-22 | SEQ 20 | SEQ 41/42 | SEQ 16 |
| pEXP-3 | SEQ 10 | SEQ 36/37 | SEQ 13 | pEXP-23 | SEQ 24 | SEQ 41/42 | SEQ 44 |
| pEXP-4 | SEQ 21 | SEQ 36/37/38 | SEQ 10 | pEXP-24 | SEQ 40 | SEQ 41/42 | SEQ 44 |
| pEXP-5 | SEQ 21 | SEQ 32/33 | SEQ 10 | pEXP-25 | SEQ 21 | SEQ 41/42 | SEQ 16 |
| pEXP-6 | SEQ 36 | SEQ 32/33 | SEQ 10 | pEXP-26 | SEQ 14 | SEQ 41/42 | SEQ 25 |
| pEXP-7 | SEQ 36 | SEQ 36/37 | SEQ 10 | pEXP-27 | SEQ 31 | SEQ 41/42 | SEQ 45 |
| pEXP-8 | SEQ 21 | SEQ 36/37 | SEQ 10 | pEXP-28 | SEQ 33 | SEQ 41/42 | SEQ 15 |
| pEXP-9 | SEQ 21 | SEQ 36/37 | SEQ 13 | pEXP-29 | SEQ 36 | SEQ 41/42 | SEQ 15 |
| pEXP-10 | SEQ 21 | SEQ 36/37/38 | SEQ 13 | pEXP-30 | SEQ 3 | SEQ 28 | SEQ 15 |
| pEXP-11 | SEQ 21 | SEQ 32/33 | SEQ 13 | pEXP-31 | SEQ 20 | SEQ 28 | SEQ 13 |
| pEXP-12 | SEQ 33 | SEQ 36/37 | SEQ 45 | pEXP-32 | SEQ 24 | SEQ 28 | SEQ 44 |
| pEXP-13 | SEQ 20 | SEQ 32/33 | SEQ 13 | pEXP-33 | SEQ 22 | SEQ 28 | SEQ 16 |
| pEXP-14 | SEQ 24 | SEQ 32/33 | SEQ 13 | pEXP-34 | SEQ 21 | SEQ 28 | SEQ 16 |
| pEXP-15 | SEQ 40 | SEQ 32/33 | SEQ 44 | pEXP-35 | SEQ 31 | SEQ 28 | SEQ 45 |
| pEXP-16 | SEQ 14 | SEQ 32/33 | SEQ 25 | pEXP-36 | SEQ 33 | SEQ 28 | SEQ 15 |
| pEXP-17 | SEQ 31 | SEQ 32/33 | SEQ 25 | pEXP-37 | SEQ 20 | SEQ 36/37 | SEQ 42 |
| pEXP-18 | SEQ 26 | SEQ 32/33 | SEQ 45 | pEXP-38 | SEQ 10 | SEQ 32/33 | SEQ 15 |
| pEXP-19 | SEQ 33 | SEQ 32/33 | SEQ 45 | pEXP-39 | SEQ 35 | SEQ 32/33 | SEQ 15 |
| pEXP-20 | SEQ 36 | SEQ 32/33 | SEQ 45 | pEXP-40 | SEQ 3 | SEQ 32/33 | SEQ 42 |

Nahezu alle identifizierten Chaperon-Gene konnten aus dem Genom von *P. aeruginosa* amplifiziert und in geeignete Vektoren kloniert werden. Bei den erzeugten Konstrukten konnte die DNA-Sequenz durch Sequenzierungen verifiziert werden. Ausgehend von diesen pENTR-Chaperon-Plasmiden wurden die Gene in den Expressionsvektor pDEST-VLT31 eingebracht, so daß sie in Coexpressionsversuche mit heterologen Zielproteinen eingesetzt werden können. Zusätzlich wurden, erneut basierend auf den pENTR-Chaperon-Plasmiden, pENTR-Chaperon-5'- und pENTR-Chaperon-3'-Vektoren reamplifiziert und kloniert. Durch Einsatz dieser Plasmide konnten 42 Expressionsplasmide mit Kombinationen aus jeweils drei Genen von Chaperonen und weiteren Faltungshelferproteinen konstruiert werden. Diese wurden im Folgenden in Coexpressionsversuche gemeinsam mit verschiedenen Zielgenen eingesetzt.

Vorbemerkung zu den folgenden Beispielen:
Im Folgenden werden mehrere Beispiele für die Expression von Zielproteinen und der Coexpression von Faltungshelferproteinen (entsprechend Beispiel 5) aufgeführt. Dazu wurden eine Reihe von Arbeitsschritten durchgeführt, die für die verschiedenen Beispiele immer in der gleichen Weise verliefen und hier zusammenfassend beschrieben sind. Zunächst wurden *E. coli* Expressionsstämme hergestellt, in denen sowohl das Zielprotein als auch ein Plasmid der Genexpressionsbank aus Beispiel 5 (Tabelle 6) enthalten war. Dazu wurde zunächst das Plasmid mit dem entsprechenden Zielgen in den *E*. *coli* Expressionsstamm eingebracht. Hiervon wurden elektrokompetente Zellen hergestellt, in die sämtliche Plasmide der Chaperon-Expressionsbank (Tabelle 6) in separaten Transformationsansätzen eingebracht wurden. Anschließend erfolgte die Anzucht der Teststämme in 20 ml Kulturen bei 30 oder 37 °C. Kulturen von *E*. *coli* wurden, soweit nicht anders angegeben, in LB-Medium angeimpft und über Nacht bei 37 °C inkubiert. Flüssigkulturen mit einem Volumen von 5 ml wurden in Reagenzgläsern in einem Reagenzglas-Rotator (Neolab, Heidelberg), größere Volumina in Erlenmeyer-Kolben auf einem Inkubationsschüttler (Unitron, Infors HT, Bottmingen, CH) bei 150 Upm inkubiert, wobei das Volumen zwischen Kulturvolumen und Volumen des verwendeten Kolbens 1:5 bis 1:10 entsprach. Bakterienstämme mit plasmid- oder genomcodierten Resistenzmarken wurden unter Selektionsdruck durch Zugabe des jeweiligen Antibiotikums kultiviert. Übernachtkulturen (ÜK) wurden mindestens 16 h bebrütet; Hauptkulturen wurden aus einer ÜK auf eine OD (580nm) = 0,05 beimpft. Zur Induktion einer Überexpression wurden die Kulturen mit 0,4 mM IPTG induziert und, je nach zu synthetisierenden Zielprotein, für 3-42 h bei 37°C bzw. 30°C bis zur Ernte der Zellen weiter inkubiert.

Gewinnung von Kulturüberständen und Gesamtzellextrakten:
Zellfreie Kulturüberstände wurden durch Zentrifugation (15 min, 6.000 Upm, 4°C) gewonnen und im Bedarfsfall sterilfiltriert (Schleicher & Schüll, NC45 Membranfilter, 0,45 µm Porendurchmesser). Das Zellsediment wurde entsprechend einer OD (580 nm) = 15 in 1 ml Zellaufschlußpuffer, Trispuffer (100 mM Tris-HCl, pH 8,0) oder Kaliumphosphatpuffer (100 mM KPᵢ, pH 6.0), aufgenommen und anschließend einer Ultraschallbehandlung unterzogen (Branson-Sonifier W250, 2 min, Leistungszyklus 50 %, 20 Watt). Die so erhaltenen Gesamtzellextrakte (GZE) konnten in Enzymtests eingesetzt, weiter fraktioniert oder bei -20°C gelagert werden.

Fraktionierung von *E. coli*-Zellen:
Die Zelltrümmer wurden durch eine Zentrifugation für 2 min bei 3.000 Upm aus dem GZE entfernt. Zur weiteren Auftrennung des GZE in lösliche und nicht-lösliche Fraktionen wurde für 30 min bei 14.000 Upm zentrifugiert. Die im Überstand lokalisierten löslichen Proteine (Rohextrakt) wurden direkt in Enzymtests eingesetzt. Die Lagerung der Proteinproben erfolgte bei -20°C.

### Beispiel 6 Coexpression von Chaperonen mit Alkoholdehydrogenase aus Rhodococcus erythropolis

Um eine verbesserte Löslichkeit der Alkohol-Dehydrogenase aus *Rhodococcus erythropolis* (RE-ADH) zu erreichen, wurde dieses Enzym in *E. coli* mit 20 verschiedenen Faltungsmodulatoren entsprechend Tabelle 6 (Beispiel 5) coexprimiert. Diese 20 verschiedenen Expressionsstämme wurden dann wie in der Vorbemerkung erwähnt angezogen, die Zellen durch Zentrifugation geerntet. Nach Zellaufschluss und Zentrifugation wurde die Aktivität der löslichen RE-ADH im Rohextrakt in einem photometrischen Assay gemessen. Dazu wurden 970 µl 100 mM KPᵢ-Puffer (pH 6,0) mit 3 mM *p*-Cl-Acetophenon, 20 µl 12,5 mM NADH sowie 10 µl Enzymlösung (Rohextrakt) eingesetzt (Endvolummen 1 ml).

In Fig. 2 sind die gemessenen Aktivitäten der RE-ADH aufgetragen, wobei der Kontrollstamm mit rekombinanter RE-ADH, aber ohne zusätzliche Faltungshelferproteine zeigt dabei eine Aktivität von ca. 50 U/ml. Im Vergleich dazu können die untersuchten Expressionsstämme in vier Gruppen unterteilt werden, die jeweils vergleichbare Enzymaktivitäten aufweisen.

Eine Anzahl von zehn untersuchten Stämmen zeigte im Vergleich mit der Kontrolle (s. Fig. 2, **A:** K) eine deutliche Verringerung der Aktivität. Teilweise konnte überhaupt keine Aktivität detektiert werden (z.B. die Stämme mit den *Multisite*-Kombinationen pEXP-5, -9 oder pEXP-24). Als Gruppe II wurden die Teststämme in Fig. 2 zusammengefaßt, die mit ca. 50 U/ ml in etwa die gleiche Enzymaktivität aufwiesen wie der Kontrollstamm LV. Bei den 16 verbleibenden Stämmen, in denen Chaperone mit dem Protein RE-ADH erzeugt wurden, konnte eine signifikant höhere ADH-Aktivität nachgewiesen werden. Diese war um den Faktor 2 bis 5 höher als bei dem Kontrollstamm LV. Das beste Ergebnis wurde erzielt, indem mit dem Vektor pEXP-13 die Gene der Faltungsmodulatoren CsaA, GroELS und PpiD exprimiert wurden.

### Beispiel 7

### Coexpression von Chaperonen mit Alkoholdehydrogenase aus Rhodococcus ruber

In einem weiteren Beispiel wurde die Verbesserung einer weiteren Alkohol-Dehydrogenase durch Coexpression von Faltungshelferproteinen untersucht, einem Enzym aus *Rhodococcus ruber* (RR-ADH). Die Vorgehensweise entspricht vollständig der in Beispiel 6 beschriebenen Methodik, lediglich das dort beschrieben Gen für RE-ADH wurde durch die RR-ADH ersetzt. Auch der Enzymtest entspricht dem vorab beschriebenen. Der RR-ADH-Expressionsstamm mit dem Leervektor (Kontrollstamm) wies eine Aktivität von ca. 8 U/ml auf (Fig. 3). Dessen ADH-Aktivität wurde mit der von Stämmen verglichen, in denen einzelne Faltungsmodulatoren coexprimiert wurden. Anhand der ermittelten RR-ADH-Aktivitäten konnten die Stämme in vier Gruppen unterteilt werden. Die erste Gruppe faßt die Stämme zusammen, in denen schwache oder keine RR-ADH-Aktivität gemessen werden konnte. Hier waren die Faltungshelfer SurA, PA0499 sowie DnaKJ in dem RR-ADH-Expressionsstamm enthalten. Enzymaktivitäten, die mit 6 - 8 U/ml in der Größenordnung des Kontrollstamms lagen, wurden durch die Coexpression der Chaperone IbpA, FklB, PA4873, GroELS und PpiC2 erzielt (Fig. 3; Gruppe II). Sechs Chaperonsysteme (Fig. 3; Gruppe III) führten zu einer Steigerung der RR-ADH-Aktivität um 25 %, verglichen mit der Kontrolle. Bei diesen Faltungshelfern handelt es sich um DsbG, PA2725, Skp, PpiD, CsaA sowie HsIU. Die deutlichste Verbesserung der RR-ADH-Aktivität um 45 % - 65 %, bezogen auf den Kontrollstamm, konnte durch die Chaperone PA3717, PA5254, HscAB und Yrfl erreicht werden.

### Beispiel 8

### Coexpressionen von Chaperonen mit dem katalytisch aktiven Teil der Esterase EstA aus P. aeruginosa

Die Esterase EstA aus dem Bakterium *P. aeruginosa* ist ein lipolytisches Enzym, welches bevorzugt kurzkettige Glycerolester von Fettsäuren hydrolysiert [37]. Bei der Überexpression des katalytisch aktiven N-Terminus der Esterase EstA (EstAα) aus *P*. *aeruginosa* im heterologen Wirt *E. coli* BL21 (DE3) akkumulierte ein großer Teil des Proteins in Form von *Inclusion bodies.* Deshalb wird im Folgenden das EstAα-Fragment zusammen mit den Faltungsmodulatoren entsprechend Beispiel 5 exprimiert, um die Löslichkeit des Proteins zu verbessern. Dabei stammen sowohl das Zielprotein als auch die coexprimierten Faltungshelferproteine aus demselben Organismus, *P. aeruginosa.*

Die Vorgehensweise der Gewinnung der Expressionsstämme und der Präparation von Rohextrakt entspricht vollständig der in Beispiel 6 beschriebenen Methodik, lediglich das dort beschrieben Gen für RE-ADH wurde durch das Gen für den N-Terminus der Esterase EstA ersetzt. Die Aktivität der Esterase EstA wurde mit folgendem Assay bestimmt: Pro Reaktion wurden 1,5 ml Testlösung [25 µl p-Nitrophenyl-Caproat in 5 ml Ethanol gelöst; plus 100 ml Puffer (100 mM KPi pH 7,2; 10mM Mg2SO₄)] mit 5-50 µl Probe vermischt und für 15 min bei 30°C inkubiert. Anschließend wurde die OD (410 nm) der Ansätze im Spektralphotometer bestimmt. Angegeben werden die Werte als relative Aktivität in U/ml (Extinktionskoeffizient ε = 10400 M⁻¹). Die Figur 4 zeigt das Ergebnis dieser Aktivitätsmessungen für die einzelnen Expressionsstämme. Die Aktivität des Kontrollstammes BL21(DE3)-EstAα-LV betrug 0,25 U/ (ml · OD (580 nm)). Im Vergleich dazu wiesen einige Stämme (Fig. 4; Gruppe I) eine leicht bis deutlich verminderte Aktivität der Esterase auf. Der Expressionsstamm 2, bei dem die Gene *tig, groELS* und *clpX* coexprimiert wurden, zeigte keinen Einfluss auf die Ausbeute an aktivem Protein, verglichen mit der Kontrolle (Fig. 4; Gruppe II). In einer weiteren Gruppe (Fig. 4; Gruppe III) konnte eine signifikante Erhöhung der Esterase-Aktivität durch die Coexpression von Faltungshelferproteinen erreicht werden. Neben den Plasmiden pEXP-5 (*ppiA* - *groELS* - *tig*) sowie pEXP-7 (*dnaJ - dnaKJ* - *tig*) zeigte auch die Expression des Vektors pEXP-11 (*ppiA* - *groELS* - *ppiD*) einen positiven Einfluß auf die Produktion des EstAα-Proteins. Gegenüber dem Vergleichsstamm lag hier eine Erhöhung der Aktivität um den Faktor 1,6 vor. Dies bedeutet eine Aktivität von ca. 0,35-0,4 U/ (ml · OD (580 nm)). Am effektivsten war die Produktion der katalytisch aktiven Domäne der Esterase EstA, wenn das Plasmid mit der Faltungshelferkombination 1 (*tig* - *dnaKJ* - *clpX*) im Expressionsstamm vorlag. Hier konnte eine Verdopplung der Aktivität im Vergleich zum Kontrollstamm auf ca. 0,5 U/ (ml · OD (580 nm)) erzielt werden.

### Beispiel 9

### Untersuchungen zur Coexpression von Chaperonen aus P. aeruginosa mit der Serratia marcescens Lipase LipA

Das Enzym LipA aus dem Bakterium *S. marcescens* besitzt durch seine Zugehörigkeit zu der Enzymklasse der Lipasen eine besondere biotechnologische Bedeutung, sie sind wichtige stereoselektive Biokatalysatoren, die sowohl bei der Veresterung als auch der Spaltung von Lipiden eine entscheidende Rolle spielen. Bei dem Versuch, die Lipase LipA aus *S. marcescens* in großen Mengen zu produzieren, akkumulierte der größte Teil des Proteins in Form von *Inclusion bodies* [38]. Daher wurde im Folgenden die Coexpression von Faltungshelfern auf die Ausbeute löslicher Lipase im heterologen Wirt *E*. *coli* auswirkt. Die in Coexpressionsversuchen mit den verschiedenen Expressionsstämmen entsprechend Beispiel 5 produzierten Proteinproben wurden in Enzymtests untersucht, die folgende Zusammensetzung hatten: Substratemulsion: 207 mg Natriumdesoxycholat, 100 mg Gummi arabicum, 90 ml Sörensen Phoshatpuffer pH 8,0 (Lösung A: 8,9 g/l Na₂HPO₄, Lösung B: 0,68 g/l KH₂HPO₄ [A:B → 17:1]); 30 mg p-Nitrophenyl-Palmitat in 10 ml Isopropanol. Pro Reaktion wurden 2,5 ml Substratemulsion mit 20-100 µl Probe vermischt und für 15 min bei 37 °C inkubiert. Anschließend wurde die OD (410nm) der einzelnen Ansätze im Spektralphotometer gemessen. Dabei entspricht die gemessene OD (410 nm) = 1 einer Lipasemenge von 0,121 ng bzw. einer Enzymaktivität von 0,161 nkat. Die erhaltenen Ergebnisse sind in Fig. 5 dargestellt. Als Positivkontrolle und Referenz gegenüber den Stämmen, in denen Chaperone und Foldasen coexprimiert wurden, diente der als LV bezeichnete Stamm, in welchem neben dem *lipA*-Expressionsplasmid ausschließlich der Chaperon-Leervektor in den Zellen vorlag. Für diesen Stamm konnte eine Lipase-Aktivität von ca. 0,8 nkat/ o.D. _{580 nm} detektiert werden (Fig. 5; **A).** Die Negativkontrolle wurde durch den Bakerienstamm repräsentiert, der beide verwendeten Expressionsplasmide als Leervektor enthielt und somit kein LipA-Protein erzeugen kann. Für diesen Stamm konnte keine Umsetzung des Lipase-Substrates p-Nitrophenyl-Palmitat im durchgeführten Enzymtest festgestellt werden.

### Beispiel 10

### Coexpression von Chaperonen aus P. aeruginosa mit der Lipase B (CALB) aus der Hefe Candida antarctica

In einem weiteren Beispiel sollte untersucht werden, ob die Coexpression von Chaperonen auch die Expression der Lipase CALB aus *Candida antarctica* verbessern kann. Diese Lipase ist biotechnologisch von besonderer Bedeutung, da sie eine hohe Enantioselektivität, vor allem bei der Umsetzung von 1-Methoxy-2-Propanolacetat (MPA), aufweist. Da die Ausbeute von heterolog in *E. coli* produziertem CALB-Protein bisher sehr gering ist, sollte untersucht werden, ob die Ausbeute durch Zugabe von Faltungsmodulatoren erhöht werden kann. Durch Fusion des Lipase-Gens hinter eine *pelB*-Signalsequenz kann die Lipase ins Periplasma der *E. coli* Zellen transportiert werden. Auf diese Weise kann in Plattentests untersucht werden, ob die Zellen Lipase produzieren oder nicht. Deshalb wurden einzelne Kolonien der unterschiedlichen Stämme auf Tributyrin-Platten überimpft. Die Testplatten hatten folgende Zusammensetzung: 7,5 ml Tributyrin und 0,75g Gummi arabicum ad 15 ml Aqua dest. mischen, das Tributyrin mit Ultraschall (3 min, 75 W, 100%) emulgieren und zu 500 ml autoklaviertem LB-Agar (60°C) geben [39]. Lipolytische Aktivität zeigt sich durch Bildung klarer Höfe um die Bakterienkolonie. In Fig. 6 sind drei solcher Tributyrin-Platten gezeigt **(A, B** und **C),** auf die unterschiedliche Expressionsstämme entsprechend Beispiel 5 aufgebracht wurden. Neben einigen Stämmen, in denen keine oder nur eine sehr geringe lipolytische Aktivität festgestellt werden konnte, fallen mehrere Expressionsstämme auf, in denen leicht bis signifikant mehr Lipase-Aktivität, verglichen mit dem Kontrollstamm, detektiert werden konnte. Mit den Plasmiden pEXP-18, -19, -28 und pEXP-33 konnte das beste Ergebnis erzielt werden. Durch die Zugabe dieser unterschiedlichen Faltungsmodulator-Kombinationen konnte die Menge aktiver Lipase deutlich gesteigert werden.

Neben diesem Plattenassay wurde der Einfluss der Faltungsmodulatoren mit löslichen Proteinproben in einem photometrischen Test mit p-Nitrophenyl-Palmitat als Substrat (s. Beispiel 9) eingesetzt und die Aktivität in U/ ml bestimmt (Fig. 7). Für den Kontrollstamm (LV) konnte eine sehr geringe Aktivität von 0,05 U/ ml bestimmt werden. Bei den weiteren zwölf Expressionsstämmen korrelieren die Ergebnisse aus den beiden Aktivitätstests. Dabei können einige Stämme identifiziert werden, die im Enzymtest eine Verbesserung der Aktivität um 60 %, verglichen mit der Kontrolle, aufweisen (Fig. 7; Gruppe II).

Bei drei weiteren Stämmen führte die Coexpression von Chaperon-Kombinationen zu einer Steigerung der CALB-Aktivität um 180 %. Dazu zählt unter anderem der Stamm mit dem Plasmid pEXP-19, in dem die Faltungsmodulatoren GroES - GroELS - PA5195 coexprimiert wurden. In beiden Enzymtests wies er eine signifikante Steigerung der lipolytischen Aktivität auf. Die Coexpression der Chaperone DsbC, GroELS und PA5195 (pEXP-18) führte bei einer Anzucht auf Agarplatten zu der deutlichsten Steigerung. Die Inkubation in Flüssigmedium rief eine Verdreifachung der Aktivität im Vergleich zum Kontrollstamm hervor, was den Umsatz des Substrates Tributyrin im ersten Enzymtest bestätigte.

Die drei Stämme mit den *Multisite*-Vektoren pEXP-33, -35 und -39 zeigen im photometrischen Test eine ebenso schwache Lipase-Aktivität auf wie der Kontrollstamm (Fig. 7; Gruppe I). In dem durchgeführten Tributyrin-Plattentest dagegen konnte für diese drei Stämme ein deutlich größerer Hof als bei dem Expressionsstamm mit dem Chaperon-Leervektor festgestellt werden. Auch für den Stamm mit dem Plasmid pEXP-12 entsprach der photometrische Aktivitätstest nicht dem Ergebnis der Tributyrin-Agarplatten. Während auf den Agarplatten mit Tributyrin als Substrat fast kein Hof um die Kolonie sichtbar wurde, zeigte sich im pNPP-Test, der mit Flüssigkulturen durchgeführt wurde, eine Verdopplung der Aktivität.

### Beispiel 11

### Coexpression von Chaperonen und Faltungskatalysatoren mit dem Grün-fluoreszierende Protein (GFP)

Das Grün-fluoreszierende Protein wird in der Wissenschaft unter anderem als Marker für Faltungsprozesse eingesetzt. Dabei konnte gezeigt werden, daß GFP durch direkten Einfluß von Chaperonen seine aktive Konformation erreicht [40, 41]. In diesem Beispiel wurde daher überprüft, ob Kombinationen von Faltungshelfer-Proteinen entsprechend Beispiel 5 die Faltung von GFP verbessern konnten. Als quantitatives Maß für die Faltung wird die Fluoreszenzemission des GFP-Proteins gemessen. Es wurde jeweils 1 ml GZE (siehe Vorbemerkung) mit einer Zelldichte, die einer OD (580 nm) von 1 entsprach, in Viertelmikroküvetten bei einer Anregungswellenlänge von 475 nm in einem Emissionsbereich von 490 - 550 nm vermessen. Die jeweilige Fluoreszenzintensität als "relative Fluoreszenzemission" wurde aus der gemessenen Fluoreszenzemission pro Zelldichte, die einer OD von 1 entsprach, und pro 1 ml Zellextrakt oder Zellsuspension berechnet. Für den Kontrollstamm wurde eine relative Fluoreszenz von ca. 240 pro ml ermittelt (Fig. 8; K). Die Proben wurden vorher auf eine Zelldichte von 1 eingestellt, ermittelt durch die OD (580 nm). Mit 28 Proben führten die meisten Chaperon-Kombinationen zu einer Reduktion der Menge an fluoreszierendem, löslichen Protein (Fig. 8; Gruppe I). Durch die Coexpression von sechs Multisite-Expressionsplasmiden wurde eine vergleichbare Fluoreszenz zum Kontrollstamm ermittelt (Fig. 8; Gruppe II). Es konnten aber auch Stämme identifiziert werden, die mehr aktives GFP produzieren konnten als die Kontrolle. Hier führte die Expression von Faltungsmodulatoren zu Werten der relativen Fluoreszenz von 400-500, also einer Steigerung der Menge aktiven Proteins um 100 % in den besten Fällen. Die effektivsten *Multisite*-Kombinationen hinsichtlich der GFP-Expression waren pEXP-1 (Tig, DnaKJ und ClpX), pEXP-40 (SurA, GroELS und HsIU), aber vor allem pEXP-19 (GroES, GroELS und PA5195), pEXP-27 (PpiC2, HslVU und PA5195) und pEXP-33 (PA3262, HscAB und LolA).

### Beispiel 12

### Verbesserung der Produktion von humanem Interferon α2b

Ein weiteres Zielprotein stellt das menschliche Interferon alpha 2b (IFNα2b) dar. Dieses Protein wird in der Medizin zur Behandlung von verschiedenen Krankheiten eingesetzt, wie Hepatitis, Krebsarten wie beispielsweise Leukämie oder bei AIDS-vermitteltem Kaposisarkom [42]. Die Produktion einer großen Menge löslichen Proteins hat demnach eine besondere Bedeutung für die pharmazeutische Industrie. Zwar kann das IFNα2b-Protein heterolog in *E. coli* exprimiert werden, doch werden zum Einen nur geringe Ausbeuten erzielt und zum Anderen akkumuliert der größte Teil in Form von Inclusion bodies. Bislang wird dann im Anschluß das IFNα2b-Protein isoliert, renaturiert und weiter verwendet [43], ein zwar gängiger, aber kosten- und zeitaufwendiger Prozeß.

In diesem Beispiel wurde daher das menschliche Protein Interferon α2b mit den entsprechend Beispiel 5 hergestellten Faltungshelferproteinen coexprimiert. Dabei wurde das IFNα2b-Protein mit einer N-terminalen Signalsequenz exprimiert, die einen Transport aus dem Cytoplasma heraus ermöglichen sollte. Als Kontrolle wurde ein BL21 (DE3)-Stamm verwendet, der neben dem Interferon-Expressionsplasmid den Chaperon-Leervektor pBBR1 MCS-3 enthielt (Fig. 10; K). Für diesen Stamm konnte gezeigt werden, daß ca. 70 % des erzeugten IFNα2b-Proteins im GZE vorlag und 30 % im Medium nachgewiesen werden konnte (s. Fig. 10).

Von 13 unterschiedlichen Teststämmen führte die Coexpression von drei Chaperon-Kombinationen dazu, daß keine nachweisbare IFNα2b-Menge, weder im GZE noch im Kulturüberstand, detektiert werden konnte (pEXP-10, -13 und pEXP-14; Daten nicht gezeigt). In weiteren Stämmen führte die zusätzliche Expression von Faltungsmodulatoren dazu, daß IFNα2b-Protein nachgewiesen werden konnte. In den meisten Fällen entsprach die Menge und Verteilung des Zielproteins der des Kontrollstammes, beispielsweise der Stamm mit dem Multisite-Expressionsvektore pEXP-2 (Tig, GroELS, ClpX). Die Coexpression des Plasmides pEXP-3 (Tig, DnaKJ und PpiD) führte dazu, daß nur ein sehr geringer Proteinanteil ins Kulturmedium gelangen konnte und ca. 90 % des produzierten IFNα2b-Proteins unlöslich in Form von Inclusion bodies im Cytoplasma akkumuliert war (Fig. 10; 3). In zwei weiteren Stämmen mit den Expressionsplasmiden pEXP-4 bzw. pEXP-12 (s. Tab. 6) war mehr IFN2αb-Protein nachweisbar als in der Kontrolle. Durch Coexpression beider Vektoren konnte beobachtet werden, daß der größte Anteil des IFNα2b-Proteins hier im Kulturmedium vorhanden war, während nur eine sehr geringe Menge des Proteins im GZE detektiert werden konnte. Fast das gesamte IFNα2b-Protein wurde also aus der Bakterienzelle heraus transportiert.

### Literaturverzeichnis

[1] P. Jonasson, S. Liljeqvist, P. A. Nygren, S. Stahl, Biotechnol Appl Biochem 2002, 35, 91.
[2] J. C. Young, V. R. Agashe, K. Siegers, F. U. Hartl, Nature Reviews Molecular Cell Biology 2004, 5, 781.
[3] J. G. Thomas, A. Ayling, F. Baneyx, Applied Biochemistry and Biotechnology 1997, 66, 197.
[4] C. Schlieker, B. Bukau, A. Mogk, J Biotechnol 2002, 96, 13.
[5] G. Fischer, H. Bang, C. Mech, Biomed Biochim Acta 1984, 43, 1101.
[6] C. L. Andersen, A. Matthey-Dubraz, D. Missiakas, S. Raina, Mol Microbiol 1997, 26, 121.
[7] J. Sambrook, F. E. F., M. T., Molecular Cloning: a Laboratory Manual, 2nd ed. Cold Spring Habor Laboratory Press, New York, 1989**.**
[8] C. K. Stover, X. Q. Pham, A. L. Erwin, et al., Nature 2000, 406, 959.
[9] S. Lindquist, E. A. Craig, Annu Rev Genet 1988, 22, 631.
[10] F. Confalonieri, M. Duguet, Bioessays 1995, 17, 639.
[11] A. F. Neuwald, L. Aravind, J. L. Spouge, E. V. Koonin, Genome Res 1999, 9, 27.
[12] F. Baneyx, M. Mujacic, Nat Biotechnol 2004, 22, 1399.
[13] R. Chen, U. Henning, Mol Microbiol 1996, 19, 1287.
[14] U. Schäfer, K. Beck, M. Muller, J Biol Chem 1999, 274, 24567.
[15] R. M. Delahay, R. K. Shaw, S. J. Elliott, et al., Mol Microbiol 2002, 43, 61.
[16] B. Bukau, A. L. Horwich, Cell 1998, 92, 351.
[17] G. Fischer, F. X. Schmid, Biochemistry 1990, 29, 2205.
[18] A. Galat, Eur J Biochem 1993, 216, 689.
[19] A. Hiniker, J. C. A. Bardwell, Biochemistry 2003, 42, 1179.
[20] H. Kadokura, F. Katzen, J. Beckwith, Annu Rev Biochem 2003, 72, 111.
[21] A. Urban, M. Leipelt, T. Eggert, K. E. Jaeger, J Bacteriol 1999, 183, 587.
[22] A. Holmgren, C. I. Branden, Nature 1989, 342, 248.
[23] S. Shuman, Proc. Natl. Acad. Sci. USA 1991, 88, 10104.
[24] A. Landy, Annu Rev Biochem 1989, 58, 9.
[25] P. Bernard, M. Couturier, J Mol Biol 1992, 226, 735.
[26] T. Miki, J. A. Park, K. Nagao, et al., J Mol Biol 1992, 225, 39.
[27] M. E. Kovach, R. W. Phillips, P. H. Elzer, et al., BioTechniques 1994, 16, 800.
[28] M. E. Kovach, P. H. Elzer, D. S. Hill, et al., Gene 1995, 166, 175.
[29] S. Wilhelm, persönliche Mitteilung 2007**.**
[30] A. A. Watson, R. A. Alm, J. S. Mattick, Gene 1996, 172, 163.
[31] V. De Lorenzo, L. Eltis, B. Keßler, K. N. Timmis, Gene 1993, 123, 17.
[32] H. Düfel, Diploma thesis, Ruhruniversität Bochum 1995**.**
[33] S. Becker, S. Theile, N. Heppeler, et al., FEBS Lett 2005, 579, 1177.
[34] J. Andexer, persönliche Mitteilung 2007**.**
[35] A. Schulz, Diplomarbeit, Heinrich-Heine Universität Düsseldorf 2005.
[36] A. Abokitse, W. Hummel, Appl Microbiol Biotechnol 2003, 62, 380.
[37] S. Wilhelm, J. Tommassen, K.-E. Jaeger, J Bacteriol 1999, 181, 6977.
[38] H. Akatsuka, R. Binet, E. Kawai, et al., J Bacteriol 1994, 179, 4754.
[39] R. G. Kok, V. M. Christoffels, B. Vosman, K. J. Hellingwerf, J Gen Microbiol 1993, 139, 2329.
[40] J. D. Pedelacq, S. Cabantous, T. Tran, et al., Nat Biotechnol 2006, 24, 79.
[41] J. D. Wang, C. Herman, K. A. Tipton, et al., Cell 2002, 111, 1027.
[42] A. Kaser, H. Tilg, Cell Mol Biol 2000, 47, 609.
[43] C. A. Valente, G. A. Monteiro, J. M. S. Cabral, et al., Protein Expr Purif 2006, 45, 226.

## Patentansprüche

1. Verfahren zur verbesserten Expression von rekombinanten Proteinen, wobei ein für ein rekombinantes Protein codierendes Expressionsplasmid in Kombination mit wenigstens drei für unterschiedliche Chaperone codierenden Nukleotidsequenzen des Organismus Pseudomonas aeruginosa, wobei die wenigstens drei für unterschiedliche Chaperone codierenden Nukleotidsequenzen entweder mittels dreier getrennter Chaperon-Expressionsplasmide oder mittels eines Multisite-Expressionsplasmid in einen Wirtsorganismus eingebracht und das rekombinante Protein und die Chaperone coexprimiert werden,
**dadurch gekennzeichnet, dass** die Kombination der drei für unterschiedlichen Chaperone codierenden Nukleotidsequenzen eine der Kombinationen pEXP-1 bis pEXP-40 gemäß der nachfolgenden Tabelle ist
| Nr | 5' | Mitte | 3' |
|---|---|---|---|
| pEXP-1 | PA1800 | PA4760/61 | PA1802 |
| pEXP-2 | PA1800 | PA4385/86 | PA1802 |
| pEXP-3 | PA1800 | PA4760/61 | PA1805 |
| pEXP-4 | PA3227 | PA4760-62 | PA1800 |
| pEXP-5 | PA3227 | PA4385/86 | PA1800 |
| pEXP-6 | PA4760 | PA4385/86 | PA1800 |
| pEXP-7 | PA4760 | PA4760/61 | PA1800 |
| pEXP-8 | PA3227 | PA4760/61 | PA1800 |
| pEXP-9 | PA3227 | AA4760/61 | PA1805 |
| pEXP-10 | PA3227 | PA4760-62 | PA1805 |
| pEXP-11 | PA3227 | PA4385/86 | PA1805 |
| pEXP-12 | PA4386 | PA4760/61 | PA5195 |
| pEXP-13 | PA3221 | PA4385/86 | PA1805 |
| pEXP-14 | PA3647 | PA4385/86 | PA1805 |
| pEXP-15 | PA4873 | PA4385/86 | PA5193 |
| pEXP-16 | PA1996 | PA4385/86 | PA3717 |
| pEXP-17 | PA4176 | PA4385/86 | PA3717 |
| pEXP-18 | PA3737 | PA4385/86 | PA5195 |
| pEXP-19 | PA4386 | PA4385/86 | PA5195 |
| pEXP-20 | PA4760 | PA4385/86 | PA5195 |
| pEXP-21 | PA1800 | PA5053/54 | PA2614 |
| pEXP-22 | PA3221 | PA5053/54 | PA2614 |
| pEXP-23 | PA3647 | PA5053/54 | PA5193 |
| pEXP-24 | PA4873 | PA5053/54 | PA5193 |
| pEXP-25 | PA3227 | PA5053/54 | PA2614 |
| pEXP-26 | PA1996 | PA5053/54 | PA3717 |
| pEXP-27 | PA4176 | PA5053/54 | PA5195 |
| pEXP-28 | PA4386 | PA5053/54 | PA2476 |
| pEXP-29 | PA4760 | PA5053/54 | PA2476 |
| pEXP-30 | PA0594 | PA3811 | PA2476 |
| pEXP-31 | PA3221 | PA3811 | PA1805 |
| pEXP-32 | PA3647 | PA3811 | PA5193 |
| pEXP-33 | PA3262 | PA3811 | PA2614 |
| pEXP-34 | PA3227 | PA3811 | PA2615 |
| pEXP-35 | PA4176 | PA3811 | PA5195 |
| pEXP-36 | PA4386 | PA3811 | PA2476 |
| pEXP-37 | PA3221 | PA4760/61 | PA5054 |
| pEXP-38 | PA1800 | PA4385/86 | PA2476 |
| pEXP-39 | PA4572 | PA4385/86 | PA2476 |
| pEXP-40 | PA0594 | PA4385/86 | PA5054 |
und der Wirtsorganismus ausgewählt ist aus der Gruppe bestehend aus *Rhodococcus erythropolis, Rhodococcus ruber, Escherichia coli, Bacillus subtilis, Saccharomyces sp., Pichia sp., Hansenula sp., Schizosaccharomyces pombe, Corynebacterium sp. oder Aspergillus sp..*

## Claims

1. Method for improved expression of recombinant proteins, wherein an expression plasmid coding for a recombinant protein in combination with at least three nucleic acid sequences of the organism *Pseudomonas aeruginosa* coding for different chaperones, wherein the at least three nucleic acid sequences coding for different chaperones are introduced either by means of three separated chaperon expression plasmids or by means of a multisite expression plasmid into a host organism and the recombinant protein and the chaperone are co-expressed,
**characterised in that** the combination of the three nucleic acid sequences coding for different chaperones is one of the combinations pEXP-1 to pEXP-40 according to the following table
| No | 5' | middle | 3' |
|---|---|---|---|
| pEXP-1 | PA1800 | PA4760/61 | PA1802 |
| pEXP-2 | PA1800 | PA4385/86 | PA1802 |
| pEXP-3 | PA1800 | PA4760/61 | PA1805 |
| pEXP-4 | PA3227 | PA4760-62 | PA1800 |
| pEXP-5 | PA3227 | PA4385/86 | PA1800 |
| pEXP-6 | PA4760 | PA4385/86 | PA1800 |
| pEXP-7 | PA4760 | PA4760/61 | PA1800 |
| pEXP-8 | PA3227 | PA4760/61 | PA1800 |
| pEXP-9 | PA3227 | PA4760/61 | PA1805 |
| pEXP-10 | PA3227 | PA4760-62 | PA1805 |
| pEXP-11 | PA3227 | PA4385/86 | PA1805 |
| pEXP-12 | PA4386 | PA4760/61 | PA5195 |
| pEXP-13 | PA3221 | PA4385/86 | PA1805 |
| pEXP-14 | PA3647 | PA4385/86 | PA1805 |
| pEXP-15 | PA4873 | PA4385/86 | PA5193 |
| pEXP-16 | PA1996 | PA4385/86 | PA3717 |
| pEXP-17 | PA4176 | PA4385/86 | PA3717 |
| pEXP-18 | PA3737 | PA4385/86 | PA5195 |
| pEXP-19 | PA4386 | PA4385/86 | PA5195 |
| pEXP-20 | PA4760 | PA4385/86 | PA5195 |
| pEXP-21 | PA1800 | PA5053/54 | PA2614 |
| pEXP-22 | PA3221 | PA5053/54 | PA2614 |
| pEXP-23 | PA3647 | PA5053/54 | PA5193 |
| pEXP-24 | PA4873 | PA5053/54 | PA5193 |
| pEXP-25 | PA3227 | PA5053/54 | PA2614 |
| pEXP-26 | PA1996 | PA5053/54 | PA3717 |
| pEXP-27 | PA4176 | PA5053/54 | PA5195 |
| pEXP-28 | PA4386 | PA5053/54 | PA2476 |
| pEXP-29 | PA4760 | PA5053/54 | PA2476 |
| pEXP-30 | PA0594 | PA3811 | PA2476 |
| pEXP-31 | PA3221 | PA3811 | PA1805 |
| pEXP-32 | PA3647 | PA3811 | PA5193 |
| pEXP-33 | PA3262 | PA3811 | PA2614 |
| pEXP-34 | PA3227 | PA3811 | PA2615 |
| pEXP-35 | PA4176 | PA3811 | PA5195 |
| pEXP-36 | PA4386 | PA3811 | PA2476 |
| pEXP-37 | PA3221 | PA4760/61 | PA5054 |
| pEXP-38 | PA1800 | PA4385/86 | PA2476 |
| pEXP-39 | PA4572 | PA4385/86 | PA2476 |
| pEXP-40 | PA0594 | PA4385/86 | PA5054 |
and the host organism is selected from the group consisting of *Rhodococcus erythropolis, Rhodococcus ruber, Escherichia coli, Bacillus subtilis, Soccharomyces sp., Pichia sp., Hansenula sp., Schizosaccharomyces pombe, Corynebacterium sp.* or *Aspergillus sp..*

## Revendications

1. Procédé destiné à une expression améliorée de protéines recombinantes, dans lequel un plasmide d'expression codant pour une protéine recombinante en combinaison avec au moins trois séquence de nucléotides de l'organisme Pseudomonas aeruginosa codant pour différents chaperons, où les au moins trois séquences de nucléotides codant pour différents chaperons sont incorporées dans un organisme hôte soit au moyen de trois plasmides d'expression de chaperons séparés, soit au moyen d'un plasmide d'expression multisite, et le protéine recombinante et les chaperons sont co-exprimés,
**caractérisé en ce que** la combinaison des trois séquences de nucléotides codant pour différents chaperons est l'une des combinaisons pEXP-1 à pEXP-40 selon le tableau suivant
| Numéro | 5' | Milieu | 3' |
|---|---|---|---|
| pEXP-1 | PA1800 | PA4760/61 | PA1802 |
| pEXP-2 | PA1800 | PA4385/86 | PA1802 |
| pEXP-3 | PA1800 | PA4760/61 | PA1805 |
| pEXP-4 | PA3227 | PA4760-62 | PA1800 |
| pEXP-5 | PA3227 | PA4385/86 | PA1800 |
| pEXP-6 | PA4760 | PA4385/86 | PA1800 |
| pEXP-7 | PA4760 | PA4760/61 | PA1800 |
| pEXP-8 | PA3227 | PA4760/61 | PA1800 |
| pEXP-9 | PA3227 | PA4760/61 | PA1805 |
| pEXP-10 | PA3227 | PA4760-62 | PA1805 |
| pEXP-11 | PA3227 | PA4385/86 | PA1805 |
| pEXP-12 | PA4386 | PA4760/61 | PA5195 |
| pEXP-13 | PA3221 | PA4385/86 | PA1805 |
| pEXP-14 | PA3647 | PA4385/86 | PA1805 |
| pEXP-15 | PA4873 | PA4385/86 | PA5193 |
| pEXP-16 | PA1996 | PA4385/86 | PA3717 |
| pEXP-17 | PA4176 | PA4385/86 | PA3717 |
| pEXP-18 | PA3737 | PA4385/86 | PA5195 |
| pEXP-19 | PA4386 | PA4385/86 | PA5195 |
| pEXP-20 | PA4760 | PA4385/86 | PA5195 |
| pEXP-21 | PA1800 | PA5053/54 | PA2614 |
| pEXP-22 | PA3221 | PA5053/54 | PA2614 |
| pEXP-23 | PA3647 | PA5053/54 | PA5193 |
| pEXP-24 | PA4873 | PA5053/54 | PA5193 |
| pEXP-25 | PA3227 | PA5053/54 | PA2614 |
| pEXP-26 | PA1996 | PA5053/54 | PA3717 |
| pEXP-27 | PA4176 | PA5053/54 | PA5195 |
| pEXP-28 | PA4386 | PA5053/54 | PA2476 |
| pEXP-29 | PA4760 | PA5053/54 | PA2476 |
| pEXP-30 | PA0594 | PA3811 | PA2476 |
| pEXP-31 | PA3221 | PA3811 | PA1805 |
| pEXP-32 | PA3647 | PA3811 | PA5193 |
| pEXP-33 | PA3262 | PA3811 | PA2614 |
| pEXP-34 | PA3227 | PA3811 | PA2615 |
| pEXP-35 | PA4176 | PA3811 | PA5195 |
| pEXP-36 | PA4386 | PA3811 | PA2476 |
| pEXP-37 | PA3221 | PA4760/61 | PA5054 |
| pEXP-38 | PA1800 | PA4385/86 | PA2476 |
| pEXP-39 | PA4572 | PA4385/86 | PA2476 |
| pEXP-40 | PA0594 | PA4385/86 | PA5054 |
et l'organisme hôte est choisi dans le groupe constitué de *Rhodococcus erythropolis, Rhodococcus ruber, Escherichia coli, Bacillus subtilis, Saccharomyces sp., Pichia sp., Hansenula sp., Schizosaccharomyces pombe, Corynebacterium sp. ou Aspergillus sp..*


### sequenzprotokoll

<110> Evocatal GmbH
<120> Chaperone toolbox
<130> 09-0253
<150> DE102008035152
   <151> 2008-07-28
<160> 48
<170> PatentIn version 3.3
<210> 1
   <211> 697
   <212> DNA
   <213> Pseudomonas aeruginosa
<400> 1
   atggttcttg catcgatatt cgcctggctg tgcatgtccg cccatgccga tctctcgttc 60
   gacggccaga accgtttcgt gatgaaaggc aagcgcctgc cgatcacgct ggtcaacgag 120
   ggcaagggcc ggccctggcg gaaatctccc tggattgggg taccgacggc aaggggcagg 180
   cgctgccctt cgccgtgtcc aggccgctgc tccgcctggg cgccggccag cgggccaagg 240
   tggaggtgct ctacagggcc agggcctgcc caccgaccgc gagacctacc tgctgttgag 300
   cgtgctggac gtgccgcacg cgcccagtac tcccgagacc ctgcagatcg ccctacgcca 360
   tcgcttcaag ctgttctatc gccgcccctg gaagccaccg tcgagcaggc gatcgagggc 420
   gtgacgtgga cgctcggcac cagcggcaat ccgaaggcac acaatccttc gccctaccac 480
   atcaccttca gccagttgga gttcctcgcg catcggagcg gagctgcggc gaagccatcg 540
   aacacctgat gctggcgccc ttctccaatc accagttcga catcgccgcc tgccatcccg     600
   acaaagtgcg ttacgcgatc gtcagcgacg gagggacctc cacccttatc acggcgcatt     660
   gaaatcaggc gtagagacca acgccaaaca cccatga 697
<210> 2
   <211> 507
   <212> DNA
   <213> Pseudomonas aeruginosa
<400> 2
   ttgagcgctc tcctcaagcc cctcgacaat cgcttgttct ggcccgcggt cgccatcggc 60
   ggcctgctga tcctcgcttt cgtcctctac ctccagcacg tgcgcggctt cgcgccctgc 120
   tcgctgtcat cttcattcgc ctggacgtgc tcggcctggt gctcgccggg atcgtcggca 180
   gcctcgcccc gcgttcgcgg atcgccggcg gcatcgccgc gctgggcatg ctcgccgcca 240
   gcctgggcgg gatcaccacg cctggtcgct ggtcgcggaa gagaaactgg ctgcccaggg 300
   catgggcagt tgcaagatgt tcatgggctt ccccgaatgg ataccgctgg atacctggct 360
   gccgcaggtc ttccagcccg agggctgtgc ggcgaagtgg tctggaccct gctcggccag 420
   tccatggcgg tctggtcgct ggcgctgttc gtgttctgcc tgctggtcct ggcggccaag 480
   ctggcgttcg gccgccgcac cgcctga 507
<210> 3
   <211> 1283
   <212> DNA
   <213> Pseudomonas aeruginosa
<400> 3
   gtgaagatca agctatgtaa ccggctgcgc ccccttgcat tgggcgcagc actcctttgc      60
   tccttcgcgc atgccgaagt ggtaccactg gaccgggtcg tcgccatcgt cgacaacgat     120
   gtgatcagca gagccagctc gaccagcgcc tgcgtgaagt gcaccagacg ctgctcaagc     180
   gcggtgcgcc gctgccgccc gagcacgtgc tgacccagca ggtgctggaa cgcctgatca     240
   tcgagaacat ccagagcaga tcggcgaccg ctccggcatc cgcatctccg acgaggagct     300
   gaaccaggcg atgggcacca tcgcccagcg caacggcatg agcctggagc agttccagac     360
   cgccctcacc cgcgacggct ttcctacgcc gacgcccgcg agcaggtccg tcgcgagatg     420
   gtgatcagcc gtgtgcgcca gcgccgggtc gccgagcgca tccaggtcag cgagcaggag     480
   gtgaagaact tccttgcctc ggacatggca agatccagct ttccgaagag taccgcctgg     540
   ccaatatcct gatcccggtg ccggaagccg cgtcctcgga cgtgatccag gccgccgcaa     600
   ggcaggccca ggagctctac cagcagctca agcaggcgcc gacttcggcc aactggcgat     660
   ttcccgctcg gccggcgaca acgccctgga aggcggcgag atcggctggc gcaaggccgc     720
   ccagctgccg cagccgttcg acagcatgat cggctccctg gcgtcggcga cgtcaccgag     780
   cctgtccgca cccccggcgg cttcatcatc ctcaagctgg aagagaagcg cggcggcagc     840
   aagatggtcc gtgacgaagt gcatgtccgc catatcctgc tcaagcccac gaaatccgca     900
   gcgaagcgga gaccgagaag ctagcgcaga agctgtacga gcgcatccag tccggggaag     960
   acttcggcga actggcgaag agcttctccg aagatccggg ttccgccctc aacggcgcga    1020
   cctgaactgg atcgatccgg aagccctggt gcccgagttc cgccaggtga tgaacgatac    1080
   cccgcagggc gagctgtcca agccgttccg ctcgcagttc ggctggcaca tcctgcaggt    1140
   cctggccgtc gcgccaccga cagcagcgag aagttccgcg agcagcaggc ggtaagcgta    1200
   ctgcgcaacc gcaagtacga cgaggagctg caagcctggc tgcggcagat ccgcgacgag    1260
   gcctacgtgg aatcaagcag tga                                            1283
<210> 4
   <211> 939
   <212> DNA
   <213> Pseudomonas aeruginosa
<400> 4
   atgcggccgg catttcgttg cggcatcctg gcgttcgcgc ttgccgttct cgccggctgt      60
   ggccagcgcg aggacggcgg cgcgggccag gcccaggccg cgcttgccaa gccatctgcc     120
   agcgccgcgc ggagcccgcg gtggcccgcc tcggtgcctt gcaggtggcg ccggacgaac     180
   tgaaggcgct gctggccgag gtgccggcgg aaacccgcgc gcagttgagc gagaaccgcg     240
   acgcgatgga acgcggatgc gtgcgcgact ggccgagaag gcgctctacg aacaggccag    300
   cgcccaggac tggcaacagc gtccgcaggt gaagacgctg atcgatgcgg cggcgcggca    360
   gatcgtcctg cgtacctacc tgagtcggtg agcacggtgc ccgaggacta ccccagcgat    420
   gccgaactgc aggccgccta ccaggcgaac aaggcgcaac tggctgtacc ggcgctgtac    480
   cgggtcagcc agatcttcat cgccgccttg cggccggcgg gcttgccgaa gcgcgcaagc    540
   gcgcccagga actctaccgg caggccgcgg acggcgactt cgccgaactg gcgcgcaagc    600
   attccgacga cccgcagacc gcccgcaacg gcggcacatc ggtgggttac tggcgcaggc    660
   gcaactgctg ccggcgatcc gtccggcgct ggaacggcta aaggtcggcg cggtgtccga    720
   gccgatccag ggcgccaacg gtttccacct ggtcaagctg accgagcgtc gcgacgcccg    780
   cctcgccacc ctcgacgagg tccgtggtcg cctgcgcgaa agcctgcgcg cgcaacgcca    840
   ggagcagata gccaaggcct acctggatgg ccggtgaaca acgctaccct gagcatcgac    900
   ggcgcgttgc tcggcaagac cctggcggaa ctgcactga                           939
<210> 5
   <211> 483
   <212> DNA
   <213> Pseudomonas aeruginosa
<400> 5
   atgcagatcg cggccaacaa ggcggtttcc atcgactata ccctgaccaa cgacgccggg     60
   gatgtcatcg acagttccgc tggtggcgcg ccgctggtct acctgcacgg tgccggcaac    120
   atcatcgcgg cctggagaag gccctggaag gcaagaacgt cggtgacgaa ctgagcgtcg    180
   ccatcgagcc ggaagacgcc tatggcgaat acagcgccga actggtcgcg accctgaccc    240
   gcgagatgtt cgaagcgtcg acgaactgga agtcggcatg cagttccacg cctcggctcc    300
   ggacggcggc atgcagatcg tcaccatccg cgacatcgac ggcgacgacg tgaccgtcga    360
   tggcaaccat ccgctggccg gcagcgcctg aacttcaagg tcaaggtagt cgacgtgcgc    420
   gaggccaacg ccgaggaaat cgcccatggt cacatccatg gcgaaggcgg tcaccaccac    480
   tga                                                                  483
<210> 6
   <211> 1897
   <212> DNA
   <213> Pseudomonas aeruginosa
<400> 6
   gtggcagaat cccgcaccgg accgctcgcg tgcgagcgga cgccattcat cgaacgcttg     60
   gatagggaca tgcaaagcag ctttccgcaa tcgaacactc cccagatccg tcgcgctgga    120
   gtcgacccaa cggcgtgatg tcggtgctca gcaagcacct ctattccacg cccaccgtgg    180
   cgttgcgcga gctggtgcag aacgcccacg actcgatcct ccgtcgccgc ctggagcagc    240
   ccgactggca agggcgtcgc gaatcgaggt gatcggcgac agtgccagca acaccgtgcg    300
   catcgtcgac accggcgccg ggctcaccga gcacgaaatc cacgcctacc tggccaccgt    360
   cggcgtcggc tacacccgcg gctgcgccag tccggccacg aggacagcgg gctgatcggc    420
   atgttcggcc tgggcttcct ctccgctttc gtcctggccc gccgggtcag cgtgcggacc    480
   acgtcctacc agcagccgga actgggctct gctacgtctc cagcaacgcc gagcaataca    540
   gcgtgacgcc gatgccggcg cgcccggtgg gtaccgagat caccctcgaa ctgcaggacg    600
   aatacagtgc gctgacccag gccgcgcgcc tgcgcagatc ctcgaacgct actgcgtgct    660
   gctgcgcgag ccgatccacg tcggcggcga cgcccagtcg atcaacccgg agccgccgcc    720
   gtggcgactg gccgccgacg ccgcgctgca cccgctgcaa cgcagcgccg cgacctggag    780
   ttcgccgcac gcttcgagca cgacttcgag ccgatctgct gcctgccggt gcgcccggac    840
   gagcgggtcg acgtgcaggg cctgctgtgg atccaggacg gcggcacctt ggcaccagcg    900
   acaaccgcaa tctctcggtt ttcctccgcg gcatgctgct cgacgatgac gcccgcgacc    960
   tgctgccgtc ctgggccggg ttcgtcggcg gggtgatcga atcgaaccgc ctgacgccac   1020
   cgccagccgc gaggacctgc agcgtgacga ccattacgcg gcgatccagc atgcgctggc   1080
   cgaggcgctg atcgctgggc tcggcgaggt ggcgcggcag cagccggagg cctggcggcg   1140
   ggtctgctgc ggcacaacga ggccctgctg ggagcggcgt tgtgcgacga gcgtctgttc   1200
   gcgctgatga tggacagcct gcgcgtaccc acctcccagg gcgacctccc ggcttccgaa   1260
   ctcctttcgc cggagcggtg catgtgctgc tggacaacga cagcggcttc gaggaaatgc   1320
   tgttccgtgc catgggcgta ccggtggccc atggcaatcg ctatgcggtg gtacccttcc   1380
   tccgtcgctg ggcgcagcca aggggatgcg cctggtggag ctgggcaccg agcagggcaa   1440
   ccgccagttg ttccgcctcg accgactgcc cgagcacgag accggctggc tggccgaaca   1500
   cctgggcggc gccgaggagc aactgtgatc gcccgcttca gccccgagga attgccgctt   1560
   gtggtggtgc ccgaccgcga cgccgaactc aagcggcgac tggaggacga cgaggccgac   1620
   aagcgtattt ccaccgcggc gttgcgcctg gccggcagtt cacccgcaag ctcgacaatc   1680
   gccagccgag ccggctctac gtcaacctcg acaatccggc ggtgcaggcg ctgctcgcgg   1740
   ccgtcggcga ggaacgcgac gaagcggccc atgcggcgtg ctgctgcgct cgttcaaagt   1800
   gatcatcgcc ggacagggac gcggccagcc cgcgacctcg ctgaaccagg ccctggccgg   1860
   actcgccgat tcggtgaagc gcctgctgga gcaataa                            1897
<210> 7
   <211> 539
   <212> DNA
   <213> Pseudomonas aeruginosa
<400> 7
   ttgaaacgag cgattctgct gctgccgctg gggatcttcc tgatcgtcgc ggtgttcctc      60
   ttccgcggcc tctggctgga ccccagcgag ctgccctcgg cgctgatcgg caagccgttc     120
   ccggcctcga cctgcccagc gtgcaggacc cggcccggcg cctgaccgag gccgacctca     180
   agggcaagcc ggcgctggtc aacgtctggg gcacctggtg cccgtcgtgc cgggtcgagc     240
   acccggaact gaccgactgg ccgagcaggg cgtggtgatc tacgggatca actacaagga     300
   cgacaacgcc gccgccatca agtggctgaa cgagctgcac aatccctacc tgctgagcat     360
   cagcgacgcc gacggcaccc tggcctggac ctgggcgtgt acggtgcgcc ggaaacctac     420
   ctgatcgaca aacagggcat catccgccac aagatcgtcg gggtggtcga ccagaaagtc      480
   tggcgcgagc aactggcgcc gctgtaccgc aactgctgga cgagccggag gcccgatga      539
<210> 8
   <211> 1891
   <212> DNA
   <213> Pseudomonas aeruginosa
<400> 8
   atgagcgtgg aaacccaaaa agagacactg ggctttcaga ctgaagtgaa gcaactgctt 60
   cacctgatga ttcattctct ctattcgaac aaggaaatct tcctccgcga actgatttcc 120
   aacgcctgga cgccgccgac aagctgcgtt tcgaagccct ggccaatccc gagctgctcg 180
   agggcggcgc cgagctgaag atccgcgtca gcttcgacaa ggaagccaac accgtcaccc 240
   tggaagacaa cggctcggca tgagccgcga ggatgtggtg actcacctgg gcaccatcgc 300
   caagtccggg accgccgact tcctgaagaa cttgtctggc gaccagaaga aggattcgca 360
   cctgatcggc cagttcggcg tggtttctac agcgccttca tcgtcgccga caaggtcgat 420
   gtctactccc gtcgcgccgg ccagccggcc agcgaaggcg tgcactggtc gtcgaagggc 480
   gagggcgagt tcgacgtcgc caccatcgca agcccgagcg cggcacccgc atcgtcctcc 540
   acctgaaaaa gggcgaggag gagttcgccg atggctggcg cctgcgcaac gtgatcaaga 600
   aatactccga tcacatcgct ttgccgatcg agctaccaag gaattccacg gcgaagaggc 660
   cgacaagccg gccgagccgg aatgggaaac ggtcaaccgc gccagcgcgc tgtggacccg 720
   tccgcgcgcc gaggtgaagg acgaggaata ccaggagttc taaagcacgt cgcccatgac 780
   ttcgagaacc cgctgagctg gagccacaac aaggtcgaag gcaagctgga atacacctcg 840
   ctgctctacg tgccgggccg cgcgccgttc gacctgtacc atcgcgaagg ccgcggggcc 900
   tgaagctgta cgtacagcgc gtgttcatca tggaccaggc cgacgagttc ctgccgctgt 960
   acctgcgctt catcaagggt gtggtcgact ccaacgacct gtcgctgaac gtctcccggg 1020
   aatcctgcag aaggatccgg tgatcgactc gatgaagtcg gcgttgacca agcgcgtgct 1080
   ggacatgctg gagaagctgg cgaagaacga gccggagcag tacaagacct tctggaagaa 1140
   cttcggcagg tcctcaagga aggcccggcg gaagacttcg gcaacaagga gaagatcgcc 1200
   ggcctgctgc gcttcgcctc caccggcgac gattcgggcg agcagagcgt agcgctggcc 1260
   gactacatcg gtcgatgaag gaaggccagg acaagatcta ctacctcacc ggcgagagct 1320
   actcgcaggt gaagaacagc ccgcacctgg aagtcttccg caagaagggc atcgaggtgc 1380
   tgctgctcac cgatcgcatc gcgagtggct gatgagctac ctgccggagt tcgacggcaa 1440
   gcagttcgtc gatgtcgccc gcggcgacct ggacctgggc agcctggact cggaagagga 1500
   caagaaggcc caggaagaag tcgccaaggc aaggaaggcc tgatcgagcg cctgaagaag 1560
   gtgctcgacg agcaggtcag cgaagtccgc gtttcccacc gcctgaccga ttcgccggcg 1620
   atcctcgcca tcggcgaaca ggaccttggc ctgcaatgcg ccagatcctc gaggccagcg 1680
   ggcagaaagt gccggactcg aagccgatct tcgagatcaa cccgcagcat ccgctgatcg 1740
   agaagctgga tgcggagccg gacgaggacc gcttcggcga gcgtcgcata ttctcttcga 1800
   ccaggccgcc ctggccgcgg gggacagcct caaggatccg ggcgcctacg tacgccgact 1860
   gaacaagctg ctggtggagc tgtccgctta a 1891
<210> 9
   <211> 495
   <212> DNA
   <213> Pseudomonas aeruginosa
<400> 9
   atgatcaagc tgcataccaa ccatggcgtc atcaccctga agctcgatga agagaaagcc      60
   ccggaaaccg cggcgaactt caaggagtac gtgaaaagcg gccactacga caacaccgtc     120
   ttccacccgt gatcaacaac ttcatgatcc agggcggcgg tttcgagccg ggcatgaagc     180
   agaagcccac tcgcgcaccg atcaagaacg aagccaacaa cggcctgacc aacaaggtcg     240
   gcagcatcgc catgctcgta ccatggaccc gcattcggcc agcgcgcagt tcttcatcaa     300
   cgtcgccgac aacgatttcc tcaaccatac cgcccccacc acccagggct ggggctatgc     360
   cgtcttcggc gaagtggtcg aggcatggac gtggtcaaca agatcaaggg cgtggccacc     420
   accatgcgca gcggccacca ggacgtgccg gccgaggacg tgatcatcga gaaggccgaa     480
   atcgtcgaag aatga                                                      495
<210> 10
   <211> 1301
   <212> DNA
   <213> Pseudomonas aeruginosa
<400> 10
   atgcaagttt ctgttgaaag cacttccgct cttgagcgcc gcatgaccgt tggtgtgcct      60
   gccgagcgta tcgagaccga agtcaacaag cgtctgcagc agaccgcccg tcgtgccaag     120
   attccagttt ccgccccggc aaggtgccga tgagtgtcat tcgtcagcgc tacgaagctt     180
   ccgcgcgcca ggaagccatg ggcgatctga tccaggaaac cttctacgaa gccgtggtcg     240
   agcagaagct gaatcggccg gttcgccgtc ggtagagccc aagtccttcg agaaaggcaa     300
   ggacctggaa tacatcgcaa ccttcgaagt cttcccggaa tttaccgttt ccggcctgga     360
   agacatcaag gtcgagcgcc tcaggccgaa gtttccgatg ctgatgtcga caatatgctc     420
   gacgtcctgc gcaagcagaa cacccgtttc gaagtagtcg agcgtgccgc ccagaacgat     480
   gaccagttga acatcgactt cgtcggcaga tcgacggcga agccttcgcc ggcggttccg     540
   ccaaaggcac cctgctggta ctgggctccg gtcgcatgat cgccggcttc gaagaagggc     600
   tggtgggggc gaaggccggt gaagagcgtg tgctcacctg accttccccg aggactacca     660
   gaacctggac ctggccaaca aggctgccga gttcaccgtt accgtcaata gcgtagccga     720
   gcccaagctg ccggaactga acgaagagtt cttcgctctg ttggcgtgaa ggaaaccggt 780
   ctcgatggct tccgcgccga agtgcagaag aacatggagc gcgagctgcg ccaggcgatc 840
   aagtccaagg tcaagaacca ggtcatggaa ggtctgctgc aggccaaccg atcgaagtgc 900
   ccaaggccct gatcggcaac gaagtgaacc gtctgcgcgt gcaggccgtc cagcagttcg 960
   gcggcaacat caagcctgac caactgccgg ccgagctgtt cgaagagcag gccaaggccg 1020
   tgtggtgctg ggcctgatcg tcgccgaagt ggtcaagcag cacgagctga aggccgacga 1080
   aggtcgcgtt cgcgaaatga tcgaggaaat ggcctcggcc taccaggagc ccgagcaggt 1140
   cgtgcctggt acttcaagaa cgagccccag ctgaacgaag tccgttctgt tgtactggaa 1200
   gaacaagtcg tagatactgt cctgcaaaag gccactgtga ccgataagca ggtatcctac 1260
   gaagaagcgg caagccggcc gaagctccgc aagcggcctg a 1301
<210> 11
   <211> 637
   <212> DNA
   <213> Pseudomonas aeruginosa
<400> 11
   atgtctcgca actcttttat tccgcacgtt cccgatatcc aggccgccgg tggcctggtg 60
   cccatggtgg tggagcagtc cgcccgcggc gagcgcgcct acgacatcta ttcgcgcctg 120
   ctgaagggcg gatcatcttc ctggtcggcc aggtcgagga ctacatggcc aacctggtgg 180
   ttgctcagtt gctgttcctg gaggctgaaa atcccgagaa ggacattcat ctctacatca 240
   actcgccggg tggtcggtga ctgccgggat gtccatctac gacaccatgc agttcatcaa 300
   gcccaacgtc tcgaccacct gtatcggcca ggcgtgcagc atgggtgccc tgctgcttgc 360
   gggcggtgcc gccggcaagc gtactgcctg ccgcattcgc ggatgatgat ccaccagccg 420
   ctgggcggtt tccagggcca ggcctcggat atcgagatcc atgccaagga aatcctcttc 480
   atcaaggagc gtctgaacca gatcctgggc accacaccgg ccagcccctg gacgtcattg 540
   cccgcgatac cgatcgtgac cgctttatga gcggtgacga agccgtcaag tatggcctga 600
   tcgacaaggt catgacccag cgcgacctgg ccgtcaa 637
<210> 12
   <211> 1272
   <212> DNA
   <213> Pseudomonas aeruginosa
<400> 12
   atgactgata cccgcaacgg cgaggacaac ggcaagctgc tgtattgctc cttctgcggc    60
   aagagccagc acgaagtgcg caagttgatt gccggcccct cggtctttat ctgcgacgag   120
   tgtgtcgcct gtgcaatgac atcatccgcg aggaggtgca ggaagcgcag gcggaaagta   180
   gtgggcacaa acttccggcg ccgaaagaga tccgcaccat cctcgatcag tacgtgatcg   240
   ggcaggagcg ggccagaagg ttctggcggt agcggtatac aaccactaca agcgcctcaa   300
   ccagcgtgac aagaaggacg atatcgagct cggcaagagc aacattctga tgatcggccc   360
   aaccggctcg ggcaagaccc tctggccgag accctcgcac gtctgctgaa cgtgccgttc   420
   accatcgccg atgcgaccac gctgacggaa gcgggctacg tgggcgagga tgtcgagaac   480
   atcatccaga aactcttgca gaagtgcgtt acgatgtgga gaaggcccag atgggcatcg   540
   tctacatcga cgagatcgac aagatttccc ggaaatcgga caacccctcc atcactcgcg   600
   acgtctccgg cgagggtgtg cagcaggccc tgctgaagct catcgagggt accgttgcct   660
   cggtaccgcc gcagggcggt cgcaagcatc cgcagcagga gttcctgcag gtcgatacgc   720
   gcacatcctg ttcatctgcg gcggcgcttt cgccggtctg gagcgggtca tccagaaccg   780
   ttccgcgcgc ggcggcatcg gtttcaatgc cgaagtgcgc agtcaggaga tgggcaagaa   840
   ggtcggcgag cgttcaagga agtcgaaccg gaagatctcg tcaagttcgg tcttattccc   900
   gagtttgtag ggcgtttgcc ggtgatcgcg actcttgatg agctcgacga ggctgcgctc   960
   atgcagatcc tgaccgaccg aagaacgctc tcaccaagca gtacgccaag ctgttcgaaa  1020
   tggaaggcgt cgatctcgaa ttccgtccgg acgcactcaa ggcggttgcc cgcaaggcgc  1080
   tcgaacgcaa gaccggtgca cgtgcttgcg ttcaattctc gaaggtatcc ttctcgatac  1140
   catgtacgag attccctccc agcaggatgt cagcaaggtg gtcatcgatg agagtgtcat  1200
   cgatggctcg tcgcaacccc tgatgatcta cagaacagcg aaaagccagc caaggctgcg  1260
   cctgaggcat ga                                                       1272
<210> 13
   <211> 1852
   <212> DNA
   <213> Pseudomonas aeruginosa
<400> 13
   atgctgcaga acatcaggga taattcccag ggctggatcg ccaagaccat cattggcgtg 60
   atcatcgttc tcctgtcgct gaccggcttc gacgcgatca tccgggccac cgaccactcc 120
   aacgtggcgc caaggtcaac ggcgacgata tcagtctcaa tgaagtccaa caggccgtgg 180
   acatgcagcg tcgccagctg ctgcaacgcc tgggcaagga tttcgatcca tccatgctgg 240
   atgacaagct gctcaggaag cggccctgaa ggggctgatc gagcgtaccc tgctgctcca 300
   ggccgccaag gacgacaagt tcgccttctc cgaccaggcg ctggaccagt tgatcctgca 360
   aactcccgag ttccaggtcg aggcaagttc aacgcggatc gcttcgacca ggtcatccgc 420
   cagatgaact acagccgcat gcagttccgc cagatgctcg gccaggaaat gctcatcggc 480
   cagcttcgcg ccggcctggc gggcaccgtt tcgtcaccga caacgaattg cagtccttcg 540
   ctcgcctcga gaagcagacc cgcgacttcg ccaccctggc gatcaaggcc gacgcctcca 600
   agagcagcgt gagcgacgac gaggtgaagg ccttcacgaa ggccacaaga gcgagttcat 660
   gactcccgag caggtggtcg tcgaatacgt ggagctgaag aagtcctcct tcttcgacca 720
   ggtcaaggtg aagcaggaag acctcgaggc gctgtaccag aagaaatcgc caacctttcc 780
   gagcagcgcg atgccgccca catcctgatc gaggtgaacg acaaggtcgg cgacgagcag 840
   gccaaggcga agatcgacga gatcaaggct cgcctggcca agggcgaggt ttcgccgcgc 900
   tggccaagga gttctcccag gatatcggct cggccgccac cggcggcgac ctgggctacg 960
   ccggtcgcgg cgtgtacgac cccgcgttcg aggaggcgct gtatgcgctg aagcaagtga 1020
   ggtatccgcc ccggtgaaga ctccgtacgg ctaccacctg atcaagctgc tgggcgtgca 1080
   ggcgccggaa gtaccgagcc tggaaagcct caagccgaag ctcgaggacg aactgaagaa 1140
   acaatggtcg agcagcgctt cgtcgaggct accaaggacc tggaaagctc cgcctacgaa 1200
   gccgccgacc tgagccagcc ggcgcaggaa atgggcctga aggtccagac cagccagccg 1260
   ttcggacgtt ggggggcgac ggcatcgctg ccaaccgcca gatcgtgcag accgcgttca 1320
   gcgccgaggt gctggaagaa gccgccaaca gtggcgccat cgagctggat ccggacaccg 1380
   tggtggtgct gcgggtcagg aacacaacaa gccgaaggag caaccgctgg agcaggtcgc 1440
   ggcgaacatc cgcgagcgcc tggccgccga aaaggccgcc gaggaggcgc agaagcgtgg 1500
   cgaggccctg atcgcagagc tgcggaaggc cgtacctctt ccgcagcggg tgagtcgtgg 1560
   aaagtggtcg aggcggcctc ccgcggccac gaaggcgtcg atccgaaact gctccaggcg 1620
   gtgttccgca tgcagcgtcc ggaggccaag gcaagccttc gttctctggc gtgaccctgg 1680
   ccaatggcga ttacgtggtg atccgcctga atggcgtcag cgagccggag gaggctatct 1740
   ccgacgacga gaaggccatg taccgccgct tcctggctcg cgcagcggac aggcagactt 1800
   cgccgccttc cgccgtcagt tgcaggacaa ggcggaagtc gagaaatact ga 1852
<210> 14
   <211> 277
   <212> DNA
   <213> Pseudomonas aeruginosa
<400> 14
   atgcccgtcg ccatggcccg ccacatcctg gtcaagaccg aagccgaggc cgcgcaactg 60
   aagcagcgcc tggccaaggg cgaggacttc gccacgctgg cgaagaagca ttcgacctgc 120
   ccgtccgcaa gcgcggcggc gacctcggcg aggtgcgccc ggggcagatg gtcaggtcca 180
   tcgacaatgc catcttcagg aaacccgtgg gcgtgctcca gggtccgctg aagagccagt 240
   tcggctacca tctctggaag tgtatttccg cgactga 277
<210> 15
   <211> 765
   <212> DNA
   <213> Pseudomonas aeruginosa
<400> 15
   atgagactgc cgcgcaacct gatcaccctt ggcctgggcc tgaccctcct caacgccggc 60
   ctggccacgg cggcggagga gctgccggcg ccgatccgca tggtccagga gaagggcgcc 120
   cgcatcccgg cagcttcgac gccccggatg gcctgcgcgg ctacgccgcc gaataccaga 180
   accagggcat ggcgctgtac ctgacgcccg acggcaagca cgtactcacc ggccacctgt 240
   tcgacgccca gggcaggacc tcagccgcga accgctggag cgcctggtgt atgcgccgct 300
   ggccaaggaa atgtggcaga agatggagca gagcgcctgg atcgccgatg gccgtgccga 360
   cgcgccgcgc gtggtctacc tttcagcgac ccgaactgcc cgtactgcac catgttctgg 420
   gagcaggcgc gaccctgggt ggatgccggc aaggtgcagt tgcggcatat catggtcggc 480
   atcatccgcg aggacagcga agccaagtcg ccgcgctgct ggccagcaag gacccgcaga 540
   aggccctgca cgaccatgag caggcgggca aggccagcac cctgaagccg ctggcgaaga 600
   tcccggcggc ggtgcgcaag cagctcgccg gcaactggaa ctgatggagt cgatgggcgc 660
   cgcggcgacc ccggcgatct tctatctcaa tgccgagggg cgcatgcagc agcaacaggg 720
   cgcgccgcag ccggaccagt tggcggagat cctcggtccg cgtga 765
<210> 16
   <211> 623
   <212> DNA
   <213> Pseudomonas aeruginosa
<400> 16
   atgcgactga tccgcacgtt gttcgttgcc gccctggcca tgggcgcttc gctggcccat     60
   gccgacgaca gcgccgccgt ccagcgcctg accggcctgc tgaacaaggc ccagaccctg    120
   accgcgcttt ttcccagctg accctggacg gtagcggcac gcgcctgcag gaaaccgccg    180
   gccaactgag cctgaagcgg ccgggactgt tccgctggca taccgatgcg ccgaacgagc    240
   aattgctcat ttccacggcg agaaggtctg gctgtacgac ccggacctgg agcaggtgac    300
   catccagaag ctcgatcagc gcctgaccca gacgccggcg ctgctgctct ccggcgacat    360
   ctcgaagatc agcgaaagct tgccatcacc tacaaggaag gcggcaacgt agtcgatttc    420
   gtgctcaagc cgaagaccaa ggacaccctc ttcgacacct tgcgcctgtc gttccgcagc    480
   ggcaaggtga acgacatgca gatgatcgtg gcgtcggcca gcgcaccaac atcctgttct    540
   tcgacgtgaa gatgaacgag gcgctggatg ccaagcaatt caccttcgac gttccgccgg    600
   gcgtcgacgt gatccaggag taa                                            623
<210> 17
   <211> 1870
   <212> DNA
   <213> Pseudomonas aeruginosa
<400> 17
   atggccctcg gggcaatatg ctgtggccac gacacacgct gtgagcgact atcgatgcca     60
   gggactgcac tgttcgaaaa gccacgctgg ttgcgcgacc tcctgcgctt cctgcccctc    120
   aagagccatt cgtcctctcc gggaacatcc gcgacctgca agcctgcgaa gtggtgccgg    180
   gtacggtcac cgcgcagagc ttcaaccaga ccctctgcga cgccctgctg gacgccggct    240
   acgcccaggt gctgcctggg atccgctggc cgggttccgc gtgctcggcc ggccgggcag    300
   cgaggcgggg gccactccgc aggtgcttct cgacctcggc ctgaccccgg tggacggcgc    360
   cgcgccggcc ggcatcgacc tctcggcgcg accctgcaac gcctggtcaa ccgtagcggc    420
   gagccgatcg ccctgatcgt cgacttcgcc tcgcgcctgg cggtgcgcaa cgacgccctg    480
   agcgccgccg agcaccagtt gttcacccgg ccctggtgct ctcccaccag gcccgcagcc    540
   gtcccgccgg ggaacagcgc aaaccgttct tcaacagcgt gctgtgggtg gtggaaaagg 600
   aaggcgacct gcccgactgg ctgctggtgg acaaccgcgc ctgcgccata tcccggtgtc 660
   gaagcccgac cagccggcgc gccgggccct ggccccggcg ctgctccgcg ggctaggcgg 720
   cgcgggcgtc gccgaggagg ccctgcaaca ggccgccgca acttcgtcga gaacaccgaa 780
   ggcctgctac tcctcgatct caacgccatc gtccagttgg cgcgcgtgga aggcctggcc 840
   atggaacgca tcgccgacgc cgtgcgccgc tacaaggtcg gcgtcaccga gacccctggc 900
   tgaagatcga ccgccagcgc atccgccagg ccgacgagat cgtccgccgc cgggtcaagg 960
   gccagcagca cgcggtgacc cacatgctgg acatcgtcaa gcgcgcgatg accgggtcgg 1020
   cgccagccgc aagggcaacc gcccgcgcgg cgtggccttc ctcgccgggc ccaccggggt 1080
   cggcaagacc gagctggcga agaccgtcac cagcctgctg ttcggcgacg agagcgccta 1140
   catcgcttcg acatgtccga gttcagcgcc gagcatgccg accagcgcct gatcggcgcg 1200
   ccgcccggct acgtcggcta cgacgtcggc ggcgaactga ccaacgcgat ccgcgaaaaa 1260
   cccttcagcg ggtgctcttc gacgagatcg agaaagccca cccccggatc ctcgacaagt 1320
   tcctgcagat cctcgacgac ggcgtgctca cctccgggcg cggcgatcgc gtgtacttct 1380
   ccgaggcgct gatcgtgtca cctcgaacct gggcatctac cgccaggggg aaaacggcga 1440
   acgagtcgcc aacgtgctcc ccggcgagcc cttcgaggcg gtgcagggca aggtccacgg 1500
   cgagatcgag cgctacttca agctgttctc aaccgcccgg aaatcctcaa ccggatcggc 1560
   gagaacatta tcgtcttcga cttcatccgc gccgacgtcg ccgagcagat cttcacccag 1620
   atggtcaacg gcaccttcgc cgacctccgc ggcagcgcct cgtcatcgaa ctggccgagg 1680
   cgccgcgcca ggcgctgcac gacctctgcc tgggcgacct gtccaacggc ggccgcggca 1740
   tccgcaacca gctcgaagcg cgcctgctca acccgttgcg cgggcactgt tcgaccagga 1800
   cgcgcagccg ggcgagcgct tcctgatcag cgccctggac gccgacggcc tgaccctgga 1860
   acgccgctga 1870
<210> 18
   <211> 861
   <212> DNA
   <213> Pseudomonas aeruginosa
<400> 18
   gtgccggccc ctcaggtcat gccggccaag gtcgcgccgc tgccaacctc cttcaggggc      60
   accagcgtcg atggcagttt cagtgtcgac gccagcggca acctgctgat cacccgcgac     120
   atccgcacct gttcgactac ttcctcagcg ccgtcggcga agagcccctg cagcaaagcc     180
   tggaccgcct gcgcgcctac atcgccgccg aactccagga gccggcgcgc ggccaggcgt     240
   tggcgctgat gcagaataca tcgactacaa gaaggaactg gtgctgctcg aacgcgacct     300
   gccgcgcctg gccgacctcg acgccctgcg ccagcgggaa gccgcggtga aagccctgcg     360
   cgcgcggatc ttcagcaacg agcgcacgtg gcgttcttcg ccgacgagga aacctacaac     420
   cagttcaccc tggagcgcct ggcgatccgc caggacggca agctcagcgc cgaggaaaag     480
   gccgccgcca tcgaccgcct gcgcgccacc tgccggaaga ccagcaggaa agcgtgctgc     540
   cgcaactgca aagcgaactg cagcagcaga ccgccgccct ccaggccgct ggcgccggcc     600
   cggaagccat ccgccagatg cgtcagcaac tggtgggcgc cgaagccaca cccgcctgga     660
   gcaactcgat cggcaacgct cggcctggaa gggccggctg gacgactatt tcgccgagaa     720
   gagccggatc gaaggcaata ccgggctgag cgaagccgac cgccgcgcgg cggtcaacgc     780
   ctggccgagg agcgcttcag cgaacaggaa cgcttgcgcc tgggcgcgct ggaacagatg     840
   cgccaggccg agcagcgctg a                                               861
<210> 19
   <211> 447
   <212> DNA
   <213> Pseudomonas aeruginosa
<400> 19
   atgagcaacg ctttttccct cgccccgctg ttccgtcatt ccgtaggctt cgatcgcttc      60
   aacgatctgt tcgagtcggc cctgcgtaat gaggccggga gtacctaccc gccctacaac     120
   gtcgaaagca cggtgacgac gagtatcgca tcgttatcgc cgccgccggc ttccaggaag     180
   aagacctgga cctgcaggtc gagcgcggcg tgctgaccgt cagtggcggc aagcgcgaga     240
   agtccacgga caactgacct acctgcacca gggcatcgcc cagcgcgcct tcaagctgtc     300
   gttccgcctc gccgaccata tcgaggtcaa ggcggcttcg ctggccaacg gcctgctgaa     360
   catcgacctg gtccggctgg tccggaagaa gccaagccga agcgtatcgc catcaatggc     420
   cagcgcccgg cactggacaa ccagtaa                                         447
<210> 20
   <211> 334
   <212> DNA
   <213> Pseudomonas aeruginosa
<400> 20
   atggacatca tcgaatggca ggatttcgaa cgcgtcgaac tgcgcgtcgg caccatcctc 60
   agcgccgctc ccaaccccaa ggcacgcaaa cccgcctatg tcctcgaggt cgacctcgga 120
   ccgctcgcct gcgcacctcc agcgcgcaac tgaccgcaca ctaccaggcc gagcaactgg 180
   tcggacgcca ggtcctctgc gtctgcaact tcgctccgaa gcagatcgcc ggggtgcgct 240
   cggaggtcct ggtcccgggg tctacgatag cgaggacaag gtggtcctgg ccggcttcga 300
   caagcccctg cccaacggtg cgcgcctggc ctga 334
<210> 21
   <211> 560
   <212> DNA
   <213> Pseudomonas aeruginosa
<400> 21
   atgctcaaac gtctcctgat cgccgcctgc accctgctct tcgccggttc cctgctggcc 60
   gccacgcctg ccgccaagcc gcacgtcctg atcgccacca gcctcggcga gatcgaggta 120
   gaactcgtcc ggccaaggcg ccgatcagcg tgaagaactt cctcgagtac gtcgacagcg 180
   gctactacga cggcaccctg ttccaccgcg tgatcccggg cttcatggtg cagaccggcg 240
   gcttcagcgc cggctgcagg agaagaagac ccgtgcaccg atcaagaacg aggccgacaa 300
   cggcctgctc aacgagcgcg gcaccctcgc catggcccgt accggagtgg tcgattcggc 360
   caccagccag ttcttcatca actgaccgac aacgacttcc tcaaccatgg tgcccgcgac 420
   ttcggctatg ccgtgttcgg caaggtggtg cgtggcatgg gcgtggtcga ccagatcgcc 480
   aaggttccca ccacccgccg caacggctcg ccgacgtgcc gagcgacgac gtggtgatcc 540
   tgtccgccaa acgcctctga 560
<210> 22
   <211> 757
   <212> DNA
   <213> Pseudomonas aeruginosa
<400> 22
   atgaaacaac atcggttggc agcagcgatc gcactggtcg gtctggttct ctccggctgc 60
   gattcgcaga ccaacgttga gttgaagacc ccggcgcaga aagcttccta tggtatcggc 120
   ctgaacaggg caagagcctg tcccaggaag gcatggacga cctggattcg aaagccgtcg 180
   ccaagggcat cgaagacgcc ctcggcaaga agaagcagca gctcaccgat gaagagctga 240
   ccgaagcctt cgcctcctgc agaaacgtgc cgaggagcgg atggccgcca tcggcgacga 300
   aaacgccaag gccggcaaga aattcctcga ggaaaacggc aagcgcgacg gcgtgaccac 360
   caccgcttcc ggcctccagt agaaatcgtg aagaaggccg atggcccgca accgaaggcc 420
   accgacgtgg tgaccgtcca ctacgaaggc cgcctgaccg atggcaccgt cttcgacagc 480
   tccatcgagc gcggcagccc gatcgaccgc cggtcagcgg cgtgatcccg ggttgggtcg 540
   aagccctgca actgatgcac gtcggcgaga agatcaagct gtacatcccc agcgagctgg 600
   cctacggcgc gcagagcccg agcccggcga tcccgccaac tcggtactgg tcttcgacat 660
   ggaactgctc ggcatcaagg atccgtccaa gccggacgcg gagcccgagg ccggtgccgc 720
   agcgcagccc gaagccaagg cggacgcgaa gaagtaa 757
<210> 23
   <211> 1108
   <212> DNA
   <213> Pseudomonas aeruginosa
<400> 23
   atgccttttc tgagcgacat gcttgaccag tcccgccggc agcaggacga ggaacaggcc 60
   ctggcgcggg agaatcttgc cgaggcaagt ctgctccagg cccacctgag tcaccgcagc 120
   gccctgccag ccgtttccgt ttcgacccgg cggcggtgat ggactgcctg cgcgccgagg 180
   tgctcggcca ggatccggcg ctacaggccg tcgaggacat gctcaaggtg gttcgcgcgg 240
   acatcgccga cccggccgtc cgctgttcag cgcgctgttc ctcggcccca ccggggtcgg 300
   caagaccgag atcgtgcgcg ccctggccag ggccctgcac ggcgacgccg aggggttctg 360
   ccgggtggac atgaacaccc ttcccaggag cactatgccg ccgccctcac cggtgcgccg 420
   ccgggctacg tcggggcgaa ggagggcacc accctgttgg agcaggacaa gctggacggc 480
   agtcccgggc gccccggcat cgttctctcg acgaactgga gaaggccagc ccggaagtgg 540
   tccatgcgct gctcaacgtg ctcgacaacg gcctgctgcg ggtcgcttcc ggcgaacgca 600
   cctaccattt ccgcaacacc ctggtgttca tgaccgcaat ctctgcgccc atgagatcca 660
   gcgctacgac gagcgtcgcc agcgcctgcc ctggcgcctg ctgccggtcg gcggcgagcg 720
   ccggcggcgg gacatcgacg ggatggtccg ggcccggctg ctaagacttt ctcgccggag 780
   ttcgtcaatc gtctcgatag cgtggtcacc ttcaactgga tcgaacgcga cgtcgtcgcg 840
   cgcctggtcg agctggaggt gcagcggctc aaccggcgcc tggagaagct cgctgccgcc 900
   tggaggcgac cccggaggtg ctggcgaaga tcgcccgcgc cggcttcgac cggcagttcg 960
   gcgcccgtgc gttgcgccgc agcgtccgtc atcatctcga ggtaccgctg gccgagatct 1020
   gctcgaccac caccagccgg gcgacgggaa ctgcacgacc tacctggcga gcctggagca 1080
   cgagcgggtt cgcttcgtgc ggcgctga 1108
<210> 24
   <211> 504
   <212> DNA
   <213> Pseudomonas aeruginosa
<400> 24
   gtgcgtaagt tcacccagtt cgtcctgatc accgcagcca tcatggcggc tccttcggcg 60
   ttcgctgaaa tgaagatcgc ggtgctcaac taccagatgg cgctgctcga gtccgatgcc 120
   gccaagcata cgccgtggat gccgagaaga agttcggccc ccagctgaac aagctgaaga 180
   acctcgagcg cgacgccaag gcgctgcagg acaagctggt ttccaacggc agcaagatgt 240
   cccagggcga ccgcgagaag gccgagctgg acttcagcag aaggcccgtg acttccagtt 300
   ccagtccaag gaattgaacg agtcgaaggc cgccgccgac cgcgacatgc tgaagaaact 360
   caagccgaag ctggaccagg ccgtcgagga gaccatcaag aaagtggtta cgacatggtc 420
   atcgaacgtg gcgccgtggt cgacgtcaag ccgcaatacg acatcacccg ccaggtgatc 480
   gagcggatga accagctgcg ttga 504
<210> 25
   <211> 340
   <212> DNA
   <213> Pseudomonas aeruginosa
<400> 25
   atgaacgacc gactgcagat cgaagacctc ctcctgggcg acggcaagga ggtagtgaag      60
   ggtgcgctga tcaccaccca gtacaagggc accctggaag acggcacgct gttcgactcc     120
   tcctacggcg cggccggcca ttccagtgcg tgatcggcac cggacgagtg atcaagggtt     180
   gggaccaggg cctgatgggc atgaaggtcg gtggcaagcg acggctgttc gtcccttccc     240
   acctggccta cggcaacggc aggtcggcgc gcacatcaag ccccactcca acctgctgtt     300
   cgagatcgaa ctgctggaag tgctcacccg cgacgactga                           340
<210> 26
   <211> 724
   <212> DNA
   <213> Pseudomonas aeruginosa
<400> 26
   atgcgcgtga cccgttttct ggctgctgcc gccctcggcc tgatgagtac cctggccctg      60
   gcagacaatg ccgatcagaa catccgcaag accctgcagg ccctgcaacc ggacctgccc     120
   atcgactcat cgccagcagc ccgctgcagg ggctctacca ggtgcagctg aagggcggcc     180
   gggtgctcta cgccagcgcc gacggccagt tcgtcatgca gggctacctc taccaggtga     240
   aggacggcaa gccgtcaacc tgaccgagaa ggccgagagc caggcgatcg ccaaggcgat     300
   caacggcgtg cccgccagcg agatggtggt gtatccggcc aagggccagg ccaaggcgca     360
   catcacggtg ttcaccgata cacctgcccg tactgccaga agctgcatgc cgaggtgccg     420
   gacctgaccg agcaggggat cgaggtgcgc tacatggcgt tcccgcggca ggggccgcag     480
   tcggcgggcg acaagcaact gcaggcggct ggtgtgcgaa ggagccgacc aaggcgatgg     540
   acgccatgat gaacggcaag gagatcaagt cttccgagtg caagaacccg gtcgacaagc     600
   agttccagat gggccagatg gtcggggtgc agggacgccg gcgatcgtcc ttgccaacgg     660
   ccagttgctc cccggctacc agccggccaa gcagttggcc aagctggcgc tggaggccaa     720
   atag                                                                  724
<210> 27
   <211> 1846
   <212> DNA
   <213> Pseudomonas aeruginosa
<400> 27
   atggccctac tgcagatcgc cgagcccggt cagagtccca agccccatga gcgtcgcctg      60
   gcggttggga tcgacctggg taccaccaat tcgctggtcg ccgcggtgcg tagcggcgtc     120
   gccgagcctt gccggatgcg cagggacgcc tgatcctgcc ttccgcggtg cgttatcacg     180
   ccgagcgggc ggaagtgggc gagagcgccc gcgcggccgc cgcggaggat cccttcaata     240
   ccgtcatttc ggtcagcgcc tgatggggcg cggcctggag gacgtcaagc aactgggcga     300
   gcaattgccc taccgcttcc gccagggcga gtcgcacatg cccttcatcg agaccgtaca     360
   ggggttgaag agtcctgtgg agtctcggcg gacatcctgc gtgagctgcg ccagcgcgcc     420
   gaaaccaccc tcggtggcga gctggtgggt gcggtgatca ctgtgccggc ctatttcgac     480
   gacgcccagc gccaggctac caaggatgcg cgcgcctggc cggactgaac gtcctgcgcc     540
   tgctcaacga gccgaccgcc gccgccgtgg cctacggcct ggacaagggc gcggaaggcc     600
   tggtggccat ctatgacctg ggtggcggaa ccttcacatt tccatcctgc gcctgacccg     660
   cggtgtcttc gaagtcctgg ccaccggcgg cgatacggcc ctgggtggcg acgacttcga     720
   ccatgccatc gctggctggg tgatcgagca ggctggcctt tcgccgatct ggacccgggc     780
   agccagcggc agttgctgca aatcgcctgt gcggccaagg agcgtcttac cgacgaggcg     840
   tcggtccggg tcgcctatgg cgactggagc ggcgagctga gccgggcgag ctcgacgagt     900
   tgatcgagcc gttcgtcgct cgcagcctga agtcctgccg ccgagcggtg cgcgactccg     960
   gtgtcgatct ggaagaaatc cgctcggtgg tgatggtcgg cggctcgacc cgcgtgcccg    1020
   tgtgcgcacg gcggttggcg agttgttcgg ctgcgagccg ctgaccgata tcgatccgga    1080
   ccaggtggtc gccatcggcg cggccatcca ggccgatgcc ctggccggca acaagcgcgg    1140
   cgagaattgc tgctgctgga cgtcattccg ctgtcgctcg gcctggaaac catgggcggg    1200
   ctgatggaga aggtgattcc gcgcaatacc accatcccgg tggcgcgcgc ccaggagttc    1260
   accacctaca ggacggccag acggccatga tgatccacgt gctgcagggc gagcgcgagc    1320
   tggtgaagga ttgtcgttcg ctggcgcgct tcgagctgcg cggcattccg ccgatggtgg    1380
   cgggcgcggc gaaaatcggg tgaccttcca ggtcgacgcc gacggcttgc tcggcgtgtc    1440
   cgcacgcgaa ctgagttccg gcgtcgaggc cagcatccag gtcaagccgt cctatgggct    1500
   gaccgacggc gagatcgcgc ggatctcaag gactccttcg actacgccgg cgacgacaag    1560
   gctgcccgcg ccctgcgcga acagcaggtc gaggcgcagc ggctgctgga ggccgtgcag    1620
   tcggcgctgg acgtcgacgg cgagcggctc cggacgaaga ggagcgcctg gccatcgcgg    1680
   cgcagatgga cactttgcgt gaactggccg gtggtagcga caccgcggcc atcgagaacc    1740
   agatcaagcg cctgtcccag gtgaccgacg ctttcgccca cggcggatgg acgcgaccgt    1800
   caaggccgcg ctctccggcc gccggctcaa cgaaatcgag gaataa                   1846
<210> 28
   <211> 518
   <212> DNA
   <213> Pseudomonas aeruginosa
<400> 28
   gtgggtaaac cctgtcattt cgcccagttc gacctgcaac cggctttcct ggtcgatctc 60
   gacgagctcg gccagcgcta tcgcgagttg gtgcgcagcg tccatcctga ccgtttcgcc 120
   gacgctccga gcgcgagcag cgtctcgccc tggaacgcgc ggcgcaactg aacgaggcct 180
   accagaccct gaagagcgct ccgcgccggg ccctgtacct gttgaccctg agtggtcacg 240
   agttgccctt ggagctacgg tgcaggatcc ggagtttctc ctgcagcaga tgcagctgcg 300
   cgaggaactg gaggagttgc aggacagcgc ggacctggcc ggcgtggcga ccttcaagcg 360
   tcggctcaag gccgcccagg cgagctggag cgtgagttcg ccgcgtgctg ggacgatgcg 420
   cagcgccgcg aagaggccga acgcctggtc cgtcgcatgc agttcctcga caagctggcg 480
   caggaagtgc gccagctgga agagcgaccg acgattaa 518
<210> 29
   <211> 349
   <212> DNA
   <213> Pseudomonas aeruginosa
<400> 29
   atgaatccac tctaccgcgc cgccatccac cagctttttc tcgctctcga tctcccgacg 60
   cccaacgacg aggagagcgt actcagcctg caggtgggcc cgcatctctg ccatctggca 120
   gaacatcgac cgatcacctg ttgatgttca cccgcctgga aggacaagga gacgctacgg 180
   ccaacgagca gaacctgttc agccaggacc cttgcaagcc gatcctgggc cgtgaccccg 240
   aaagcggcga acggctgctc tggaaccgcc agccctgcag ttgctggaca gggcccagat 300
   ccaccatcaa ctcgaacaac tggtcgccgc cgccgaagag ctacgctga 349
<210> 30
   <211> 813
   <212> DNA
   <213> Pseudomonas aeruginosa
<400> 30
   atgagttgct cccactacga agaacgcgtc gagaaggtcg aactgccgca actgaaggtc 60
   aacggcatgg ccatcggcga agacctgctg gcccgcgaac tgcaatacca cccggcggac 120
   agctatggct ggcgctggag gccgcctgcc gtgcgctgat cgtccgccag ttgctcctgc 180
   aacgggccga cgcgctgggc atcgaggccc gctgcgaaga cggcgagacg ccggaggaag 240
   cgcgcatccg ccaatgctgg aggaagaggt gcaggtcccg gaagccgacg aagacgcctg 300
   ccggacctgg tacgcggcca atccggggcg cctgctcggc ccctggcgcc tgcaattgcg 360
   ccatgtgctg ctggcctgcg cccggacgac ctggaaggcc gcgagacggc gcgcaagcag 420
   gccgcggagc tgctcgacga gttgcgcggg catcccgagc gcttcgtcga cctggcccgg 480
   cgtttctccg cctgcccgtc gaaggagtgg gtggcgacct tggctggatc gagccggggc 540
   agaccgtccc cgaattcgag aagcgcctgc tgcgccgcgc gcccgggctg ctcgagcatc 600
   cgctggaaag ccgctatggc ctgcatgtgg tggaatgctg gcgcgcgaag gcggcgagcc 660
   gctcgacttc gacgcggccc gcgcacagat cgccgcgcac ctgcaagcgc aggtgttgca 720
   gcgagcggtg ggacagtaca tcggcgtgct ggcgggagac gctgcatcga gggcttcgcc 780
   ttcgagggcg cggatggggc gctggtgcaa tag 813
<210> 31
   <211> 280
   <212> DNA
   <213> Pseudomonas aeruginosa
<400> 31
   atggcccgag ccactgcccg ccacatcctg gtttccagcg aagccaagtg caacgaactg 60
   aagaccgcca tcgaaggcgg tgccgacttc gccgaagtcg cccgcgagca ttcctcctgc 120
   ccctccgccg cgacggcggc aacctgggct cgttcggccc cggccagatg gtccgcgagt 180
   tcgaccaggt ggtcttcagc gcgccgctga acgtggtcca ggggccggtg aagacccagt 240
   tcggctacca cctgtggaag tcaccagccg ccaggactga 280
<210> 32
   <211> 1632
   <212> DNA
   <213> Pseudomonas aeruginosa
<400> 32
   atggctgcca aagaagttaa gttcggcgat tccgctcgca agaaaatgct ggtcggcgtg      60
   aacgtgctgg ccgatgccgt caaggccacc ctcggcccga aaggccgcaa cgtggttctg     120
   gacaagtctt cggcgctccg accatcacca aggacggcgt ttccgtcgcc aaggaaatcg     180
   agctgaaaga caagttcgag aacatgggcg cgcaactggt gaaagacgtt gcctccaagg     240
   ccaacgacgc tgccgtgacg gcaccaccac cgcgaccgtc ctggcccagg ccatcgtcaa     300
   cgaaggcctg aaggccgttg ccgccggcat gaacccgatg gacctgaagc gtggcatcga     360
   caaggccacc gtggctatcg tgcccagctg aaagagctgg ccaagccctg cgccgacacc     420
   aaggccatcg cccaggtagg caccatctcc gccaactccg acgagtccat cggccagatc     480
   attgccgaag ccatggaaaa agtcggtaag aaggcgtgat caccgtcgag gaaggctcgg     540
   gcctggaaaa cgaactgtcc gtcgtcgaag gcatgcagtt cgatcgcggc tacctgtccc     600
   cctacttcgt gaacaagccg gacaccatgg ctgccagctg gatagcccgc tgctgctgct     660
   ggtcgacaag aagatctcca acatccgcga aatgctgccg gtgctggaag ccgtcgccaa     720
   ggccggccgt ccgctgctga tcgtcgccga ggacgtcgag gggaagccct ggccaccctg     780
   gtggtgaaca acatgcgtgg catcgtcaag gtcgcggctg tcaaggctcc gggcttcggc     840
   gatcgccgca aggccatgct gcaggacatc gccatcctca ccggcggtac gtgatcagcg     900
   aagaagtcgg cctgagcctg gaaggcgcta ccctggagca cctgggcaac gccaagcgcg     960
   tcgtgatcaa caaggaaaac accaccatca tcgatggcgc cggtgtgcag gctgattcga    1020
   agcccgcgtc ctgcagatcc gcaagcagat cgaggaaacc acttccgact acgaccgcga    1080
   gaagctgcaa gagcgcctgg ccaagctggc cggcggtgtt gccgtgatca aggtaggcgc    1140
   tgcaccgaag tcgagatgaa agagaagaaa gcccgcgtcg aagacgccct gcacgctacc    1200
   cgtgcagcgg tggaagaggg cgtggttccc ggcggcggcg tagccctggt tcgtgccctg    1260
   caagccatcg aaggcctgaa gggtgacaac gaggagcaga acgtcggtat cgccctgcgc    1320
   gtcgcgccgt cgaatcgccg ctgcgccaga tcgtggccaa cgccggcgac gagccgagcg    1380
   tagtggtcga caaggtcaag cagggttccg gcaactacgg cttcaacgct gctaccggcg    1440
   tgtacgcgac atgatcgaga tgggcatcct ggacccggcc aaggtcactc gttccgctct    1500
   gcaggctgcg gcctccatcg gcggtctgat gatcaccacc gaagccatgg ttgccgagat    1560
   cgtggaagac aaccggccat gggcggcatg cctgacatgg gcggcatggg cggcatgggc    1620
   ggcatgatgt aa                                                        1632
<210> 33
   <211> 292
   <212> DNA
   <213> Pseudomonas aeruginosa
<400> 33
   atgaagcttc gtcctctgca tgatcgcgtc gttatccgtc gcagcgagga agagaccaag      60
   accgcaggcg gcatcgtgct gccgggttcc gccgccgaga agccgaaccg cggtgaagtg 120
   gtagccgagg taccggtcgt gtactggaca acggcgaagt gcgcgctctg gcagtgaagg 180
   tgggcgacaa ggtggtcttc gggccttact ccggcagcaa cgccatcaag gtcgatggcg 240
   aggaactgct ggtgtgggcg agtccgaaat cctcgccgtc ctggaagact ga 292
<210> 34
   <211> 438
   <212> DNA
   <213> Pseudomonas aeruginosa
<400> 34
   atggctgagt cgacccgcat tggccaggag agccgcgtca ccctgcattt cgccctcaag 60
   ctggaggacg gcaacgtcgt cgacagcacc ttcgacaagc agccggcctc gttcaaggtc 120
   ggcgacgcaa cctgctgccg ggcttcgaac aggcgctgtt cggcttgaag gctggcgaca 180
   agcgcaccct gagcattctt cccgaacagg gcttcggcca gccgaatccg cagaacgtgc 240
   agatcatgcc gcgcaccagt tccaggacat ggagctggcc gagggcctgc tggtgatctt 300
   caacgacgcg gccaagaccg aactgccggg cgtggtcaag gcgttcgatg agcagcaggt 360
   taccgtcgac ttcaatcatc cctggccggc aagaccctgg ccttcgaagt cgagatcatc 420
   gacgtccagc cggcctga 438
<210> 35
   <211> 614
   <212> DNA
   <213> Pseudomonas aeruginosa
<400> 35
   atgagcgaac tcaacctgac cactgacgaa gcccgtgtca gctacggcat cggccgtcaa 60
   ctgggcgacc aactgcgcga gaatccggtt ccgggcatga ccctggacgc agtcctggcc 120
   ggcctgtcga tgcgttcgca ggtatcgaca gccgggtttc cggcgaggcg ctgtcggcca 180
   gcttccaggt gatccgcgag cgcatgcagg ccgaagccca ggccaaggcc gaagccgccg 240
   ctggcgaagg ccgccctacc tggccgagaa cgccaagcgc gaaggcgtga ccgtgctgcc 300
   ctcgggcctg cagttcgaag tgctgagcac tggcgagggc gccaagcctt cccgcgagga 360
   taccgtgcgc acccactacc aggcaccctg atcgacggca ccgtattcga cagctcctac 420
   cagcgtggcc agccggccga gttcccggtg ggcggcgtga tcgccggctg ggtcgaagcg 480
   ctgcaactga tgaacgccgg ctccaagtgc gcctgcacgt accgagcgaa ctggcctatg 540
   gcggccaggc cgtcggcagc attcccccgc acagcgtgct ggtgttcgac gtcgaactgc 600
   tggaaatcct ctga 614
<210> 36
   <211> 1126
   <212> DNA
   <213> Pseudomonas aeruginosa
<400> 36
   atggcgaaac gagactttta cgaggttctg ggggttgagc gtggcgccag cgaggcggac      60
   ctgaagaagg cctaccgccg cctggcgatg aaataccacc cggaccgcaa tcctggcgac     120
   aaggaagcga ggacaagttc aaggaggcca acgaggccta cgaggtcctc tccgatgcca     180
   gcaagcgggc ggcctacgac cagtacggcc atgccggcgt cgatccgaat atgggtgggg     240
   gcgctggcgc cggttcggcg gggcgagctt ctccgacatc ttcggcgatg tgttcagcga     300
   tttcttcggt ggcggcggtg cccgcggtgg ctcgcgcggc ggcgcgcagc gcggcgcgga     360
   cctgcgctac accctggacc tgacctggag gaggcggtac gtggcaccac cgtcaccatc     420
   cgcgtgccga ccctggtcgg ttgcaagacc tgcaacggca gcggcgccaa gcccggcacc     480
   acaccggtca cctgcaccac ttgcggcgta tcggccaggt gcgcatgcag cagggcttct     540
   tctcggtcca gcagacctgc ccgcgctgcc acggcaccgg caagatgatt tccgatccct     600
   gtggctcctg ccatgggcag gggcgcgtgg aagagagaag accctgtcgg tgaaggtgcc     660
   ggccggcgtc gacaccggcg accgcatccg cctcaccggc gaaggcgagg ccggcagcat     720
   gggtggtccg gcgggcgacc tgtacgtggt ggtcaatgtg cggagcaccc gatcttccag     780
   cgcgacggca agcatctgta ctgcgaagtg ccgatcagtt tcgccgacgc tgcgttgggt     840
   ggcgagctgg aagtgccgac cctggatggc cgggtcaagc tgaagattcg gaaagcaccc     900
   agaccggcaa gctgttccgc ctgcgcggca agggcgtggc cccggtgcgc ggcggcggcg     960
   ccggcgacct gatgtgcaag gtggtggtgg aaaccccggt gaacctggac aagcgcagcg    1020
   cgagctgctc gaggagttcc gcaagtcgtt gcagagcgac acctcgcatt cgcccaaggc    1080
   cagtggctgg ttcgaaggca tgaagcgttt cttcgacgat ctataa                   1126
<210> 37
   <211> 1900
   <212> DNA
   <213> Pseudomonas aeruginosa
<400> 37
   atgggcaaaa tcattggcat cgacctgggt accaccaact cctgcgtggc tatcctggag 60
   aacggtaacg tcaaggtcat cgagaacgcc gagggcgcgc gtaccacccc ctcgatcatc 120
   gcctacacaa cgatggcgaa accctggtgg gccagccggc caagcgccag gcggtcacca 180
   acccgcagaa caccctgtat gcggtgaagc gcctgatcgg ccgtcgcttc gaagagaacg 240
   tggttcagaa agactccaga tggtgccgta cagcatcgtc aaggccgaca acggcgacgc 300
   ctgggtggaa gtgaagggcc agaagatggc gcctccgcag atttccgccg aagtgctgaa 360
   gaaaatgaag aagaccgccg agactacctc ggcgagccgg tcaccgaagc ggtgatcacc 420
   gttccggcct acttcaacga cagccagcgc caggccacca aggacgccgg ccgcatcgcc 480
   ggcctcgacg tcaagcggat catcaacggc cgaccgcggc ggcgctggcc tacggcctgg 540
   acaaggccaa gggcgaccac accgtgatcg tctatgacct gggcggcggt accttcgacg 600
   tgtcggtgat cgagatcgcc gaagtcgacg gcgagaccag ttcgaagtgc tggccaccaa 660
   cggcgacacc ttcctcggcg gcgaagactt cgacatccgc ctgatcgact acctcgtcga 720
   cgagttcaag aaggaaagcg gcatcaacct gaagggcgac ccctggccat gcagcgtctg 780
   aaggaagcgg ccgagaaagc caagatcgag ctgtcctcga cccagcagac cgacgtcaac 840
   ctgccgtacg tgactgccga cgcgagcggt ccgaagcacc tgaacgtcag gtttcccgcg 900
   ccaagctgga gtccctggtc gaagacctgg tgcagcgcac catcgagccg tgccgcaccg 960
   cgctgaagga tgccggcctg gatgtttccg atatccatga agtgatcctg gtcggcgcca 1020
   gacccgcatg ccgctggtac agaagaccgt tgccgagttc ttcggcaagg aagcgcgcaa 1080
   ggacgtcaac ccggacgaag ccgtggctgt cggtgccgcc atccagggcg cggtcctggc 1140
   cgggacgtga aggacgtgct gctgctcgac gtgaccccgc tgaccctcgg catcgagacc 1200
   ctgggcggcg tgatgaccgg tctgatcgag aagaacacca ccatcccgac caagaagtcg 1260
   caggtgttct caccgccgac gacaaccagg gcgcggtgac catccacgtg ctgcagggcg 1320
   agcgcaagca ggccgcgcag aacaagtcgc tgggcaagtt cgacctggcc gacattccgc 1380
   cggctccgcg cggcgtgcgc agatcgaggt gaccttcgat atcgatgcca acggcatcct 1440
   gcacgtctcg gcgaaggaca aggccaccgg caagcagcag tccatcgtga tcaaggcatc 1500
   ctccggcctg tccgaggatg agatcagcag atggtccgcg atgccgaggc gaacgccgag 1560
   gaggaccgca agttcgagga actggctgcc gctcgcaacc agggcgacgc gctggtccac 1620
   gcgacccgca agatgatcac cgaggcgggc gcaaggccac cgccgaggac aaggcgacca 1680
   tcgagaaggc gctgggcgag ctggaagcgg cggtgaaggg cgacgacaag gccgagatcg 1740
   aggccaagat gaacgctctg tcccaggctt ccaccccgtg gcgcagaaga tgtacgccga 1800
   acaggcccag cagggcgaag acgctcccca gggcgagcag gcgaaagccg ctgacgacgt 1860
   ggtggacgcc gagttcgaag aggtcaagga caacaagtaa 1900
<210> 38
   <211> 557
   <212> DNA
   <213> Pseudomonas aeruginosa
<400> 38
   atggctgacg aacagcagac cctggatcaa cagacccccg agcagcccac cggcgccgcc      60
   gaggacctga ccgcccgcgt gcaggaactg gaggagcagc tggctgccgc ccaggaccag     120
   gcgctgccat ggtcgcggat ctgcagaacg tacgccggcg tgcggagcag gatgtcgaga     180
   aggcgcacaa gttcgcgctg gagaaattcg ccggcgacct gctggcggtg gtcgacaccc     240
   tcgagcgcgg cctgagatgt ccgatcccaa cgacgaggcg atcaagccga tgcgcgaagg     300
   gatggaactg accctgaaga tgttcgacga caccctgcgc cgctaccagg tggaggcgct     360
   gaacccggaa ggcgagcctt taaccccgag cagcaccagg cgatggccat gcaggaaagc     420
   gcttcggccg agccgggcag tgtcctcaag gtgttccaga aaggctacct gctcaacggc     480
   cgcctgctgc gtccggcgat ggtggtagca gcaaggcgcc ggcggaaacc ccgccgtcca     540
   tcgacgagca ggcttga                                                    557
<210> 39
   <211> 1763
   <212> DNA
   <213> Pseudomonas aeruginosa
<400> 39
   atgcgccgcc tgctgaccct gatcctcctg ctggtcgccc tgcccgccgg cgcgggcctg      60
   ttcgacagcc gtcccggcgc ctccctcggc ggcggcctcg atggcggtgg cttcctcaag     120
   gtcgaccggc gttcaagccc agcgtcgagc gcagcgacgc gcagcacgtg ctgctgcgct     180
   tcgtcaacgc cgagggctac tacctctacc gccaccgctt ccagttcaag gtcgagccag     240
   cccaggtcag cctcgccagg tgcagctgcc ggcgggcaag cagcaccacg acgagtactt     300
   cggcgatacc gaggtctact acaacatcgt cgacctggag attcccctga acaacccggc     360
   ccggcagccc tataccctcg tgtgacgtac cagggctgcg ccgacaaggg cctctgctac     420
   cccccggaaa ccaggcgctt cgacatcgac ggcggtgccg cgacgccagc cgcgagcgac     480
   gcggccggca agggcccgct cgagcacagg gcaagcgcag cctgctgttc ttcttcctcg     540
   ccggcctgac cctgaccttc accccctgcg tgctgccgat gctgccgatc ctctccggag     600
   tggtcctgcg cggccggccg ggcggtggtc gcggctcgtc ctgtcgctcg cctacgtgct     660
   gccgatggcg ctctgcttcg ccctgctcgg cgcgctgatg ggcatgttcg gcgccagcct     720
   caatctccag gcgcaattgc agtcgccctg ggtactggtg ccttcgcggc gttcttcgcg     780
   ctgttcgccg tggccatgtt cggcttcttc gagctgcgcc tgcccggctt catccgcgaa     840
   ccgctcgacc gcctggccgg cgatgcgcgc ggcggctcga tcctcggcgg gccaccctcg     900
   gcgtgctttc cagcctgctg gtctcgccct gcgtctccgc cccgctggcc gcctcgctgc     960
   tctatatcag cgccagcggc gatgcctggg ggggcggcct gcaactgttc gccctcgcct    1020
   gggcatgggt acgccgctgg tggtgttcgg cgccggcggc ggcgccctgc tgcccaagag    1080
   cggagcctgg atgaacggcg tgcgcaacgc cttcggcgtg ctgttgctgg cggtcgccgt    1140
   gtgctgctgg agcgggtggt gtccggcccg gtggcgctga tgctctgggg catgctcgcc    1200
   ggcggcgccg gcctggccct gggcgccctc gaattcaccc cgaagagcgc cgcacgccgc    1260
   ctgctgcaac gcttggcctg atgttcctga cctacgcggt ggccgcctgg atcggcgccc    1320
   tgcagggcga gtcggacccg atccatccgc tgggccgctc ggtgccgtcg atccatgccg    1380
   ggccgccgag cacgcccgcg aatggcagaa cctgaccacc ccggcgcagt tggacgccgc    1440
   cctggccgag gcacgtcagg ccggcaagcc ggtgctgctg gactggtatg cggactggtg    1500
   catcagttgc aaggtcatcg agcgcaggtt ctcaccgatc ccacggtgca ggcacgactg    1560
   ccggcctacc gcctgctgcg cttcgacatc accgaaagca acccggccca gcgcggcctg    1620
   ctggaccgct acaacctgtt cggtccgccg ggatcctgtt cttcgccccg ggcggtgacg    1680
   aatggagcga cttgcgcgtc atcggagaga tcgacgccgc cgggctcgcc gaacgactgc    1740
   gccgggccgc tacccggcag tga                                             1763
<210> 40
   <211> 1257
   <212> DNA
   <213> Pseudomonas aeruginosa
<400> 40
   atgaacccga gcaccgccgc ccgcgccctg ggcatcgatt ttggcacctc caactccacc 60
   gtcggctggt ggcggccgga ggtcgaaccg ctgatcgagc tggaggacgg caagatcacc 120
   ctgccctggt ggtgttcttc aacgtcgagg agcgccgccc ggtctacggc cgccaggcgc 180
   tcggcgagta cctggagggc tacgagggcc ggctgatgcg ctcgctgaag agcctgctgg 240
   gctccaagct gctgagagcg agaccaccgt gctcggcagc gccctgccgt tcaaggacct 300
   gctcggcctg ttcatcggcc agctcaaggc gcgcggcgag gccgccgccg ggcaagcctt 360
   cgacgcggtg gtgctgggtc gccggtgttc ttcgtcgacg acgacccgga ggccgaccgc 420
   gaggcccagg acaccctggt gcaggtggcc aacaagctcg gcttcaagga ggtttccttc 480
   cagtacgaac cgatcgccgc ggccttcgct acgagcgctg catccagcgc gaggaactgg 540
   tcctgatcgt cgacatcggc ggcggcacct cggacttctc cctggtgcgc ctggcgccgg 600
   agcggcgcaa cctggccgag cgccaggacg acatctggcc accggcggcg tgcacatcgg 660
   cggtaccgac ttcgacaagc agctcagcct ggaaggcgtg atgccgctgt tcggctacgg 720
   cagccggatg aagagcgatg ccttcatgcc caccagctac cactgaacct ggccacctgg 780
   cacaccatca acgcggtgta cgcgcagaag tcgcaattgg cgctgaagaa catgcgctac 840
   gacatcgtcg acagcaccgg catcgaccgc ctcttcaggc tcatcgagga cgcgccgggc 900
   actggctggc gatgcaggtg gaagacagca agatccgcct gaccgaaacc gagcgcctgc 960
   acctctcgct ggagcgcatc gaggccgggc tcggcgtgga gctgacccgt gggctgtcga 1020
   gaacgccgtc gacggactgc tggagcgcgt gcgcaacagc gtcgcgcaac tgctggcctc 1080
   ggccggggtc gacccggacc gggtcgacac ggtgttcttc accggcggtt cgagcggcat 1140
   tccgcgctgc gccggagcgt ctcggcgatg ctgcccaacg cccgtcatgt cgagggcaac 1200
   ctgttcggca gcatcggcag cggcctggcc atcgaggcga agaagcgcta cggctga 1257
<210> 41
   <211> 530
   <212> DNA
   <213> Pseudomonas aeruginosa
<400> 41
   ttgaccacca tcgtatctgt ccgccgcaac ggcaaagtcg tcatgggcgg cgacggccag 60
   gtttccctgg gcaacaccgt gatgaaaggc aacgccaaga aggtccggcg cctgtatcac 120
   ggtcaggact ggccggtttc gccggcgcca ccgccgatgc cttcaccctg ttcgagcgct 180
   tcgagcagca attggagaaa caccaggggc acctggtccg cgccgccgtc gaactggcca 240
   aggactggcg caccaccgct ccctgagccg cctcgaagcc atgctcgcgg tcgccaacaa 300
   ggatgcctcg ctgatcatca ccggcaacgg cgacgtggtc gaacccgaac acggcctgat 360
   cgccatgggc tccggcggcg gttcgcccag gccgcggccc tggccctgct gcagcacaac 420
   gccgagctga gcgctcggga agtcgccgag accgcgctga acatcgccgg ctcgatctgc 480
   gtcttcacca accagaacct gaccatcggg agctggacag cgccgtctga 530
<210> 42
   <211> 1334
   <212> DNA
   <213> Pseudomonas aeruginosa
<400> 42
   atgtccatga cgccccgcga gatcgtccac gaactcaacc gccacatcat cggccaggac 60
   gacgccaagc gcgccgtcgc catcgccctg cgcaaccgct ggcgacgcat gcaactgccg 120
   gccgagcgcg cgccgaggta acaccgaaga acatcctcat gatcggcccc accggcgtcg 180
   gcaagaccga gatcgcccgc cgcctggcgc gcctggcgaa cgcgccgttc atcaaggtcg 240
   aggcgaccaa gttcccgagg tcggctatgt cggacgcgac gtcgaatcga tcatccgcga 300
   tctcgccgac gccgcggtga agatgctccg cgaacaggag atccagaagg tcaagtatcg 360
   cgccgaggac gccgccgaag acgcatcctc gatgccctgc tgccggccgc gcgtcccgcc 420
   atgggcttcg gcgacgagcc ggcacgcgag gacagcaaca cccgccagct gttccgcaag 480
   cgcctgcgcg aaggccagct ggacgacagg aaatcgacat cgaggtggcc gacaacccgg 540
   ccggcgtgga gatcatggcc ccgcccggca tggaggagat gaccaaccag ttgcagaacc 600
   tgttctccgg catgagcaag ggcaagaaga agaccgcaag ctgaaggtcg ccgaggccct 660
   gaagctgatc cgcgacgaag aggcggtgcg cctggtcaac gaggaagagc tcaaggcacg 720
   cgccctggag gcggtcgagc agcacggcat cgtcttcatc gagagatcga caagatcgcc 780
   aagcgcgcca acgccggcgg cgccgacgtc tcccgcgagg gcgtacagcg cgacctgctg 840
   ccgctgatcg agggctgcac ggtgaacacc aagctgggca tggtcaagac gaccacatcc 900
   tgttcatcgc ctccggcgcc ttccacctga gcaagccgag cgacctggtt cccgagctgc 960
   agggccgcct gccgatccgc gtggagctca aggccctcag tccgaacgat ttcgaggcat 1020
   cctcaccgag ccgcatgcct cgctcaccga gcagtaccgc gagctgctga agaccgaggg 1080
   gctggccatc gagttcgccg aggacggcat caagcgcctt gccgagatcg cctggcaggt 1140
   caagagaaga ccgagaacat cggtgcccgc cgcctgcata cgctgctcga gcggctgctg 1200
   gaagaggtct cgttcagcgc cgccgacctg gccagcgagc atagcgacaa gccgatcctg 1260
   atcgatgccg ctacgtcaac agccacctcg gcgagctggc cgaggacgag gacctgtccc 1320
   gctacatcct ttga 1334
<210> 43
   <211> 489
   <212> DNA
   <213> Pseudomonas aeruginosa
<400> 43
   atgaccgagc aagctaccaa cggcgctgcc gacgagcagc aaccccagtt ctccctgcag 60
   cgcatctacc tgcgcgacct gtcgttcgag tcgccgaagt caccggaaat cttccgccag 120
   gaatggaccc gtccatcagc ctggacctga acactcgcca gaagcagctg gacggcgact 180
   tctacgaagt ggtcctgacc gtatcggtga ccgtgaagaa cggcgaggac accactgcct 240
   tcatcgccga agtgagcagg ccggcatctt cctgatcaag aacctcgatc cgtcgagcat 300
   gagccatacc cttggcgcgt tctgcccgaa catcctcttc ccgtacgctc gcgaagccct 360
   ggacaacctg gtggtacgcg gtcgttcccg gcgctgatgc tgtcgccggt gaacttcgac 420
   gccctctacg cccaggaaat cgcccgcatg caggcctccg gcgagatccc gacgccgtcg 480
   gtccagtaa 489
<210> 44
   <211> 888
   <212> DNA
   <213> Pseudomonas aeruginosa
<400> 44
   atgtcccatt cagatcagag ccagcgtttc cttttcgacg ataccgacgt gcgcggcgag 60
   atggtcgatc tcgagcgcag ctacagcgaa gtgctggcca aacatcccta tccggaaccg 120
   gtcgcgcgct gctcggggag atgctcgcgg cggcctcgct gctctgcggc acgctcaagt 180
   tcgacggcct gctggtattg caggcgcgct ccagcggcgc ggttccgctg ttgatggtgg 240
   agtgttccag cgacgccagg tgcgtggcct ggcccgctat tcggcggagt ccatcggcgc 300
   cgacgccggc atgcaggaac tgatgcccga aggcgtgctg accctcaccg tggacccggt 360
   caagggccag cgctaccagg ggatcgtcgc cctggagggg gtgaacctgg cggaatgcct 420
   gtccaactac ttcgccagct cggaacagct gccgacccgt ttctggctca acgccaacgg 480
   ccgccgtgcc cgcggcctgc gctgcagcag cttcccgccg accgcctgaa ggaccccgag 540
   gcccgcgagg ccagttggca gcacctgacc accctggccg acacgctgac cgccgaagag 600
   ctgctggcgc tggacaacga gaccgtctgc accgcctcta ccacgaggaa accgtgcgcc 660
   tgttcgagcc gcaaccgctg gtcttccatt gcagttgctc gcgggaacgt tcggccaacg 720
   ccctggtcag tctcgggcag gccgactgcg agcgctgctc gaggaggaag aaggctcgat 780
   cagcatcgac tgccagttct gcaaccagcg ctacctgttc gacgcctccg acgtcgccca 840
   gttgttcgct ggcgctggca gccaggggcc ttccgagacc cccactga 888
<210> 45
   <211> 393
   <212> DNA
   <213> Pseudomonas aeruginosa
<400> 45
   gtgagcgaga aagacgacga caaggtgcgc ctggacaagt ggctctgggc ggcgcgcttc     60
   ttcaagaccc gggcgctggc caaggccgcg atagagggcg gcaaggtgca ttgccggggc    120
   gagcggtcaa gccgagcaag gaacccaggg taggggagga gttgaccatt cgtacgggct    180
   tcgacgagcg aaccgtgacc attcgcgcgc ttgccgtagt ccgtcgtggc gcgcaggagg    240
   cccaggctct ctacaggaaa ccgcggacag cgtggaaagc cgcgagcgcg cggcggccat    300
   gcgcaaggcc ggggcgctgg gcgtacagac cgaggggcgg ccgagcaaga agcagcggcg    360
   gcagatccac cagttgcgcg gggccaggac tga                                 393
<210> 46
   <211> 626
   <212> DNA
   <213> Pseudomonas aeruginosa
<400> 46
   atgccgcgcc gccttttcat cggtcttttt ctactgccgc tgccgctttt cgccgcgcct     60
   cccaaggacg aactggccta tgccgtaggc gcccgcctgg gtatgcgcct acagcaggaa    120
   atgcccgcct agagctatcc gagctacttc tcggactgcg ccaggcctat cgcggcgagg    180
   cacttgaaat cccccccgaa cgcatcgaac aacttctgct gcaacacgag aacgccacga    240
   cggaaacacc gcggccacac ccgccgaggc ccgcttcctg gccaacgaaa aagcccgctt    300
   cggcgtccgc gaattgaccg gcggcgtcct cgtcagcgaa ttacgtcgtg gccaaggtaa    360
   cggcatcggc gcggcaaccc agtacacgtc cgttaccgcg gcctgcttgc cgacggtcaa    420
   gtgttcgacc agagcgaaag cgccgagtgg ttcgccctgg acagtgtgat agagggatgg    480
   cggacagcgt tgcgagcgat gcctgtcgcg cgcgctggcg agtggtgatt ccttcggcgc    540
   aagcctatgg tcacgaaggc gctggcgacc tgatcccgcc cgacgctccg ctggtcttcg    600
   agatcgacct gctgggcttc cgctga                                         626
<210> 47
   <211> 489
   <212> DNA
   <213> Pseudomonas aeruginosa
<400> 47
   atgcccctgg ccagcccccg tcagcttttc cttctcgcgt tcctggcctg cgtcgccatc 60
   atgggcgggg cgctgtacct ggaacatgtg gttggcctgg aggcctgccc gctgtgcgtc 120
   gtgcagcgat cttcttcatc ctgatcggcc tgacctgcct tgctggcgcg atccaggggc 180
   ccggcctgcg tgggcggcgt atctactccg tgctggtgtt cctgctcgct ctcggcggcg 240
   gggccacggc cgccgccagg tatggttgca gaccgttccg ctggaccaac tgccggcctg 300
   cctgcccagc ctcgactaca tgatgcaggc gcttcccttc caggaagtga tccgcctggt 360
   cctgcatggc accgcggatt ggcccaggtg agctggacgc tattcaccct gagcattccc 420
   gaatggagcc tgctggcgtt cgttgcctat ctcggcttct ccatcgtgca gttcctccga 480
   cgtgcctga 489
<210> 48
   <211> 632
   <212> DNA
   <213> Pseudomonas aeruginosa
<400> 48
   atgcgtaacc tgattctcac cgccatgctg gccatggcca gcctgttcgg catggccgcc 60
   caggccgacg actataccgc cggcaaggaa tacgtcgagc tgagcagccc ggtgccggtg 120
   tcccagcggg caagatcgaa gtggtggaac tgttctggta tggctgcccg cattgctacg 180
   cgttcgagcc gaccatcgtg ccgtggagcg agaagctgcc ggcagatgtc catttcgtgc 240
   gcctgcctgc cctgtcggcg gtatctggaa cgtccatggg cagatgttcc tgaccctgga 300
   aagcatgggt gtcgagcatg acgtacacaa cgccgtgttc gaggcgatcc acaaggagca 360
   caagaagctc gccactccgg agaaatggcc gatttcctcg ccggcaaggg cgtggacaag 420
   gaaaaattcc tgagcaccta taattccttt gccatcaagg gccagatgga aaaggccaag 480
   aagctggcga tggcctacca ggtcaccgcg taccgaccat ggtggtcaat ggcaaatatc 540
   gcttcgacat cggctccgcc ggtggtccgg aggaaaccct caagctggcc gactacctga 600
   tcgagaaaga gcgcgcagcg gccaagaagt ag 632